(19) Europäisches Patentamt
European Patent Office
Office européen des brevets

(11) EP 0 502 314 B1

(12) # EUROPÄISCHE PATENTSCHRIFT

(45) Veröffentlichungstag und Bekanntmachung des Hinweises auf die Patenterteilung:
**20.05.1998 Patentblatt 1998/21**

(21) Anmeldenummer: **92101579.8**

(22) Anmeldetag: **31.01.1992**

(51) Int Cl.$^6$: **C07D 235/08**, A61K 31/415, C07D 401/04, C07D 403/04, C07D 403/10, C07D 401/14, C07D 403/14, C07D 471/04, C07D 487/04, C07D 513/04

(54) **Benzimidazole, diese Verbindungen enthaltende Arzneimittel und Verfahren zu ihrer Herstellung**

Benzimidazol derivatives, medicaments containing them and process for their preparation

Benzimidazoles, médicaments les contenant et procédé pour leur préparation

(84) Benannte Vertragsstaaten:
**AT BE CH DE DK ES FR GB GR IT LI LU NL PT SE**

(30) Priorität: **06.02.1991 DE 4103492**
**25.05.1991 DE 4117121**
**16.11.1991 DE 4137812**

(43) Veröffentlichungstag der Anmeldung:
**09.09.1992 Patentblatt 1992/37**

(73) Patentinhaber: **Dr. Karl Thomae GmbH**
**D-88397 Biberach (DE)**

(72) Erfinder:
- **Hauel, Norbert, Dr. Dipl.-Chem.**
  **W-7951 Eberhardzell (DE)**
- **Narr, Berthold, Dr. Dipl.-Chem.**
  **W-7950 Biberach 1 (DE)**
- **Ries, Uwe, Dr. Dipl.-Chem.**
  **W-7950 Biberach 1 (DE)**
- **van Meel, Jacques, Dr.**
  **W-7951 Mittelbiberach (DE)**
- **Wienen, Wolfgang, Dr. Dipl.-Biol.**
  **W-7951 Äpfingen (DE)**
- **Entzeroth, Michael, Dr. Dipl.-Chem.**
  **W-7951 Warthausen (DE)**

(74) Vertreter: **Laudien, Dieter, Dr.**
**c/o Boehringer Ingelheim GmbH**
**55216 Ingelheim (DE)**

(56) Entgegenhaltungen:

| | |
|---|---|
| **EP-A- 392 317** | **EP-A- 399 731** |
| **EP-A- 399 732** | **EP-A- 400 835** |
| **EP-A- 400 974** | **EP-A- 420 237** |
| **EP-A- 459 136** | **EP-A- 468 470** |
| **US-A- 4 880 804** | |

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 502 314 B1

Printed by Jouve, 75001 PARIS (FR)

## Beschreibung

In den Europäischen Offenlegungsschriften 0,468,470, 0,459,136 und 0,420,237, welche nur teilweise eine Vorveröffentlichung darstellen, sowie in der EP-A-0,400,835, EP-A-0,399,732 und US-A-4,880,804 werden bereits Benzimidazole, welche Angiotensin-II-Antagonisten darstellen, beschrieben.

Es wurde nun gefunden, daß die neuen Benzimidazole der allgemeinen Formel

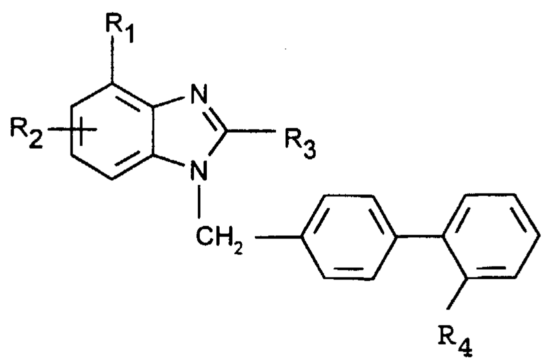

(I),

wobei sich diese von den in den vorstehend erwähnten Offenlegungsschriften beschriebenen Benzimidazolen immer durch den Rest $R_2$ unterscheiden sowie die Verbindungen der allgemeinen Formel I, in denen $R_2$ eine Pyridyl- oder Imidazolylgruppe bedeutet, eine Auswahl aus der EP-A-0,400,835 darstellen, noch wertvollere Angiotensin-II-Antagonisten als die literaturbekannten darstellen.

Gegenstand der vorliegenden Erfindung sind somit die neuen Benzimidazole der obigen allgemeinen Formel I und deren Salze, insbesondere für die pharmazeutische Anwendung deren physiologisch verträglichen Salze mit anorganischen oder organischen Säuren oder Basen, diese Verbindungen enthaltende Arzneimittel und Verfahren zu ihrer Herstellung.

In der obigen allgemeinen Formel I bedeutet

$R_1$ ein Fluor-, Chlor- oder Bromatom, eine Alkyl-, Cycloalkyl-, Fluormethyl-, Difluormethyl- oder Trifluormethylgruppe,

$R_2$ eine gegebenenfalls durch eine oder zwei Alkylgruppen oder durch eine Tetramethylen- oder Pentamethylengruppe substituierte 5-, 6- oder 7-gliedrige Alkylenimino- oder Alkenyleniminogruppe, in welcher eine Methylengruppe durch eine Carbonyl- oder Sulfonylgruppe ersetzt ist,

eine gegebenenfalls durch eine Alkyl- oder Phenylgruppe monooder disubstituierte Maleinsäureimidogruppe, wobei die Substituenten gleich oder verschieden sein können,

eine gegebenenfalls in 1-Stellung durch eine Alkylgruppe mit 1 bis 6 Kohlenstoffatomen oder eine Cycloalkylgruppe substituierte Benzimidazol-2-yl- oder 4,5,6,7-Tetrahydro-benzimidazol-2-yl-gruppe, wobei der Phenylkern einer der vorstehend erwähnten Benzimidazolgruppen zusätzlich durch ein Fluoratom, eine Methyl- oder Trifluormethylgruppe substituiert sein kann, eine Imidazo[2,1-b]thiazol-6-yl-, Imidazo[1,2-a]pyridin-2-yl-, 5,6,7,8-Tetrahydro-imidazo[1,2-a]pyridin-2-yl-,Imidazo-[1,2-a]pyrimidin-2-yl-, Imidazo[4,5-b]pyridin-2-yl-, Imidazo-[4,5-c]pyridin-2-yl-, Imidazo[1,2-c]pyrimidin-2-yl-, Imidazo-[1,2-a]pyrazin-2-yl-, Imidazo[1,2-b]pyridazin-2-yl-, Purin-8-yl-, Imidazo [4,5-b]pyrazin-2-yl-, Imidazo[4,5-c]pyridazin-2-yl- oder Imidazo[4,5-d]pyridazin-2-yl-gruppe,

eine Pyridylgruppe oder

eine gegebenenfalls in 1-Stellung durch eine Alkyl- oder Benzylgruppe über ein Kohlenstoffatom gebundene Imidazolylgruppe,

welche zusätzlich im Kohlenstoffgerüst durch eine Alkylgruppe substituiert sein kann,

$R_3$ eine Alkylgruppe mit 1 bis 5 Kohlenstoffatomen oder eine Cycloalkylgruppe mit 3 bis 5 Kohlenstoffatomen und

$R_4$ eine Carboxy- oder IH-Tetrazolylgruppe,

wobei, soweit nichts anderes erwähnt wurde, ein vorstehend erwähnter Alkylteil jeweils 1 bis 3 Kohlenstoffatome sowie ein vorstehend erwähnter Cycloalkylteil jeweils 3 bis 7 Kohlenstoffatome enthalten kann.

Für die bei der Definition der Reste $R_1$ bis $R_3$ eingangs erwähnten Bedeutungen kommt beispielsweise für

$R_1$ die Bedeutung des Fluor-, Chlor- oder Bromatoms, der Methyl-, Ethyl-, n-Propyl-, Isopropyl-, Cyclopropyl-, Cyclobutyl-, Cyclopentyl-, Cyclohexyl-, Cycloheptyl-, Fluormethyl-, Difluormethyl- oder Trifluormethylgruppe,
$R_2$ die der 2-Oxo-pyrrolidino-, 2-Oxo-piperidino-, 2-Oxo-hexamethylenimino-, Propansultam-1-yl-, Butansultam-1-yl-, Pentansultam-1-yl-, Maleinsäureimido-, 2-Methyl-maleinsäureimido-, 2-Phenyl-maleinsäureimido-, 2-Methyl-3-phenyl-maleinsäureimido-, Pyridin-2-yl-, 4-Methyl-imidazol-2-yl-, 1-Methyl-imidazol-4-yl-, 1-Methyl-imidazol-5-yl-, 1-Benzyl-imidazol-4-yl-, 1-Benzyl-imidazol-5-yl-, 1,2-Dimethyl-imidazol-4-yl-, 1,2-Di-methyl-imidazol-5-yl-, 1-Benzyl-2-methyl-imidazol-4-yl-, l-Benzyl-2-methyl-imidazol-5-yl-, Benzimidazol-2-yl-, 1-Methylbenzimidazol-2-yl-, 1-Ethyl-benzimidazol-2-yl-, 1-n-Propyl-benzimidazol-2-yl-, l-Isopropyl-benzimidazol-2-yl-, 1-n-Butyl-benzimidazol-2-yl-, 1-Isobutyl-benzimidazol-2-yl-, 1-n-Pentyl-benzimidazol-2-yl-, l-n-Hexyl-benzimidazol-2-yl-, 1-Cyclopropyl-benzimidazol-2-yl-, l-Cyclobutyl-benzimidazol-2-yl-, 1-Cyclopentyl-benzimidazol-2-yl-, 1-Cyclohexyl-benzimidazol-2-yl-, 5-Methyl-benzimidazol-2-yl-, 1,5-Dimethyl-benzimidazol-2-yl-, 1,6-Dimethyl-benzimidazol-2-yl-, 1,4-Dimethyl-benzimidazol-2-yl-, 5-Fluor-1-methyl-benzimidazol-2-yl-, 6-Fluor-1-methyl-benzimidazol-2-yl-, 5-Trifluormethyl-benzimidazol-2-yl-, 5-Trifluormethyl-1-methyl-benzimidazol-2-yl-, 4,5,6,7-Tetrahydro-benzimidazol-2-yl-, 4,5,6,7-Tetrahydro-1-methyl-benzimidazol-2-yl-, 4,5,6,7-Tetrahydro-1-ethylbenzimidazol-2-yl-, 4,5,6,7-Tetrahydro-1-n-butyl-benzimidazol-2-yl-, 4,5,6,7-Tetrahydro-1-n-hexyl-benzimidazol-2-yl-, 4,5,6,7-Tetrahydro-1-cyclopropyl-benzimidazol-2-yl-, 4,5,6,7-Tetrahydro-1-cyclohexyl-benzimidazol-2-yl-, Imidazo-[1,2-a]pyrimidin-2-yl-, 5,6,7,8-Tetrahydro-imidazo[1,2-a]pyridin-2-yl-, Imidazo[4,5-c]pyridin-2-yl-, Imidazo[2,1-b]thiazol-6-yl-, Imidazo[1,2-c]pyrimidin-2-yl-, Imidazo[1,2-a]pyrazin-2-yl-, Imidazo[1,2-b]pyridazin-2-yl-, Purin-8-yl-, Imidazo-[4,5-b]pyrazin-2-yl-, Imidazo[4,5-c]pyridazin-2-yl- oder Imidazo [4,5-d]pyridazin-2-yl-gruppe und
für $R_3$ die der Methyl-, Ethyl-, n-Propyl-, Isopropyl-, n-Bu-tyl-, Isobutyl-, tert.Butyl-, n-Pentyl-, 1-Methyl-butyl-, 2-Methyl-butyl-, 3-Methyl-butyl-, Cyclopropyl-, Cyclobutyl-oder Cyclopentylgruppe in Betracht.

Bevorzugte Verbindungen der obigen allgemeinen Formel I sind diejenigen, in denen

$R_1$ ein Chloratom, eine Alkylgruppe mit 1 bis 3 Kohlenstoffatomen oder eine Trifluormethylgruppe,

$R_2$ eine 5-, 6- oder 7-gliedrige Alkyleniminogruppe, in welcher eine Methylengruppe durch eine Carbonyl- oder Sulfonylgruppe ersetzt ist,

eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können,

eine gegebenenfalls in 1-Stellung durch eine Alkylgruppe mit 1 bis 6 Kohlenstoffatomen oder eine Cycloalkylgruppe substituierte Benzimidazol-2-yl- oder 4,5,6,7-Tetrahydro-benzimidazol-2-yl-gruppe, wobei der Phenylkern einer der vorstehend erwähnten Benzimidazolgruppen zusätzlich durch ein Fluoratom, eine Methyl- oder Trifluormethylgruppe substituiert sein kann, eine Imidazo[2,1-b]thiazol-6-yl-, Imidazo[1,2-a]pyridin-2-yl-, 5,6,7,8-Tetrahydroimidazo[1,2-a]pyridin-2-yl-, Imidazo[1,2-a]-pyrimidin-2-yl-, Imidazo[4,5-b]pyridin-2-yl-, Imidazo[4,5-c]-pyridin-2-yl-, Imidazo[1,2-c]pyrimidin-2-yl-, Imidazo[1,2-a]-pyrazin-2-yl-, Imidazo[1,2-b]pyridazin-2-yl-, Purin-8-yl-, Imidazo [4,5-b]pyrazin-2-yl-, Imidazo[4,5-c]pyridazin-2-yl-oder Imidazo [4,5-d]pyridazin-2-yl-gruppe,

eine Pyridylgruppe oder

eine in 1-Stellung durch eine Alkylgruppe mit 1 bis 3 Kohlenstoffatomen oder durch eine Benzylgruppe substituierte Imidazol-4-yl-gruppe, welche zusätzlich im Kohlenstoffgerüst durch eine Alkylgruppe mit 1 bis 3 Kohlenstoffatomen substituiert sein kann,

$R_3$ eine Alkylgruppe mit 1 bis 5 Kohlenstoffatomen oder eine Cycloalkylgruppe mit 3 bis 5 Kohlenstoffatomen und

$R_4$ eine Carboxy- oder IH-Tetrazolylgruppe bedeuten,

und deren Salze mit anorganischen oder organischen Säuren oder Basen.

Besonders bevorzugte Verbindungen der obigen allgemeinen Formel I sind diejenigen, in denen

$R_1$ eine Methylgruppe oder ein Chloratom,

$R_2$ eine 5-, 6- oder 7-gliedrige Alkyleniminogruppe, in welcher eine Methylengruppe durch eine Carbonyl- oder Sulfonylgruppe ersetzt ist,

eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können,

eine gegebenenfalls in 1-Stellung durch eine Alkylgruppe mit 1 bis 3 Kohlenstoffatomen substituierte Benzimidazol-2-yl- oder 4,5,6,7-Tetrahydro-benzimidazol-2-yl-gruppe, wobei der Phenylkern einer der vorstehend erwähnten Benzimidazolgruppen zusätzlich durch ein Fluoratom substituiert sein kann, eine Imidazo[1,2-a]pyridin-2-yl-, 5,6,7,8-Tetrahydro-imidazo[1,2-a]- pyridin-2-yl-, Imidazo[1,2-a]pyrimidin-2-yl- oder Imidazo-[2,1-b]thiazol-6-yl-gruppe oder

eine in 1-Stellung durch eine Alkylgruppe mit 1 bis 3 Kohlenstoffatomen substituierte Imidazol-4-yl-gruppe,

$R_3$ eine Alkylgruppe mit 1 bis 5 Kohlenstoffatomen oder eine Cycloalkylgruppe mit 3 bis 5 Kohlenstoffatomen und

$R_4$ eine Carboxy- oder IH-Tetrazolylgruppe bedeuten,

und deren Salze mit anorganischen oder organischen Säuren oder Basen.

Erfindungsgemäß erhält man die Verbindungen nach folgenden Verfahren:

a) Cyclisierung einer Verbindung der allgemeinen Formel

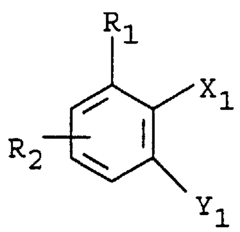

(II),

in der
$R_1$ und $R_2$ wie eingangs definiert sind,
einer der Reste $X_1$ oder $Y_1$ eine Gruppe der allgemeinen Formel

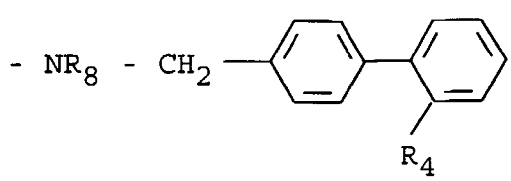

und der andere der Reste $X_1$ oder $Y_1$ eine Gruppe der allgemeinen Formel

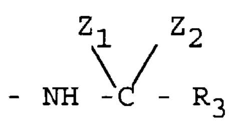

darstellen, wobei

$R_3$ und $R_4$ wie eingangs definiert sind,
$R_8$ ein Wasserstoffatom oder eine $R_3CO$-Gruppe, wobei $R_3$ wie vorstehend erwähnt definiert ist,
$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder
$Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxyoder Alkylendithiogruppe mit jeweils 2 oder 3 Kohlenstoffatomen bedeuten, wobei jedoch einer der Reste $X_1$ oder $Y_1$ eine Gruppe der allgemeinen Formel

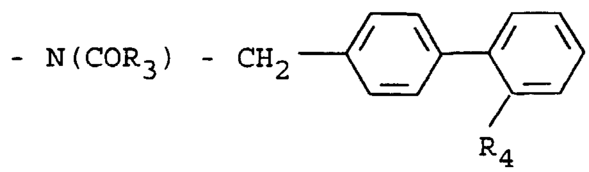

oder

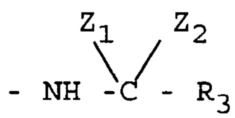

darstellen muß, und gegebenenfalls Reduktion eines so erhaltenen entsprechenden N-Oxids.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin oder in einem Überschuß des zur Herstellung der Verbindung der allgemeinen Formel II verwendeten Acylierungsmittel, z.B. in dem entsprechenden Nitril, Anhydrid, Säurehalogenid, Ester oder Amid, beispielsweise bei Temperaturen zwischen 0 und 250°C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Methansulfonsäure, Salzsäure, Phosphorsäure, Polyphosphorsäure, Essigsäureanhydrid oder gegebenenfalls auch in Gegenwart einer Base wie Kaliumäthylat oder Kaliumtert.butylat durchgeführt. Die Cyclisierung kann jedoch auch ohne Lösungsmittel und/ oder Kondensationsmittel durchgeführt werden.

Besonders vorteilhaft wird die Umsetzung jedoch in der Weise durchgeführt, daß eine Verbindung der allgemeinen Formel II im Reaktionsgemisch durch Reduktion einer entsprechenden o-Nitro-aminoverbindung gegebenenfalls in Gegenwart einer Carbonsäure der allgemeinen Formel $R_3COOH$ oder durch Acylierung einer entsprechenden o-Diaminoverbindung hergestellt wird. Bei Abbruch der Reduktion der Nitrogruppe auf der Hydroxylaminstufe erhält man bei der anschließenden Cyclisierung das N-Oxid einer Verbindung der allgemeinen Formel I. Das so erhaltene N-Oxid wird anschließend mittels Reduktion in eine entsprechende Verbindung der allgemeinen Formel I übergeführt.

Die anschließende Reduktion des erhaltenen N-Oxids der Formel I wird vorzugsweise in einem Lösungsmittel wie Wasser, Wasser/-Äthanol, Methanol, Eisessig, Essigsäureäthylester oder Dimethylformamid mit Wasserstoff in Gegenwart eines Hydrierungskatalysators wie Raney-Nickel, Platin oder Palladium/Kohle, mit Metallen wie Eisen, Zinn oder Zink in Gegenwart einer Säure wie Essigsäure, Salzsäure oder Schwefelsäure, mit Salzen wie Eisen-(II)sulfat, Zinn(II)chlorid oder Natriumdithionit, oder mit Hydrazin in Gegenwart von Raney-Nickel bei Temperaturen zwischen 0 und 50°C, vorzugsweise jedoch bei Raumtemperatur durchgeführt.

b) Umsetzung eines Benzimidazols der allgemeinen Formel

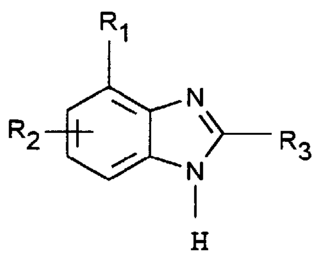

(III),

in der
$R_1$ bis $R_3$ wie eingangs definiert sind, mit einer Biphenylverbindung der allgemeinen Formel

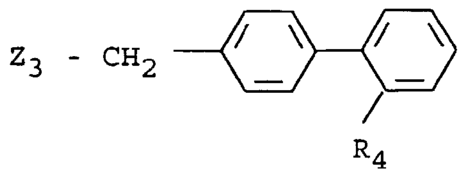

(IV),

in der
$R_4$ wie eingangs definiert ist und
$Z_3$ eine nukleophile Austrittsgruppe wie ein Halogenatom, z.B. ein Chlor-, Brom- oder Jodatom, oder eine substituierte Sulfonyloxygruppe, z.B. eine Methansulfonyloxy-, Phenylsulfonyloxy- oder p-Toluolsulfonyloxygruppe, darstellt.

Die Umsetzung wird zweckmäßigerweise in einem Lösungsmittel oder Lösungsmittelgemisch wie Methylenchlorid, Diethylether, Tetrahydrofuran, Dioxan, Dimethylsulfoxid, Dimethylformamid oder Benzol gegebenenfalls in Gegenwart eines säurebindenden Mittels, wie Natriumcarbonat, Kaliumcarbonat, Natriumhydroxid, Kalium-tert. butylat, Triethylamin oder Pyridin, wobei die beiden letzteren gleichzeitig auch als Lösungsmittel verwendet werden können, vorzugsweise bei Temperaturen zwischen 0 und 100°C, z.B. bei Temperaturen zwischen Raumtemperatur und 50°C, durchgeführt.

Bei der Umsetzung erhält man vorzugsweise ein Gemisch der 1-und 3-Isomeren, welches gewünschtenfalls anschließend, vorzugsweise chromatographisch unter Verwendung eines Trägers wie Kieselgel oder Aluminiumoxid, in das entsprechende 1- und 3-Isomere aufgetrennt wird.

c) Zur Herstellung einer Verbindung der allgemeinen Formel I, in der $R_4$ eine Carboxygruppe darstellt:

Überführung einer Verbindung der allgemeinen Formel

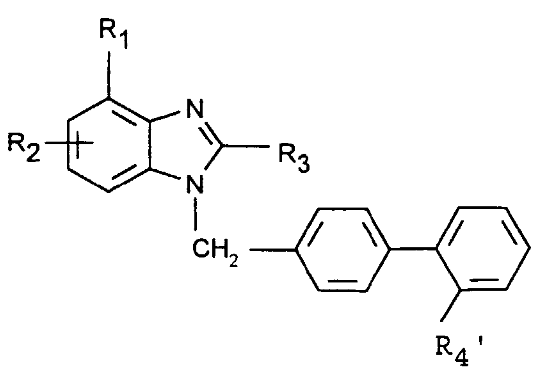

(V),

in der
$R_1$ bis $R_3$ wie eingangs definiert sind und
$R_4'$ eine mittels Hydrolyse, Thermolyse oder Hydrogenolyse in eine Carboxygruppe überführbare Gruppe darstellen.

Beispielsweise können funktionelle Derivate der Carboxygruppe wie deren unsubstituierte oder substituierte Amide, Ester, Thiolester, Orthoester, Iminoäther, Amidine oder Anhydride, die Nitrilgruppe oder die Tetrazolylgruppe mittels Hydrolyse in eine Carboxygruppe, Ester mit tertiären Alkoholen, z.B. der tert. Butylester, mittels Thermolyse in eine Carboxygruppe und Ester mit Aralkanolen, z.B. der Benzylester, mittels Hydrogenolyse in eine Carboxygruppe übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10°C und 120°C, z.B. bei Temperaturen zwischen Raumtemperatur und der Siedetemperatur des Reaktionsgemisches, durchgeführt. Bei der Hydrolyse in Gegenwart einer organischen Säuren wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet $R_4$' in einer Verbindung der allgemeinen Formel V eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen O und 50°C in die Carboxygruppe übergeführt werden.

Bedeutet $R_4$' in einer Verbindung der allgemeinen Formel V beispielsweise die tert. Butyloxycarbonylgruppe, so kann die tert.-Butylgruppe auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40°C und 100°C, abgespalten werden.

Bedeutet $R_4$' in einer Verbindung der allgemeinen Formel V beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid vorzugsweise bei Temperaturen zwischen 0 und 50°C, z.B. bei Raumtemperatur, und einem Wasserstoffdruck von 1 bis 5 bar abgespalten werden. Bei der Hydrogenolyse können gleichzeitig andere Reste, z.B. eine Nitrogruppe zur Aminogruppe, eine Benzyloxygruppe zur Hydroxygruppe, eine Vinylidengruppe zur entsprechenden Alkylidengruppe oder eine Zimtsäuregruppe zur entsprechenden Phenyl-propionsäuregruppe, mitreduziert oder durch Wasserstoffatome, z.B. ein Halogenatom durch ein Wasserstoffatom, ersetzt werden.

d) Zur Herstellung einer Verbindung der allgemeinen Formel I, in der $R_4$ eine IH-Tetrazolylgruppe darstellt:

Abspaltung eines Schutzrestes von einer Verbindung der allgemeinen Formel

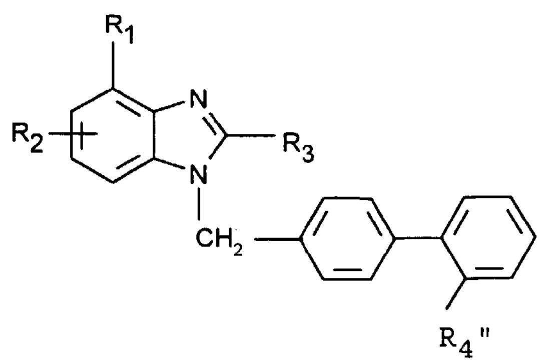

(VI),

in der

$R_1$, $R_2$ und $R_3$ wie eingangs definiert sind und

$R_4$" eine in 1- oder 3-Stellung durch einen Schutzrest geschützte IH-Tetrazolylgruppe darstellt.

Als Schutzrest kommt beispielsweise die Triphenylmethyl-, Tributylzinn- oder Triphenylzinngruppe in Betracht. Die Abspaltung eines verwendeten Schutzrestes erfolgt vorzugsweise in Gegenwart eines Halogenwasserstoffes, vorzugsweise in Gegenwart von Chlorwasserstoff, in Gegenwart einer Base wie Natriumhydroxid oder alkoholischem Ammoniak in einem geeigneten Lösungsmittel wie Methylenchlorid, Methanol, Methanol/Ammoniak, Ethanol oder Isopropanol bei Temperaturen zwischen 0 und 100°C, vorzugsweise jedoch bei Raumtemperatur, oder auch, falls die Umsetzung in Gegenwart von alkoholischem Ammoniak durchgeführt wird, bei erhöhten Temperaturen, z.B. bei Temperaturen zwischen 100 und 150°C, vorzugsweise bei Temperaturen zwischen 120 und 140°C.

e) Zur Herstellung einer Verbindung der allgemeinen Formel I, in der $R_4$ eine 1H-Tetrazolylgruppe darstellt:

Umsetzung einer Verbindung der allgemeinen Formel

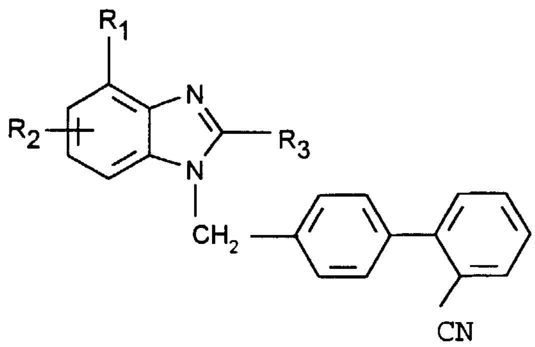

(VII),

in der
$R_1$ bis $R_3$ wie eingangs definiert sind, mit Stickstoffwasserstoffsäure oder deren Salzen.

Die Umsetzung wird vorzugsweise in einem Lösungsmittel wie Benzol, Toluol oder Dimethylformamid bei Temperaturen zwischen 80 und 150°C, vorzugsweise bei 125°C, durchgeführt. Hierbei wird zweckmäßigerweise entweder die Stickstoffwasserstoffsäure während der Umsetzung aus einem Alkaliazid, z.B. aus Natriumazid, in Gegenwart einer schwachen Säure wie Ammoniumchlorid freigesetzt oder das im Reaktionsgemisch bei der Umsetzung mit einem Salz der Stickstoffwassersäure, vorzugsweise mit Aluminiumazid oder Tributylzinnazid, welche außerdem zweckmäßigerweise im Reaktionsgemisch durch Umsetzung von Aluminiumchlorid oder Tributylzinnchlorid mit einem Alkaliazid wie Natriumazid hergestellt werden, erhaltene Tetrazolidsalz anschließend durch Ansäuern mit einer verdünnten Säure wie 2N Salzsäure oder 2N Schwefelsäure freigesetzt.

f) Zur Herstellung von Verbindungen der allgemeinen Formel I, in der $R_2$ eine der eingangs erwähnten Imidazo [1,2-a]pyridin2-yl-, Imidazo[1,2-a]pyrimidin-2-yl-, Imidazo[1,2-c]pyrimidin-2-yl-, Imidazo[1,2-a]pyrazin-2-yl-, Imidazo[1,2-b]pyridazin-2-yl- oder Imidazo[2,1-b]thiazol-6-yl-gruppen darstellt:

Umsetzung einer Verbindung der allgemeinen Formel

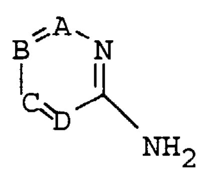

(VIII),

in der
einer der Reste A, B, C oder D eine gegebenenfalls durch eine Methylgruppe substitierte Methingruppe oder ein Stickstoffatom und
die übrigen der Reste A, B, C oder D Methingruppen oder A und B jeweils eine Methingruppe und die -C=D-Gruppe ein Schwefelatom bedeuten, mit einer Verbindung der allgemeinen Formel

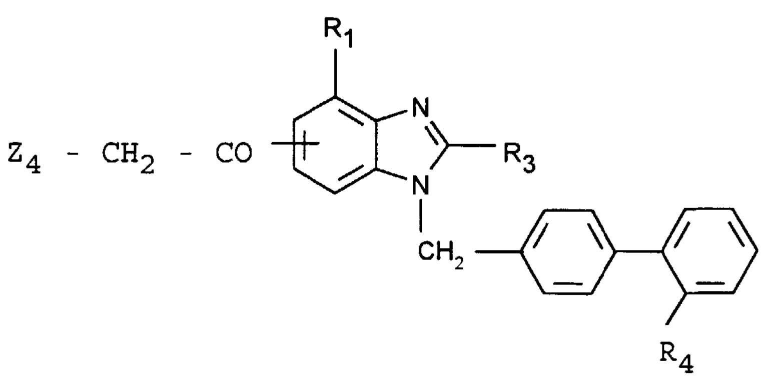

(IX),

in der
$R_1$, $R_3$ und $R_4$ wie eingangs definiert sind und
$Z_4$ eine nukleophile Austrittsgruppe wie ein Halogenatom, z.B. ein Chlor- oder Bromatom, darstellt.

Die Umsetzung wird zweckmäßigerweise in einem Lösungsmittel oder Lösungsmittelgemisch wie Ethanol,

Isopropanol, Benzol, Glykol, Glykolmonomethylether, Dimethylformamid oder Dioxan beispielsweise bei Temperaturen zwischen 0 und 150°C, vorzugsweise bei Temperaturen zwischen 20 und 100°C, durchgeführt. Die Umsetzung kann jedoch auch ohne Lösungsmittel durchgeführt werden.

g) Zur Herstellung von Verbindungen der allgemeinen Formel I, in der $R_2$ eine der eingangs erwähnten Benzimidazol-2-yl-, Imidazo[4,5-b]pyridin-2-yl-, Imidazo[4,5-c]pyridin-2-yl-, Imidazo[4,5-b]pyrazin-2-yl-, Imidazo[4,5-c]pyridazin-2-yl-, Imidazo[4,5-d]pyridazin-2-yl- oder Purin-8-yl-gruppen darstellt:

Cyclisierung einer Verbindung der allgemeinen Formel

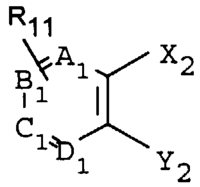

(X) ,

in der

null, einer oder zwei der Reste $A_1$, $B_1$, $C_1$ oder $D_1$ ein Stickstoffatom und
die verbleibenden Reste der Reste $A_1$, $B_1$, $C_1$ oder $D_1$ Methingruppen sowie
$R_{11}$ ein Wasserstoff- oder Fluoratom, eine Methyl- oder Trifluormethylgruppe,
einer der Reste $X_2$ oder $Y_2$ eine $R_{13}$-NH-Gruppe und der andere der Reste $X_2$ oder $Y_2$ eine Gruppe der allgemeinen Formel

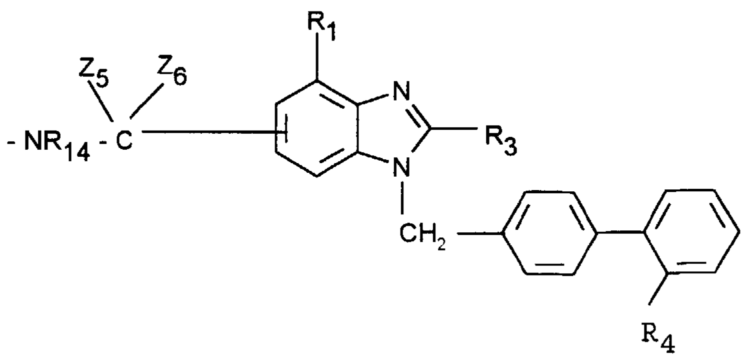

darstellen, wobei $R_1$, $R_3$ und $R_4$ wie eingangs definiert sind, einer der Reste $R_{13}$ oder $R_{14}$ ein Wasserstoffatom und der andere der Reste $R_{13}$ oder $R_{14}$ ein Wasserstoffatom, eine Alkylgruppe mit 1 bis 6 Kohlenstoffatomen oder eine Cycloalkylgruppe mit 3 bis 7 Kohlenstoffatomen,
$Z_5$ und $Z_6$, die gleich oder verschieden sein können, gegebenenfalls substituierte Aminogruppen oder gegebenenfalls durch niedere Alkylgruppen substituierte Hydroxy- oder Mercaptogruppen oder
$Z_5$ und $Z_6$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxyoder Alkylendithiogruppe mit jeweils 2 oder 3 Kohlenstoffatomen bedeuten, und gegebenenfalls anschließende Reduktion eines so erhaltenen entsprechenden N-Oxids und und gegebenenfalls anschließende Hydrolyse.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin oder in einem Überschuß des zur Herstellung der Verbindung der allgemeinen Formel X verwendeten Acylierungsmittel, z.B. in dem entsprechenden Nitril, Anhydrid, Säurehalogenid, Ester oder Amid, beispielsweise bei Temperaturen zwischen 0 und 250°C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Methansulfonsäure, Salzsäure, Phosphorsäure, Polyphosphorsäure, Essigsäureanhydrid oder gegebenenfalls auch in Gegenwart einer Base wie Kaliumäthylat oder Kaliumtert.-butylat durchgeführt. Die Cyclisierung kann jedoch auch ohne Lösungsmittel und/oder Kondensationsmittel durchgeführt werden.

Besonders vorteilhaft wird die Umsetzung jedoch in der Weise durchgeführt, daß eine Verbindung der allgemeinen

Formel X im Reaktionsgemisch durch Reduktion einer entsprechenden o-Nitroaminoverbindung gegebenenfalls in Gegenwart einer Carbonsäure der allgemeinen Formel

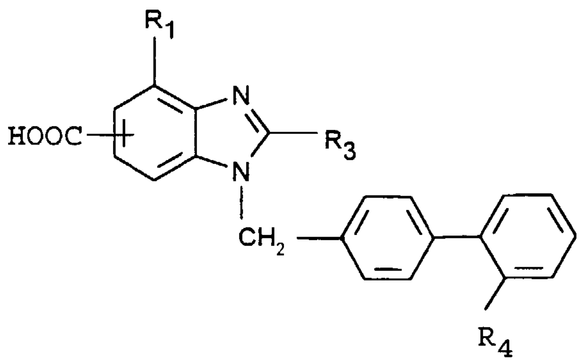

, (XI)

in der
$R_1$, $R_3$ und $R_4$ wie eingangs definiert sind, oder durch Acylierung einer entsprechenden o-Diaminoverbindung mit einer Carbonsäure der allgemeinen Formel XI hergestellt wird.

Bei Abbruch der Reduktion der Nitrogruppe auf der Hydroxylaminstufe erhält man bei der anschließenden Cyclisierung das N-Oxid einer Verbindung der allgemeinen Formel I. Das so erhaltene N-Oxid wird anschließend mittels Reduktion in eine entsprechende Verbindung der allgemeinen Formel I übergeführt.

Die anschließende Reduktion eines so erhaltenen N-Oxids wird vorzugsweise in einem Lösungsmittel wie Wasser, Wasser/Äthanol, Methanol, Eisessig, Essigsäureäthylester oder Dimethylformamid mit Wasserstoff in Gegenwart eines Hydrierungskatalysators wie Raney-Nickel, Platin oder Palladium/Kohle, mit Metallen wie Eisen, Zinn oder Zink in Gegenwart einer Säure wie Essigsäure, Salzsäure oder Schwefelsäure, mit Salzen wie Eisen(II)sulfat, Zinn(II)chlorid oder Natriumdithionit, oder mit Hydrazin in Gegenwart von Raney-Nickel bei Temperaturen zwischen 0 und 50°C, vorzugsweise jedoch bei Raumtemperatur durchgeführt.

Die anschließende Hydrolyse wird zweckmäßigerweise entweder in Gegenwart einer Säure wie Salzsäure, Schwefelsäure, Phosphorsäure, Trichloressigsäure oder Trifluoressigsäure in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10°C und 120°C, z.B. bei Temperaturen zwischen Raumtemperatur und der Siedetemperatur des Reaktionsgemisches, durchgeführt. Bei der Hydrolyse in Gegenwart einer organischen Säuren wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bei den vorstehend beschriebenen Umsetzungen können gegebenenfalls vorhandene reaktive Gruppen wie Hydroxy-, Amino- oder Alkylaminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.- Butyl-, Benzyl- oder Tetrahydropyranylgruppe und als Schutzrest für eine Amino-, Alkylamino- oder Iminogruppe die Acetyl-, Benzoyl-, Ethoxycarbonyl- oder Benzylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

Ein so erhaltenes Isomerengemisch einer Verbindung der allgemeinen Formel I kann gewünschtenfalls vorzugsweise chromatographisch unter Verwendung eines Trägers wie Kieselgel oder Aluminiumoxid getrennt werden.

Desweiteren können die erhaltenen Verbindungen der allgemeinen Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der allgemeinen Formel I, falls diese eine Carboxy- oder IH-Tetrazolylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der allgemeinen Formeln II bis XI sind teilweise literaturbekannt oder man erhält diese nach literaturbekannten Verfahren.

So erhält man beispielsweise eine Verbindung der allgemeinen Formel II durch Alkylierung einer entsprechenden o-Amino-nitroverbindung und anschließende Reduktion der Nitrogruppe.

Eine als Ausgangsstoff verwendete Verbindung der allgemeinen Formeln III, V, VI, VII, IX oder X erhält man durch Acylierung eines entsprechenden o-Phenylendiamins oder einer entsprechenden o-Amino-nitroverbindung, anschließender Reduktion der Nitrogruppe und anschließender Cyclisierung einer so erhaltenen o-Diaminophenylverbindung und gegebenenfalls anschließender Abspaltung eines verwendeten Schutzrestes oder durch Cyclisierung eines entsprechend substituierten Benzimidazols mit einem entsprechenden Amin oder durch NH-Alkylierung eines entsprechenden IH-Benzimidazols, wobei das so erhaltene Isomerengemisch anschließend mittels üblicher Methoden, z.B. mittels Chromatographie, aufgetrennt werden kann. Die vorstehend erwähnten Ausgangsverbindungen werden teilweise in der EP-A-0 392 317 beschrieben.

Beispielsweise erhält man 2-n-Propyl-5-(imidazo[1,2-a]pyridin-2-yl)-3H-benzimidazol durch Umsetzung von p-Amino-acetophenon mit Buttersäurechlorid, anschließender Nitrierung, Bromierung, Ringschluß mit 2-Aminopyridin zu dem 6-n-Butanoylamido-3-(imidazo[1,2-a]pyridin-2-yl)-nitrobenzol, anschließend nach Reduktion der Nitrogruppe mittels Cyclisierung in die gewünschte Verbindung übergeführt wird oder

2-n-Propyl-4-methyl-6-(1-methylbenzimidazol-2-yl)-1H-benzimidazol durch Nitrierung von 3-Methyl-4-n-butanoylamido-benzoesäuremethylester, anschließender Reduktion der Nitrogruppe und Cyclisierung zu 2-n-Butyl-4-methyl-6-methoxycarbonyl1H-benzimidazol, welches anschließend mit 2-Methylamino-anilin unter Cyclisierung in die gewünschte Verbindung übergeführt wird.

Die neuen Verbindungen der allgemeinen Formel I und deren physiologisch verträgliche Salze weisen wertvolle pharmakologische Eigenschaften auf. Sie stellen Angiotensin-Antagonisten, insbesondere Angiotensin-II-Antagonisten, dar.

Beispielsweise wurden die Verbindungen

A = 4'-[[2-n-Propyl-4-methyl-6-(1-methylbenzimidazol-2-yl)-benzimidazol-1-yl]-methyl]-biphenyl-2-carbonsäure,

B = 4'-[[2-n-Propyl-4-methyl-6-(1-methylbenzimidazol-2-yl)-benzimidazol-1-yl]-methyl]-2-(1H-tetrazol-5-yl)-biphenyl,

C = 4'-[[2-n-Propyl-4-methyl-6-(butansultam-1-yl)-benzimidazol-1-yl]-methyl]-2-(1H-tetrazol-5-yl)-biphenyl,

D = 4'-[[2-n-Butyl-6-(2,3-dimethylmaleinsäureimino)-4-methylbenzimidazol-1-yl]-methyl]-biphenyl-2-carbonsäure-semihydrat,

E = 4'-[(2-Cyclopropyl-4-methyl-6-(1-methylbenzimidazol2-yl)-benzimidazol-1-yl)-methyl]-biphenyl-2-carbonsäure,

F = 4'-[(2-n-Propyl-4-methyl-6-(1-methyl-5-fluor-benzimidazol-2-yl)-benzimidazol-1-yl)-methyl]-biphenyl-2-carbonsäure,

G = 4'-[(2-n-Propyl-4-methyl-6-(imidazo[1,2-a]pyrimidin-2-yl)-benzimidazol-1-yl)-methyl]-2-(1H-tetrazol-5-yl)-biphenyl,

H = 4'-[(2-n-Propyl-4-methyl-6-(5,6,7,8-tetrahydro-imidazo[1,2-a]pyridin-2-yl)-benzimidazol-1-yl)-methyl]-biphenyl-2-carbonsäure,

I = 4'-[(2-n-Propyl-4-methyl-6-(5,6,7,8-tetrahydro-imidazo-[1,2-a]pyridin-2-yl)-benzimidazol-1-yl)-methyl]-2-(1H-tetrazol-5-yl)-biphenyl,

J = 4'-[(2-n-Propyl-4-chlor-6-(1-methylbenzimidazol-2-yl)-benzimidazol-1-yl)-methyl]-2-(1H-tetrahol-5-yl)-biphenyl-hydrochlorid und

K = 4'-[[2-n-Propyl-4-methyl-6-(imidazo[2,1-b]thiazol-6-yl)-benzimidazol-1-yl]-methyl]-biphenyl-2-carbonsäure

auf ihre biologischen Wirkungen wie folgt untersucht:

Methodenbeschreibung Angiotensin II-Rezeptorbindung

Das Gewebe (Rattenlunge) wird in Tris-Puffer (50 mMol Tris, 150 mMol NaCl, 5 mMol EDTA, pH 7.40) homogenisiert und zweimal je 20 Min. bei 20.000 x g zentrifugiert. Das endgültige Pellet wird in Inkubations-Puffer (50 mMol Tris, 5 mMol $MgCl_2$, 0,2 % BSA, pH 7,40) 1:75, bezogen auf das Feuchtgewicht des Gewebes, resuspendiert. Je 0,1 ml Homogenat wird für 60 Min. bei 37°C mit 50 pM [$125^I$]-Angiotensin II (NEN, Dreieich, FRG) und steigenden Konzentrationen der Testsubstanz in einem Gesamtvolumen von 0,25 ml inkubiert. Die Inkubation wird durch rasche Filtration durch Glasfiber-Filtermatten beendet. Die Filter werden je 4 ml eiskaltem Puffer (25 mMol Tris, 2.5 mMol $MgCl_2$, 0,1 % BSA, pH 7,40) gewaschen. Die gebundene Radioaktivität wird in einem GammaCounter ermittelt. Aus der Dosis-Wirkungskurve wird der entsprechende $IC_{50}$-Wert ermittelt.

Die Substanzen A bis K zeigen in dem beschriebenen Test folgende $IC_{50}$-Werte:

| Substanz | $IC_{50}$ [nM] |
|---|---|
| A | 3,7 |
| B | 14,0 |
| C | 1,2 |
| D | 20,0 |
| E | 12,0 |
| F | 26,0 |
| G | 3,4 |
| H | 1,2 |
| I | 1,7 |
| J | 20,0 |
| K | 7,8 |

Zusätzlich wurden die Verbindungen A, B, C, D, E und G an wachen, renal hypertensiven Ratten auf ihre Wirkung nach oraler Gabe nach literaturbekannten Methoden getestet. Bei einer Dosis von 10 mg/kg zeigten diese Verbindungen eine blutdrucksenkende Wirkung.

Desweiteren konnten bei der Applikation der vorstehenden Verbindungen bis zu einer Dosis von 30 mg/kg i.v. keine toxischen Nebenwirkungen, z.B. keine negativ inotrope Wirkung und keine Herzrhythmusstörungen, beobachtet werden. Die Verbindungen sind demnach gut verträglich.

Aufgrund ihrer pharmakologischen Eigenschaften eignen sich die neuen Verbindungen und deren physiologisch verträgliche Salze zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen.

Weiterhin eignen sich die neuen Verbindungen und deren physiologisch verträgliche Salze zur Behandlung pulmonaler Erkrankungen, z.B. von Lungenödemen und der chronischen Bronchitis, zur Prävention von arterieller Re-Stenosis nach Angioplastie, von Verdickungen der Gefäßwand nach Gefäßoperationen, der Arteriosklerose und der diabetischen Angiopathie. Auf Grund der Beeinflußung der Acetylcholin- und Dopamin-Freisetzung durch Angiotensin im Gehirn eignen sich die neuen Angiotensin-Antagonisten auch zur Behebung zentralnervöser Störungen, z.B. von Depressionen, der Alzheimer'schen Krankheit, des Parkinson-Syndroms, der Bulimie, sowie von Störungen kognitiver Funktionen.

Die zur Erzielung einer entsprechenden Wirkung am Erwachsenen erforderliche Dosierung beträgt zweckmäßigerweise bei intravenöser Gabe 20 bis 100 mg, vorzugsweise 30 bis 70 mg, und bei oraler Gabe 50 bis 200 mg, vorzugsweise 75 bis 150 mg, jeweils 1 bis 3 x täglich. Hierzu lassen sich die erfindungsgemäß hergestellten Verbindungen der allgemeinen Formel I, gegebenenfalls in Kombination mit anderen Wirksubstanzen wie z.B. Blutdrucksenker, Diuretika und/oder Kalzium-Antagonisten, zusammen mit einem oder mehreren inerten üblichen Trägerstoffen

und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Für die oben erwähnten Kombinationen kommen somit als weitere Wirksubstanzen beispielsweise Bendroflumethiazid, Chlorthiazid, Hydrochlorthiazid, Spironolacton, Benzthiazid, Cyclothiazid, Ethacrinsäure, Furosemid, Metoprolol, Prazosin, Atenolol, Propranolol, (Di)hydralazin-hydrochlorid, Diltiazem, Felodipin, Nicardipin, Nifedipin, Nisoldipin und Nitrendipin in Betracht. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 15 bis 200 mg Hydrochlorthiazid, 125 bis 2000 mg Chlorthiazid, 15 bis 200 mg Ethacrinsäure, 5 bis 80 mg Furosemid, 20 bis 480 mg Propranolol, 5 bis 60 mg Felodipin, 5 bis 60 mg Nifedipin oder 5 bis 60 mg Nitrendipin.

Die nachfolgenden Beispiele sollen die Erfindung näher erläutern:

Beispiel A

4'-[[2-n-Butyl-7-[5-(imidazol-1-yl)-pentyloxy]-4-methylbenzimidazol-1-yl]-methyl]-biphenyl-2-carbonsäure-hydrat

0,7 g (1,15 mMol) 4'-[[2-n-Butyl-7-[5-(imidazol-1-yl)-pentyloxy]-4-methyl-benzimidazol-1-yl]-methyl]-biphenyl-2-carbonsäure-tert.butylester werden in 35 ml Methylenchlorid gelöst, 5 ml Trifluoressigsäure zugefügt und 12 Stunden bei Raumtemperatur gerührt. Man verdünnt mit Methylenchlorid und schüttelt mit Wasser und mit gesättigter Natriumbicarbonatlösung aus. Die organische Phase wird über Natriumsulfat getrocknet und im Vakuum eingeengt. Das so erhaltene Rohprodukt wird über eine Kieselgelsäule (Korngröße: 0,063-0,02 mm, Essigester/Ethanol/Ammoniak = 90: 10:0,1) gereinigt und aus Aceton kristallisiert.
Ausbeute: 0,19 g (29,9 % der Theorie),
Schmelzpunkt: 185-187°C
$C_{34}H_{38}N_4O_3$ x $H_2O$ (550,70)

| | | | | | | |
|---|---|---|---|---|---|---|
| Ber. | C | 71,81 | H | 7,09 | N | 9,85 |
| Gef. | | 72,03 | | 7,19 | | 9,71 |

Massenspektrum: m/e = $M^+$ 550

Beispiel 1

4'-[[2-n-Propyl-4-methyl-6-(1-methylbenzimidazol-2-yl)-benzimidazol-1-yl]-methyl]-biphenyl-2-carbonsäure

Hergestellt analog Beispiel A aus 4'-[[2-n-Propyl-4-methyl-6-(1-methylbenzimidazol-2-yl)-benzimidazol-1-yl]-methyl]-biphenyl-2-carbonsäure-tert.butylester und Trifluoressigsäure in Dimethylformamid.
Ausbeute: 63,9 % der Theorie,
Schmelzpunkt: 261-263°C
$C_{33}H_{30}N_4O_2$ (514,60)

| | | | | | | |
|---|---|---|---|---|---|---|
| Ber. | C | 77,02 | H | 5,87 | N | 10,89 |
| Gef. | | 76,90 | | 5,85 | | 10,99 |

Analog Beispiel 1 werden folgende Verbindungen erhalten:

4'-[[2-n-Propyl-4-methyl-6-(1-n-propylbenzimidazol-2-yl)-benzimidazol-1-yl]-methyl]-biphenyl-2-carbonsäure

4'-[[2-n-Propyl-4-methyl-6-(1-n-hexylbenzimidazol-2-yl)-benzimidazol-1-yl]-methyl]-biphenyl-2-carbonsäure

4'-[[2-n-Propyl-4-methyl-6-(1-cyclopropylbenzimidazol-2-yl)-benzimidazol-1-yl]-methyl]-biphenyl-2-carbonsäure

4'-[[2-n-Propyl-4-methyl-6-(1-cyclohexylbenzimidazol-2-yl)-benzimidazol-1-yl]-methyl]-biphenyl-2-carbonsäure

Beispiel 2

4'-[[2-n-Propyl-4-methyl-6-(1-methylbenzimidazol-2-yl)-benzimidazol-1-yl]-methyl]-2-(1H-tetrazol-5-yl)-biphenyl

Zu einer Lösung von 1,60 g (3,3 mMol) 4'-[[2-n-Propyl-4-methyl-6-(1-methylbenzimidazol-2-yl)-benzimidazol-1-yl]-methyl]-2-cyano-biphenyl in 50 ml Dimethylformamid werden 4,3 g (66 mMol) Natriumazid und 3,5 g (66 mMol) Ammoniumchlorid gegeben und das Gemisch 24 Stunden lang bei 140°C gerührt. Anschließend wird mit Wasser versetzt und der Niederschlag abgesaugt. Das so erhaltene Rohprodukt wird über Kieselgel (300 g Kieselgel, Methylenchlorid + 6 % Ethanol) chromatographisch gereinigt.
Ausbeute: 900 mg (51 % der Theorie),
Schmelzpunkt: 228-230°C
$C_{33}H_{30}N_8$ (538,70)

| | | | | | | |
|---|---|---|---|---|---|---|
| Ber. | C | 73,58 | H | 5,61 | N | 20,80 |
| Gef. | | 73,48 | | 5,55 | | 20,70 |

Analog Beispiel 2 werden folgende Verbindungen erhalten:

4'-[[2-n-Propyl-4-methyl-6-(1-n-hexylbenzimidazol-2-yl)-benzimidazol-1-yl]-methyl]-2-(1H-tetrazol-5-yl)-biphenyl

4'-[[2-n-Propyl-4-methyl-6-(1-cyclobutylbenzimidazol-2-yl)benzimidazol-1-yl]-methyl]-2-(1H-tetrazol-5-yl)-biphenyl

4'-[[2-n-Propyl-4-methyl-6-(1-cyclohexylbenzimidazol-2-yl)-benzimidazol-1-yl]-methyl]-2-(1H-tetrazol-5-yl)-biphenyl

Beispiel 3

4'-[[2-n-Propyl-4-methyl-6-(butansultam-1-yl)-benzimidazol-1-yl]-methyl]-2-(1H-tetrazol-5-yl)-biphenyl

Hergestellt analog Beispiel 2 aus 4'-[[2-n-Propyl-4-methyl-6-(butansultam-1-yl)-benzimidazol-1-yl]-methyl]-2-cyano-biphenyl und Natriumazid in Dimethylformamid.
Ausbeute: 49,0 % der Theorie,
Schmelzpunkt: ab 186°C sintern
$C_{29}H_{31}N_7O_2S$ (541,70)

| | | | | | | | | |
|---|---|---|---|---|---|---|---|---|
| Ber. | C | 64,30 | H | 5,77 | N | 18,10 | S | 5,92 |
| Gef. | | 64,10 | | 5,39 | | 18,01 | | 5,98 |

Beispiel 4

4'-[[2-Ethyl-4-methyl-6-(butansultam-1-yl)-benzimidazol-1-yl]-ethyl]-2-(1H-tetrazol-5-yl)-biphenyl

Hergestellt analog Beispiel 2 aus 4'-[[2-Ethyl-4-methyl-6-(butansultam-1-yl)-benzimidazol-1-yl]-methyl]-2-cyano-biphenyl und Natriumazid in Dimethylformamid.
Ausbeute: 60,0 % der Theorie,
Schmelzpunkt: amorph, ab 194°C sintern
$C_{28}H_{29}N_7O_2S$ (527,70)

| | | | | | | | | |
|---|---|---|---|---|---|---|---|---|
| Ber. | C | 63,74 | H | 5,54 | N | 18,58 | S | 6,08 |
| Gef. | | 63,83 | | 5,66 | | 18,41 | | 5,82 |

Beispiel 5

4'-[[2-n-Butyl-4-methyl-6-(butansultam -1-yl)-benzimidazol1-yl]-methyl]-2-(1H-tetrazol-5-yl)-biphenyl

Hergestellt analog Beispiel 2 aus 4'-[[2-n-Butyl-4-methyl-6-(butansultam-1-yl)-benzimidazol-1-yl]-methyl]-2-cyano-biphenyl und Natriumazid in Dimethylformamid.
Ausbeute: 48,0 % der Theorie,
Schmelzpunkt: amorph, ab 183°C sintern
$C_{30}H_{33}N_7O_2S$ (555,70)

| | | | | | | | | |
|---|---|---|---|---|---|---|---|---|
| Ber. | C | 64,84 | H | 5,99 | N | 17,64 | S | 5,77 |
| Gef. | | 64,53 | | 5,66 | | 17,63 | | 5,55 |

Beispiel 6

4'-[[2-n-Propyl-4-ethyl-6-(butansultam -1-yl)-benzimidazol-1-yl]-methyl]-2-(1H-tetrazol-5-yl)-biphenyl

Hergestellt analog Beispiel 2 aus 4'-[[2-n-Propyl-4-ethyl-6-(butansultam-1-yl)-benzimidazol-1-yl]-methyl]-2-cyano-biphenyl und Natriumazid in Dimethylformamid.
Ausbeute: 27,0 % der Theorie,
Schmelzpunkt: amorph, ab 189°C sintern
$C_{30}H_{33}N_7O_2S$ (555,70)

| | | | | | | | | |
|---|---|---|---|---|---|---|---|---|
| Ber. | C | 64,84 | H | 5,99 | N | 17,64 | S | 5,77 |
| Gef. | | 64,81 | | 5,68 | | 17,87 | | 5,31 |

Beispiel 7

4'-[[2-Ethyl-4-ethyl-6-(butansultam-1-yl)-benzimidazol-1-yl]-methyl]-2-(1H-tetrazol-5-yl)-biphenyl

Hergestellt analog Beispiel 2 aus 4'-[[2-Ethyl-4-ethyl-6-(butansultam-1-yl)-benzimidazol-1-yl]-methyl]-2-cyano-biphenyl und Natriumazid in Dimethylformamid.
Ausbeute: 39,0 % der Theorie,
Schmelzpunkt: amorph, ab 212°C sintern
$C_{29}H_{31}N_7O_2S$ (541,70)

| | | | | | | | | |
|---|---|---|---|---|---|---|---|---|
| Ber. | C | 64,30 | H | 5,77 | N | 18,10 | S | 5,92 |
| Gef. | | 64,30 | | 5,51 | | 17,99 | | 5,59 |

Beispiel 8

4'-[[2-n-Propyl-4-isopropyl-6-(butansultam-1-yl)-benzimidazol-1-yl]-methyl]-2-(1H-tetrazol-5-yl)-biphenyl

Hergestellt analog Beispiel 2 aus 4'-[[2-n-Propyl-4-isopropyl-6-(butansultam-1-yl)-benzimidazol-1-yl]-methyl]-2-cyanobiphenyl und Natriumazid in Dimethylformamid.
Ausbeute: 22,0 % der Theorie,
Schmelzpunkt: amorph
$C_{31}H_{35}N_7O_2S$ (569,70)

| | | | | | | | | |
|---|---|---|---|---|---|---|---|---|
| Ber. | C | 65,35 | H | 6,19 | N | 17,21 | S | 5,63 |
| Gef. | | 65,13 | | 6,10 | | 17,54 | | 5,40 |

Beispiel 9

4'-[[2-Ethyl-4-isopropyl-6-(butansultam-1-yl)-benzimidazol-1-yl]-methyl]-2-(1H-tetrazol-5-yl)-biphenyl

Hergestellt analog Beispiel 2 aus 4'-[[2-Ethyl-4-isopro pyl-6-(butansultam-1-yl)-benzimidazol -1-yl]-methyl]-2-cyanobiphenyl und Natriumazid in Dimethylformamid.
Ausbeute: 24,0 % der Theorie,
Schmelzpunkt: amorph, ab 209°C sintern
$C_{30}H_{33}N_7O_2S$ (555,70)

| | | | | | | | | |
|---|---|---|---|---|---|---|---|---|
| Ber. | C | 64,84 | H | 5,99 | N | 17,64 | S | 5,77 |
| Gef. | | 64,99 | | 5,71 | | 17,43 | | 5,71 |

Beispiel 10

4'-[[2-n-Propyl-4-trifluormethyl-6-(butansultam-1-yl)-benzimidazol-1-yl]-methyl]-2-(1H-tetrazol-5-yl)-biphenyl

Hergestellt analog Beispiel 2 aus 4'-[[2-n-Propyl-4-trifluormethyl-6-(butansultam-1-yl)-benzimidazol-1-yl]-methyl]-2-cyano-biphenyl und Natriumazid in Dimethylformamid. Ausbeute: 17,0 % der Theorie,
Schmelzpunkt: 199-203°C
$C_{29}H_{28}F_3N_7O_2S$ (595,70)

| | | | | | | |
|---|---|---|---|---|---|---|
| Ber. | C | 58,48 | H | 4,74 | N | 16,46 |
| Gef. | | 58,28 | | 4,43 | | 16,22 |

Beispiel 11

4'-[[2-n-Butyl-4-methyl-6-(1-methylbenzimidazol-2-yl)-benzimidazol-1-yl]-methyl]-biphenyl-2-carbonsäure

Hergestellt analog Beispiel A aus 4'-[[2-n-Butyl-4-methyl-6-(1-methylbenzimidazol-2-yl)-benzimidazol-1-yl]-methyl]-biphenyl-2-carbonsäure-tert.butylester und Trifluoressigsäure in Methylenchlorid.
Ausbeute: 48,0 % der Theorie,
Schmelzpunkt: 233-235°C
$C_{34}H_{32}N_4O_2$ (528,70)

| | | | | | | |
|---|---|---|---|---|---|---|
| Ber. | C | 77,25 | H | 6,10 | N | 10,60 |
| Gef. | | 77,10 | | 5,98 | | 10,46 |

Beispiel 12

4'-[[2-n-Butyl-4-methyl-6-(1-methylbenzimidazol-2-yl)-benzimidazol-1-yl]-methyl]-2-(1H-tetrazol-5-yl)-biphenyl

Hergestellt analog Beispiel 2 aus 4'-[[2-n-Butyl-4-methyl-6-(1-methylbenzimidazol-2-yl)-benzimidazol-1-yl]-methyl]-2-cyano-biphenyl und Natriumazid in Dimethylformamid.
Ausbeute: 41,0 % der Theorie,
Schmelzpunkt: 235-237°C
$C_{34}H_{32}N_8$ (552,70)

| | | | | | | |
|---|---|---|---|---|---|---|
| Ber. | C | 73,89 | H | 5,84 | N | 20,28 |
| Gef. | | 73,67 | | 5,81 | | 19,93 |

Analog Beispiel 12 werden folgende Verbindungen erhalten:

4'-[[2-n-Butyl-4-methyl-6-(1-ethylbenzimidazol-2-yl)-benzimidazol-1-yl]-methyl]-2-(1H-tetrazol-5-yl)-biphenyl

4'-[[2-n-Butyl-4-methyl-6-(1-cyclopropylbenzimidazol-2-yl) benzimidazol-1-yl]-methyl]-2-(1H-tetrazol-5-yl)-biphenyl

4'-[[2-n-Butyl-4-methyl-6-(1-n-pentylbenzimidazol-2-yl)-benzimidazol-1-yl]-methyl]-2-(1H-tetrazol-5-yl)-biphenyl

4'-[[2-n-Butyl-4-methyl-6-(1-cyclopentylbenzimidazol-2-yl)-benzimidazol-1-yl]-methyl]-2-(1H-tetrazol-5-yl)-biphenyl

Beispiel 13

4'-[[2-n-Propyl-4-methyl-6-(2-oxo-piperidin-1-yl)-benzimidazol-1-yl]-methyl]-2-(1H-tetrazol-5-yl)-biphenyl

Hergestellt analog Beispiel 2 aus 4'-[[2-n-Propyl-4-methyl-6-(2-oxo-piperidin-1-yl)-benzimidazol-1-yl]-methyl]-2-cyano-biphenyl und Natriumazid in Dimethylformamid.
Ausbeute: 51,0 % der Theorie,
Schmelzpunkt: amorph, ab 140°C sintern
$C_{30}H_{31}N_7O$ (505,60)

| | | | | | | |
|---|---|---|---|---|---|---|
| Ber. | C | 71,26 | H | 6,18 | N | 19,39 |
| Gef. | | 71,08 | | 6,22 | | 19,47 |

Beispiel 14

4'-[[2-n-Butyl-4-methyl-6-(2-oxo-piperidin-1-yl)-benzimidazol-1-yl]-methyl]-2-(1H-tetrazol-5-yl)-biphenyl

Hergestellt analog Beispiel 2 aus 4'-[[2-n-Butyl-4-methyl-6-(2-oxo-piperidin-1-yl)-benzimidazol-1-yl]-methyl]-2-cyano-biphenyl und Natriumazid in Dimethylformamid.
Ausbeute: 39,0 % der Theorie,
Schmelzpunkt: amorph, ab 128°C sintern
$C_{31}H_{33}N_7O$ (519,70)

| | | | | | | |
|---|---|---|---|---|---|---|
| Ber. | C | 71,65 | H | 6,40 | N | 18,87 |
| Gef. | | 71,44 | | 6,23 | | 18,59 |

Beispiel 15

4'-[[2-n-Propyl-4-methyl-6-(2-oxo-piperidin-1-yl)-benzimidazol-1-yl]-methyl]-2-(1H-tetrazol-5-yl)-biphenyl

Hergestellt aus 4'-[[2-n-Propyl-4-methyl-6-(2-oxo-piperidin-1-yl)-benzimidazol-1-yl]-methyl]-2-(2-triphenylmethyl-tetrazol-5-yl)-biphenyl durch Abspaltung der Triphenylmethylgruppe mittels methanolischer Salzsäure.
Ausbeute: 51,0 % der Theorie,
Schmelzpunkt: amorph, ab 115°C sintern
$C_{30}H_{31}N_7O$ (505,60)

| | | | | | | |
|---|---|---|---|---|---|---|
| Ber. | C | 71,26 | H | 6,18 | N | 19,39 |
| Gef. | | 71,51 | | 6,39 | | 19,09 |

Beispiel 16

4'-[[2-n-Propyl-4-methyl-6-(imidazo[1,2-a]pyridin-2-yl)-benzimidazol-1-yl]-methyl]-biphenyl-2-carbonsäure

Hergestellt analog Beispiel A aus 4'-[[2-n-Propyl-4-methyl-6-(imidazo[1,2-a]pyridin-2-yl)-benzimidazol-1-yl]-methyl]-biphenyl-2-carbonsäure-tert.butylester und Trifluoressigsäure in Methylenchlorid.
Ausbeute: 38,0 % der Theorie,
Schmelzpunkt: 195-197°C (nach Abdampfen des Lösungsmittels)
Schmelzpunkt: 299-303°C (Methylenchlorid/Ethanol = 20:1)
$C_{32}H_{28}N_4O_2$ (500,60)

| | | | | | | |
|---|---|---|---|---|---|---|
| Ber. | C | 76,78 | H | 5,64 | N | 11,19 |
| Gef. | | 76,55 | | 5,61 | | 10,87 |

Beispiel 17

4'-[[2-n-Propyl-4-methyl-6-(imidazo[1,2-a]pyridin-2-yl)-benzimidazol-1-yl]-methyl]-2-(1H-tetrazol-5-yl)-biphenyl

Hergestellt analog Beispiel 2 aus 4'-[[2-n-Propyl-4-methyl-6-(imidazo[1,2-a]pyridin-2-yl)-benzimidazol-1-yl]-methyl]-2-cyano-biphenyl und Natriumazid in Dimethylformamid. Ausbeute: 21,0 % der Theorie,
Schmelzpunkt: ab 181°C sintern
$C_{32}H_{28}N_8$ (524,60)

| | | | | | | |
|---|---|---|---|---|---|---|
| Ber. | C | 73,26 | H | 5,38 | N | 21,36 |
| Gef. | | 73,10 | | 5,24 | | 21,13 |

Analog Beispiel 17 wird folgende Verbindung erhalten:

4'-[[2-n-Propyl-4-methyl-6-(imidazo[1,2-a]pyrimidin-2-yl) benzimidazol-1-yl]-methyl]-2-(1H-tetrazol-5-yl)-biphenyl

Beispiel 18

4'-[[2-n-Butyl-4-methyl-6-(imidazo[1,2-a]pyridin-2-yl)-benzimidazol-1-yl]-methyl]-biphenyl-2-carbonsäure

Hergestellt analog Beispiel A aus 4'-[[2-n-Butyl-4-methyl-6-(imidazo[1,2-a]pyridin-2-yl)-benzimidazol-1-yl]-methyl]-biphenyl-2-carbonsäure-tert.butylester und Trifluoressigsäure in Methylenchlorid.
Ausbeute: 51,0 % der Theorie,
Schmelzpunkt: 194-197°C
$C_{33}H_{30}N_4O_2$ (514,60)

| | | | | | | |
|---|---|---|---|---|---|---|
| Ber. | C | 77,02 | H | 5,88 | N | 10,89 |
| Gef. | | 76,81 | | 5,78 | | 10,64 |

Beispiel 19

4'-[[2-n-Butyl-4-methyl-6-(imidazo[1,2-a]pyridin-2-yl)-benzimidazol-1-yl]-methyl]-2-(1H-tetrazol-5-yl)-biphenyl

Hergestellt analog Beispiel 2 aus 4'-[[2-n-Butyl-4-methyl-6-(imidazo[1,2-a]pyridin-2-yl)-benzimidazol-1-yl]-methyl]-2-cyano-biphenyl und Natriumazid in Dimethylformamid. Ausbeute: 26,0 % der Theorie,
$C_{33}H_{30}N_8$ (538,60)

| | | | | | | |
|---|---|---|---|---|---|---|
| Ber. | C | 73,58 | H | 5,61 | N | 20,80 |
| Gef. | | 73,39 | | 5,40 | | 20,92 |

Beispiel 20

4'-[[2-n-Propyl-4-methyl-6-(imidazo[1,2-a]pyrimidin-2-yl)-benzimidazol-1-yl]-methyl]-biphenyl-2-carbonsäure

Hergestellt analog Beispiel A aus 4'-[[2-n-Propyl-4-methyl-6-(imidazo[1,2-a]pyrimidin-2-yl)-benzimidazol-1-yl]-methyl]-biphenyl-2-carbonsäure-tert.butylester und Trifluoressigsäure in Methylenchlorid.
Ausbeute: 47 % der Theorie,
Schmelzpunkt: 224-226°C (nach Abdampfen des Lösungsmittels)
Schmelzpunkt: 294-297°C (Methylenchlorid/Ethanol = 20:1)
$C_{31}H_{27}N_5O_2$ (501,60)

| | | | | | | |
|---|---|---|---|---|---|---|
| Ber. | C | 74,23 | H | 5,43 | N | 13,96 |
| Gef. | | 74,10 | | 5,31 | | 13,66 |

Beispiel 21

4'-[[2-n-Propyl-4-methyl-6-(imidazo[2,1-b]thiazol-6-yl)-benzimidazol-1-yl]-methyl]-biphenyl-2-carbonsäure

Hergestellt analog Beispiel A aus 4'-[[2-n-Propyl-4-methyl-6-(imidazo[2,1-b]thiazol-6-yl)-benzimidazol-1-yl]-methyl]-biphenyl-2-carbonsäure-tert.butylester und Trifluoressigsäure in Methylenchlorid.
Ausbeute: 43 % der Theorie,
Schmelzpunkt: 192-195°C (nach Abdampfen des Lösungsmittels)
Schmelzpunkt: >300°C (Methylenchlorid/Ethanol = 20:1)
$C_{30}H_{26}N_4O_2S$ (506,64)

| | | | | | | | | |
|---|---|---|---|---|---|---|---|---|
| Ber. | C | 71,12 | H | 5,17 | N | 11,06 | S | 6,33 |
| Gef. | | 70,97 | | 5,19 | | 10,88 | | 6,09 |

Beispiel 22

4'-[[2-n-Propyl-4-methyl-6-(imidazo [2,1-b]thiazol-6-yl)-benzimidazol-1-yl]-methyl]-2-(1H-tetrazol-5-yl)-biphenyl

Hergestellt analog Beispiel 2 aus 4'-[[2-n-Propyl-4-methyl-6-(imidazo [2,1-b]thiazol-6-yl)-benzimidazol-1-yl]-methyl]-2-cyano-biphenyl und Natriumazid in Dimethylformamid.
Ausbeute: 21 % der Theorie,
Schmelzpunkt: amorph, ab 196°C sintern
$C_{30}H_{26}N_8S$ (530,67)

| | | | | | | | | |
|---|---|---|---|---|---|---|---|---|
| Ber. | C | 67,90 | H | 4,94 | N | 21,12 | S | 6,04 |
| Gef. | | 67,77 | | 4,84 | | 21,00 | | 5,87 |

Beispiel 23

4'-[[2-n-Propyl-4-methyl-6-(benzimidazol-2-yl)-benzimidazol-1-yl]-methyl]-2-(1H-tetrazol-5-yl)-biphenyl

Hergestellt analog Beispiel 2 aus 4'-[[2-n-Propyl-4-methyl-6-(benzimidazol-2-yl)-benzimidazol-1-yl]-methyl]-2-cyano-biphenyl und Natriumazid in Dimethylformamid.
Ausbeute: 28 % der Theorie,
Schmelzpunkt: 202-205°C
$C_{32}H_{28}N_8$ (524,64)

| | | | | | | |
|---|---|---|---|---|---|---|
| Ber. | C | 73,26 | H | 5,38 | N | 21,36 |
| Gef. | | 73,01 | | 5,22 | | 21,56 |

Analog Beispiel 23 werden folgende Verbindungen erhalten:

4'-[[2-Ethyl-4-methyl-6-(benzimidazol-2-yl)-benzimidazol-1-yl]-methyl]-2-(1H-tetrazol-5-yl)-biphenyl

4'-[[2-n-Butyl-4-methyl-6-(benzimidazol-2-yl)-benzimidazol-1-yl]-methyl]-2-(1H-tetrazol-5-yl)-biphenyl

4'-[[2-n-Propyl-4-methyl-6-(1-n-hexyl-benzimidazol-2-yl)-benzimidazol-1-yl]-methyl]-2-(1H-tetrazol-5-yl)-biphenyl

4'-[[2-n-Propyl-4-methyl-6-(1-cyclopropyl-benzimidazol-2-yl)-benzimidazol-1-yl]-methyl]-2-(1H-tetrazol-5-yl)-biphenyl

4'-[[2-n-Propyl-4-methyl-6-(1-cyclohexyl-benzimidazol-2-yl)-benzimidazol-1-yl]-methyl]-2-(1H-tetrazol-5-yl)-bi-

phenyl

Beispiel 24

4'-[[2-n-Propyl-4-methyl-6-(benzimidazol-2-yl)-benzimidazol-1-yl]-methyl]-biphenyl-2-carbonsäure

Hergestellt analog Beispiel A aus 4'-[[2-n-Propyl-4-methyl-6-(benzimidazol-2-yl)-benzimidazol-1-yl]-methyl]-biphenyl-2-carbonsäure-tert.butylester und Trifluoressigsäure in Methylenchlorid.
Ausbeute: 43 % der Theorie,
Schmelzpunkt: 239-242°C
$C_{32}H_{28}N_4O_2$ (500,61)

| | | | | | | |
|---|---|---|---|---|---|---|
| Ber. | C | 76,78 | H | 5,64 | N | 11,19 |
| Gef. | | 76,55 | | 5,60 | | 11,41 |

Analog Beispiel 24 werden folgende Verbindungen erhalten:

4'-[[2-Ethyl-4-methyl-6-(benzimidazol-2-yl)-benzimidazol-1-yl]-methyl]-biphenyl-2-carbonsäure

4'-[[2-n-Butyl-4-methyl-6-(benzimidazol-2-yl)-benzimidazol-1-yl]-methyl]-biphenyl-2-carbonsäure

4'-[[2-n-Propyl-4-methyl-6-(1-n-hexyl-benzimidazol-2-yl)-benzimidazol-1-yl]-methyl]-biphenyl-2-carbonsäure

4'-[[2-n-Propyl-4-methyl-6-(1-cyclopropyl-benzimidazol-2-yl)-benzimidazol-1-yl]-methyl]-biphenyl-2-carbonsäure

4'-[[2-n-Propyl-4-methyl-6-(1-cyclohexyl-benzimidazol-2-yl)-benzimidazol-1-yl]-methyl]-biphenyl-2-carbonsäure

Beispiel 25

4'-[[2-n-Butyl-6-(2,3-dimethylmaleinsäureimino)-4-methyl-benzimidazol-1-yl]-methyl]-biphenyl-2-carbonsäure-semihydrat

Hergestellt analog Beispiel A aus 4'-[[2-n-Butyl-6-(2,3-di-methylmaleinsäureimino) -4-methyl-benz-imidazol-1-yl] -methyl]-biphenyl-2-carbonsäure-tert.butylester und Trifluoressigsäure in Methylenchlorid.
Ausbeute: 88,9 % der Theorie,
Schmelzpunkt: 321-322°C
$C_{32}H_{31}N_3O_4$ x 0,5 $H_2O$ (530,62)

| | | | | | | |
|---|---|---|---|---|---|---|
| Ber. | C | 72,43 | H | 6,08 | N | 7,92 |
| Gef. | | 72,89 | | 6,16 | | 7,89 |

Beispiel 26

4'-[[6-(2,3-Dimethylmaleinsäureimino)-2-n-propyl-4-methylbenzimidazol-1-yl]-methyl]-biphenyl-2-carbonsäure-semihydrat

Hergestellt analog Beispiel A aus 4'-[[6-(2,3-Dimethylmaleinsäureimino)-2-n-propyl-4-methyl-benzimidazol-1-yl]-methyl]-biphenyl-2-carbonsäure-tert.butylester und Trifluoressigsäure in Methylenchlorid.
Ausbeute: 75,4 % der Theorie,
Schmelzpunkt: 329-331°C
$C_{31}H_{29}N_3O_4$ x 0,5 $H_2O$ (516,60)

| | | | | | | |
|---|---|---|---|---|---|---|
| Ber. | C | 72,08 | H | 5,85 | N | 8,13 |
| Gef. | | 72,04 | | 5,84 | | 7,96 |

Beispiel 27

4'-[(2-n-Propyl-4-ethyl-6-(1-methylbenzimidazol-2-yl)-benzimidazol-1-yl)-methyl]-biphenyl-2-carbonsäure

Hergestellt analog Beispiel A aus 4'-[(2-n-Propyl-4-ethyl-6-(1-methylbenzimidazol-2-yl)-benzimidazol-1-yl)-methyl]-bi-phenyl-2-carbonsäure-tert.butylester und Trifluoressigsäure in Methylenchlorid.
Ausbeute: 64 % der Theorie,
Schmelzpunkt: 217-219°C
$C_{34}H_{32}N_4O_2$ (528,70)

| | | | | | | |
|---|---|---|---|---|---|---|
| Ber. | C | 77,24 | H | 6,10 | N | 10,60 |
| Gef. | | 77,12 | | 6,09 | | 10,75 |

Beispiel 28

4'-[(2-n-Propyl-4-ethyl-6-(1-methylbenzimidazol-2-yl)-benzimidazol-1-yl)-methyl]-2-(1H-tetrazol-5-yl)-biphenyl

Hergestellt analog Beispiel 2 aus 4'-[(2-n-Propyl-4-ethyl-6-(1-methylbenzimidazol-2-yl)-benzimidazol-1-yl)-methyl]-2-cyano-biphenyl und Natriumazid in Dimethylformamid.
Ausbeute: 15 % der Theorie,
Schmelzpunkt: 215-217°C
$C_{34}H_{32}N_8$ (552,70)

| | | | | | | |
|---|---|---|---|---|---|---|
| Ber. | C | 73,89 | H | 5,84 | N | 20,28 |
| Gef. | | 73,66 | | 6,02 | | 20,56 |

Beispiel 29

4'-[(2-Cyclopropyl-4-methyl-6-(1-methylbenzimidazol-2-yl)-benzimidazol-1-yl)-methyl]-biphenyl-2-carbonsäure

Hergestellt analog Beispiel A aus 4'-[(2-Cyclopropyl-4-methyl-6-(1-methylbenzimidazol-2-yl)-benzimidazol-1-yl)-methyl]-biphenyl-2-carbonsäure-tert.butylester und Trifluoressigsäure in Methylenchlorid.
Ausbeute: 52 % der Theorie,
Schmelzpunkt: 244-246°C
$C_{33}H_{28}N_4O_2$ (512,60)

| | | | | | | |
|---|---|---|---|---|---|---|
| Ber. | C | 77,32 | H | 5,51 | N | 10,93 |
| Gef. | | 77,75 | | 5,71 | | 10,94 |

Beispiel 30

4'-[(2-Cyclopropyl-4-methyl-6-(1-methylbenzimidazol-2-yl)-benzimidazol-1-yl)-methyl]-2-(1H-tetrazol-5-yl)-biphenyl

Hergestellt analog Beispiel 2 aus 4'-[(2-Cyclopropyl-4-methyl-6-(1-methylbenzimidazol-2-yl)-benzimidazol-1-yl)-methyl]-2-cyano-biphenyl und Natriumazid in Dimethylformamid. Ausbeute: 59 % der Theorie,
Schmelzpunkt: 245-247°C
$C_{33}H_{28}N_8$ (536,65)

| | | | | | | |
|---|---|---|---|---|---|---|
| Ber. | C | 73,86 | H | 5,26 | N | 20,88 |
| Gef. | | 73,95 | | 5,42 | | 20,90 |

Beispiel 31

4'-[(2-Cyclobutyl-4-methyl-6-(1-methylbenzimidazol-2-yl)-benzimidazol-1-yl)-methyl]-biphenyl-2-carbonsäure

Hergestellt analog Beispiel A aus 4'-[(2-Cyclobutyl-4-methyl-6-(1-methylbenzimidazol-2-yl)-benzimidazol-1-yl)-

methyl]-biphenyl-2-carbonsäure-tert.butylester und Trifluoressigsäure in Methylenchlorid.
Ausbeute: 63 % der Theorie,
Schmelzpunkt: 189-191°C
$C_{34}H_{30}N_4O_2$ (526,60)

| | | | | | | |
|---|---|---|---|---|---|---|
| Ber. | C | 77,55 | H | 5,74 | N | 10,64 |
| Gef. | | 77,35 | | 5,92 | | 10,40 |

Beispiel 32

4'-[(2-Cyclobutyl-4-methyl-6-(1-methylbenzimidazol-2-yl)-benzimidazol-1-yl)-methyl]-2-(1H-tetrazol-5-yl)-biphenyl

Hergestellt analog Beispiel 2 aus 4'-[(2-Cyclobutyl-4-methyl-6-(1-methylbenzimidazol-2-yl)-benzimidazol-1-yl)-methyl]-2-cyano-biphenyl und Natriumazid in Dimethylformamid.
Ausbeute: 61 % der Theorie,
Schmelzpunkt: 197-199°C
$C_{34}H_{30}N_8$ (550,70)

| | | | | | | |
|---|---|---|---|---|---|---|
| Ber. | C | 74,16 | H | 5,49 | N | 20,35 |
| Gef. | | 74,12 | | 5,74 | | 20,67 |

Beispiel 33

4'-[(2-n-Propyl-4-methyl-6-(1-methyl-5-fluor-benzimidazol-2-yl)-benzimidazol-1-yl)-methyl]-biphenyl-2-carbonsäure

Hergestellt analog Beispiel A aus 4'-[(2-n-Propyl-4-methyl-6-(1-methyl-5-fluor-benzimidazol-2-yl)-benzimidazol-1-yl)-methyl]-biphenyl-2-carbonsäure-tert.butylester und Trifluoressigsäure in Methylenchlorid.
Ausbeute: 34 % der Theorie,
Schmelzpunkt: 250-252°C
$C_{33}H_{29}FN_4O_2$ (532,60)

| | | | | | | |
|---|---|---|---|---|---|---|
| Ber. | C | 74,42 | H | 5,49 | N | 10,52 |
| Gef. | | 74,14 | | 5,64 | | 10,54 |

Analog Beispiel 33 wird folgende Verbindung erhalten:

4'-[(2-n-Propyl-4-methyl-6-(pyridin-2-yl)-benzimidazol-1-yl)-methyl]-biphenyl-2-carbonsäure

Beispiel 34

4'-[(2-n-Propyl-4-methyl-6-(imidazo[1,2-a]pyrimidin-2-yl)-benzimidazol-1-yl)-methyl]-2-(1H-tetrazol-5-yl)-biphenyl

Hergestellt analog Beispiel 2 aus 4'-[(2-n-Propyl-4-methyl-6-(imidazo[1,2-a]pyrimidin-2-yl)-benzimidazol-1-yl)-methyl]-2-cyano-biphenyl und Natriumazid in Dimethylformamid.
Ausbeute: 16,5 % der Theorie,
Schmelzpunkt: ab 275°C (Zers.)
$C_{31}H_{27}N_9 \times H_2O$ (543,65)

| | | | | | | |
|---|---|---|---|---|---|---|
| Ber. | C | 68,49 | H | 5,38 | N | 23,19 |
| Gef. | | 68,25 | | 5,50 | | 23,37 |

Analog Beispiel 34 werden folgende Verbindungen erhalten:

4'-[(2-n-Propyl-4-methyl-6-(pyridin-2-yl)-benzimidazol-1-yl)-methyl]-2-(1H-tetrazol-5-yl)-biphenyl

Beispiel 35

4'-[(2-n-Propyl-4-methyl-6-(5,6,7,8-tetrahydro-imidazo[1,2-a]-pyridin-2-yl)-benzimidazol-1-yl)-methyl]-biphenyl-2-carbon-säure

Hergestellt analog Beispiel A aus 4'-[(2-n-Propyl-4-methyl-6-(5,6,7,8-tetrahydro-imidazo[1,2-a]-pyridin-2-yl)-benzimidazol-1-yl)-methyl]-biphenyl-2-carbonsäure-tert.butylester und Trifluoressigsäure in Methylenchlorid.
Ausbeute: 67 % der Theorie,
Schmelzpunkt: ab 240°C (sintern)
$C_{32}H_{32}N_4O_2$ (504,64)

| | | | | | | |
|---|---|---|---|---|---|---|
| Ber. | C | 76,16 | H | 6,39 | N | 11,10 |
| Gef. | | 75,94 | | 6,46 | | 11,20 |

Analog Beispiel 35 werden folgende Verbindungen erhalten:

4'-[(2-n-Butyl-4-methyl-6-(5,6,7,8-tetrahydro-imidazo[1,2-a]-pyridin-2-yl)-benzimidazol-1-yl)-methyl]-biphenyl-2-carbonsäure

4'-[(2-Ethyl-4-methyl-6-(5,6,7,8-tetrahydro-imidazo[1,2-a]-pyridin-2-yl)-benzimidazol-1-yl)-methyl]-biphenyl-2-carbonsäure

Beispiel 36

4'-[(2-n-Propyl-4-methyl-6-(5,6,7,8-tetrahydro-imidazo[1,2-a]-pyridin-2-yl)-benzimidazol-1-yl)-methyl]-2-(1H-tetrazol-5-yl)-biphenyl

Hergestellt analog Beispiel 2 aus 4'-[(2-n-Propyl-4-methyl-6-(5,6,7,8-tetrahydro-imidazo[1,2-a]-pyridin-2-yl)-benzimidazol-1-yl)-methyl]-2-cyano-biphenyl und Natriumazid in Dimethylformamid.
Ausbeute: 73,5 % der Theorie,
Schmelzpunkt: ab 275°C (Zers.)
$C_{32}H_{32}N_8$ (528,67)

| | | | | | | |
|---|---|---|---|---|---|---|
| Ber. | C | 72,70 | H | 6,10 | N | 21,20 |
| Gef. | | 72,40 | | 6,07 | | 21,48 |

Analog Beispiel 36 werden folgende Verbindungen erhalten:

4'-[(2-n-Butyl-4-methyl-6-(5,6,7,8-tetrahydro-imidazo[1,2-a]-pyridin-2-yl)-benzimidazol-1-yl)-methyl]-2-(1H-tetrazol-5-yl)-biphenyl

4'-[(2-Ethyl-4-methyl-6-(5,6,7,8-tetrahydro-imidazo[1,2-a]-pyridin-2-yl)-benzimidazol-1-yl)-methyl]-2-(1H-tetrazol-5-yl)-biphenyl

Beispiel 37

4'-[(2-n-Propyl-4-methyl-6-(1-methyl-6-fluor-benzimidazol-2-yl)-benzimidazol-1-yl)-methyl]-biphenyl-2-carbonsäure

Hergestellt analog Beispiel A aus 4'-[(2-n-Propyl-4-methyl-6-(1-methyl-6-fluor-benzimidazol-2-yl)-benzimidazol-1-yl)-methyl]-biphenyl-2-carbonsäure-tert.butylester und Trifluoressigsäure in Methylenchlorid.
Ausbeute: 76 % der Theorie,
Schmelzpunkt: 243-245°C
$C_{33}H_{29}FN_4O_2$ (532,60)

| | | | | | | |
|---|---|---|---|---|---|---|
| Ber. | C | 74,42 | H | 5,49 | N | 10,52 |
| Gef. | | 74,74 | | 5,52 | | 10,77 |

Massenspektrum: m/e = 532

Beispiel 38

4'-[(2-n-Propyl-4-chlor-6-(1-methylbenzimidazol-2-yl)-benzimidazol-1-yl)-methyl]-biphenyl-2-carbonsäure

Hergestellt analog Beispiel A aus 4'-[(2-n-Propyl-4-chlor-6-(1-methylbenzimidazol-2-yl)-benzimidazol-1-yl)-methyl]-biphenyl-2-carbonsäure-tert.butylester und Trifluoressigsäure in Methylenchlorid.
Ausbeute: 52,7 % der Theorie,
Schmelzpunkt: 292-295°C
$C_{32}H_{27}ClN_4O_2$ (535,06)
$R_f$-Wert: 0,30 (Kieselgel; Methylenchlorid/Ethanol = 19:1)

| | | | | | | | | |
|---|---|---|---|---|---|---|---|---|
| Ber. | C | 71,90 | H | 5,08 | N | 10,45 | Cl | 6,63 |
| Gef. | | 71,29 | | 5,21 | | 10,40 | | 6,76 |

Beispiel 39

4'-[(2-n-Propyl-4-chlor-6-(1-methylbenzimidazol-2-yl)-benzimidazol-1-yl)-methyl]-2-(1H-tetrazol-5-yl)-biphenyl-hydrochlorid

Hergestellt analog Beispiel 2 aus 4'-[(2-n-Propyl-4-chlor-6-(1-methylbenzimidazol-2-yl)-benzimidazol-1-yl)-methyl]-2-cyano-biphenyl und Natriumazid in Dimethylformamid.
Ausbeute: 54,8 % der Theorie,
Schmelzpunkt: ab 204°C sintern
$C_{32}H_{27}ClN_8$ x HCl (595,55)
$R_f$-Wert: 0,20 (Kieselgel; Petrolether/Essigester = 1:1 und 1 % Eisessig)

| | | | | | | | | |
|---|---|---|---|---|---|---|---|---|
| Ber. | C | 62,55 | H | 4,71 | N | 18,85 | Cl | 11,85 |
| Gef. | | 62,34 | | 4,97 | | 18,84 | | 11,57 |

Beispiel 40

4'-[(2-n-Propyl-4-methyl-6-(1-methyl-imidazol-4-yl)-benzimidazol-1-yl)-methyl]-biphenyl-2-carbonsäure

a) 3-Methyl-4-butyrylamino-5-nitro-acetophenon

32,6 g (148 mMol) 3-Methyl-4-butyrylamino-acetophenon werden unter Rühren portionsweise bei -15°C in 300 ml rauchende Salpetersäure eingetragen und weitere 30 Minuten bei -15°C gerührt. Das Reaktionsgemisch wird dann unter Rühren auf 3 1 Eis gegossen, das ausgefallene Rohprodukt abgesaugt, mit 400 ml Wasser gewaschen, getrocknet und durch Umkristallisieren aus Ethanol/Diethylether (1:1) gereinigt.
Ausbeute: 23,8 g (61,0 % der Theorie),
$R_f$-Wert: 0,32 (Kieselgel; Methylenchlorid)
$R_f$-Wert: 0,48 (Kieselgel; Methylenchlorid/Methanol = 50:1)

b) 3-Methyl-4-butyrylamino-5-nitro-ω-bromacetophenon

Zu einer Lösung von 23,8 g (90 mMol) 3-Methyl-4-butyrylamino-5-nitro-acetophenon in 900 ml Dichlormethan wird bei Raumtemperatur unter Rühren eine Lösung von 16,0 g (200 mMol) Brom in 140 ml Dioxan so langsam zugetropft, daß stets vollständige Entfärbung des Reaktionsgemisches stattfindet. Anschließend wird weitere zwei Stunden gerührt, dann das Reaktionsgemisch im Vakuum bis zur Trockne eingeengt, der so erhaltene Rückstand mit ca. 20 ml Dichlormethan/Diethylether (1:1) verrieben, abgesaugt und dann getrocknet. Man erhält so 23 g (74 % der Theorie) 3-Methyl-4-butyrylamino-5-nitro-ω-bromacetophenon, in den noch ca. 10 % Ausgangsmaterial enthalten sind. Das Produkt wird ohne weitere Reinigung weiter umgesetzt.
$R_f$-Wert: 0,69 (Kieselgel; Methylenchlorid/Methanol = 50:1)
$R_f$-Wert: 0,84 (Kieselgel; Methylenchlorid/Methanol = 9:1)

c) 2-Butyrylamino-3-nitro-5-(imidazol-4-yl)-toluol

Eine Lösung von 6,8 g (20 mMol) 3-Methyl-4-butyrylamino-5-nitro-ω-bromacetophenon in 20 ml Formamid wird 2 Stunden lang auf 140°C erhitzt. Die abgekühlte Lösung wird dann in ca. 50 ml 1 N Ammoniak gegossen und ca. 15 Minuten lang gerührt. Das ausgefallene Rohprodukt wird abgesaugt, mit ca. 50 ml Wasser gewaschen und getrocknet. Man erhält so 4,4 g (75 % der Theorie) des Produktes, das ohne weitere Reinigung weiter umgesetzt wird.
$R_f$-Wert: 0,29 (Kieselgel; Methylenchlorid/Methanol = 9:1)

d) 2-Butyrylamino-3-nitro-5-(1-methyl-imidazol-4-yl)-toluol

Zu einer Lösung von 2,5 g (8,7 mMol) 2-Butyrylamino-3-nitro-5-(imidazol-4-yl)-toluol und 5,2 g (30 mMol) Kaliumcarbonat-Dihydrat in 30 ml Dimethylsulfoxid werden 1,3 g (9,5 mMol) Methyljodid bei Raumtemperatur zugetropft und anschließend 2 Stunden lang gerührt. Das Reaktionsgemisch wird dann in ca. 150 ml Wasser eingerührt und anschließend viermal mit je 25 ml Essigester extrahiert. Die organischen Extrakte werden mit ca. 30 ml Wasser gewaschen, getrocknet und eingeengt. Das so erhaltene Rohprodukt wird durch Säulenchromatographie (300 g Kieselgel, Laufmittel: Methylenchlorid/Methanol = 30:1) gereinigt.
Ausbeute: 640 mg (24 % der Theorie),
$R_f$-Wert: 0,54 (Kieselgel; Methylenchlorid/Methanol = 9:1)

e) 2-Butyrylamino-3-amino-5-(1-methyl-imidazol-4-yl)-toluol

640 mg (2,1 mMol) 2-Butyrylamino-3-nitro-5-(1-methyl-imidazol-4-yl)-toluol werden in 30 ml Methanol nach Zugabe von ca. 200 mg Palladium/Kohle (20 %) bei Raumtemperatur und einem Wasserstoff-Druck von 5 bar hydriert. Nach vollständiger Wasserstoffaufnahme wird vom Katalysator abfiltriert und das Filtrat eingeengt. Das so erhaltene Rohprodukt wird ohne weitere Reinigung weiter umgesetzt.
Ausbeute: 600 mg (100 % der Theorie),
$R_f$-Wert: 0,23 (Kieselgel; Methylenchlorid/Methanol = 9:1)

f) 2-n-Propyl-4-methyl-6-(1-methyl-imidazol-4-yl)-benzimidazol

600 mg (2,1 mMol) 2-Butyrylamino-3-amino-5-(1-methyl-imidazol-4-yl)-toluol werden in 10 ml Eisessig eine Stunde lang zum Rückfluß erhitzt. Dann wird im Vakuum zur Trockene eingedampft, der Rückstand mit ca. 15 ml Wasser versetzt, mit Ammoniak alkalisch gestellt und viermal mit je ca. 10 ml Essigester extrahiert. Die organischen Extrakte werden mit ca. 15 ml Wasser gewaschen, getrocknet und schließlich eingeengt. Das so erhaltene Rohprodukt wird ohne weitere Reinigung weiter umgesetzt.
Ausbeute: 420 mg (79 % der Theorie),
$R_f$-Wert: 0,37 (Kieselgel; Methylenchlorid/Methanol = 9:1)

g) 4'-[(2-n-Propyl-4-methyl-6-(1-methyl-imidazol-4-yl)-benzimidazol-1-yl)-methyl]-biphenyl-2-carbonsäure-tert.butylester

Zu einer Lösung von 200 mg (0,79 mMol) 2-n-Propyl-4-methyl-6-(1-methyl-imidazol-4-yl)-benzimidazol und 90 mg (0,8 mMol) Kalium-tert.butylat in 5 ml Dimethylsulfoxid werden 280 mg (0,8 mMol) 4'-Brommethyl-biphenyl-2-carbonsäure-tert.butylester gegeben und das Gemisch 90 Minuten lang bei Raumtemperatur gerührt, dann in ca. 40 ml Wasser eingerührt, viermal mit je ca. 10 ml Essigester extrahiert, dann die organischen Extrakte mit 10 ml Wasser gewaschen, getrocknet und zur Trockne eingeengt. Das so erhaltene Rohprodukt wurde durch Säulenchromatographie (100 g Kieselgel, Laufmittel; Dichlormethan/Methanol = 30:1) gereinigt.
Ausbeute: 230 mg (56 % der Theorie),
$R_f$-Wert: 0,61 (Kieselgel; Methylenchlorid/Methanol = 9:1)

h) 4'-[(2-n-Propyl-4-methyl-6-(1-methyl-imidazol-4-yl)-benzimidazol-1-yl)-methyl]-biphenyl-2-carbonsäure

Eine Lösung von 230 mg (0,44 mMol) 4'-[(2-n-Propyl-4-methyl-6-(1-methyl-imidazol-4-yl)-benzimidazol-1-yl)-methyl]-biphenyl-2-carbonsäure-tert.butylester und 2 ml Trifluoressigsäure in 10 ml Dichlormethan wurde bei Raumtemperatur über Nacht gerührt und anschließend zur Trockne eingeengt. Der Rückstand wurde in ca. 5 ml verdünnter Natronlauge gelöst, die Lösung mit Essigsäure neutralisiert, der danach ausgefallene Niederschlag abgesaugt, mit Wasser gewaschen und getrocknet. Ausbeute: 120 mg (59 % der Theorie),
Schmelzpunkt: 293-295°C

$R_f$-Wert: 0,39 (Kieselgel; Methylenchlorid/Methanol = 9:1)
Analog Beispiel 40 werden folgende Verbindungen erhalten:

4'-[(2-n-Propyl-4-methyl-6-(1-ethyl-imidazol-4-yl)-benzimidazol-1-yl)-methyl]-biphenyl-2-carbonsäure

4'-[(2-n-Propyl-4-methyl-6-(1-benzyl-imidazol-4-yl)-benzimidazol-1-yl)-methyl]-biphenyl-2-carbonsäure

4'-[(2-n-Propyl-4-methyl-6-(1-isopropyl-imidazol-4-yl)-benzimidazol-1-yl)-methyl]-biphenyl-2-carbonsäure

Beispiel 41

4'-[(2-n-Propyl-4-methyl-6-(1-methyl-imidazol-4-yl)-benzimidazol-1-yl)-methyl]-2-(1H-tetrazol-5-yl)-biphenyl

Hergestellt analog Beispiel 2 aus 4'-[(2-n-Propyl-4-methyl-6-(1-methyl-imidazol-4-yl)-benzimidazol-1-yl)-methyl]-2-cyano-biphenyl und Natriumazid in Dimethylformamid.
Ausbeute: 24 % der Theorie,
Schmelzpunkt: 255-257°C
$R_f$-Wert: 0,24 (Kieselgel; Methylenchlorid/Methanol = 9:1)
$C_{29}H_{28}N_8$ x $H_2O$ (506,62)

| | | | | | | |
|---|---|---|---|---|---|---|
| Ber. | C | 68,75 | H | 5,97 | N | 22,12 |
| Gef. | | 68,90 | | 5,97 | | 22,03 |

Analog Beispiel 41 werden folgende Verbindungen erhalten:

4'-[(2-n-Propyl-4-methyl-6-(1-ethyl-imidazol-4-yl)-benzimidazol-1-yl)-methyl]-2-(1H-tetrazol-5-yl)-biphenyl

4'-[(2-n-Propyl-4-methyl-6-(1-benzyl-imidazol-4-yl)-benzimidazol-1-yl)-methyl]-biphenyl-2-carbonsäure

4'-[(2-n-Propyl-4-methyl-6-(1-isopropyl-imidazol-4-yl)-benzimidazol-1-yl)-methyl]-2-(1H-tetrazol-5-yl)-biphenyl

Beispiel 42

4'-[(2-Ethyl-4-methyl-6-(5,6,7,8-tetrahydro-imidazo[1,2-a]pyridin-2-yl)-benzimidazol-1-yl)-methyl]-2-(1H-tetrazol-5-yl)-biphenyl

Hergestellt analog Beispiel 2 aus 4'-[(2-Ethyl-4-methyl-6-(5,6,7,8-tetrahydro-imidazo[1,2-a]pyridin-2-yl)-benzimidazol-1-yl)-methyl]-2-cyano-biphenyl und Natriumazid in Dimethylformamid.
Ausbeute: 21 % der Theorie,
Schmelzpunkt: amorph
$R_f$-Wert: 0,27 (Kieselgel; Methylenchlorid/Ethanol = 9:1)
$C_{31}H_{30}N_8$ (514,64)

| | | | | | | |
|---|---|---|---|---|---|---|
| Ber. | C | 72,35 | H | 5,88 | N | 21,78 |
| Gef. | | 72,01 | | 5,82 | | 21,44 |

Beispiel 43

4'-[(2-n-Propyl-4-methyl-6-(8-methyl-imidazo[1,2-a]pyridin2-yl)-benzimidazol-1-yl)-methyl]-biphenyl-2-carbonsäure

Hergestellt analog Beispiel A aus 4'-[(2-n-Propyl-4-methyl-6-(8-methyl-imidazo[1,2-a]pyridin-2-yl)-benzimidazol-1-yl)-methyl]-biphenyl-2-carbonsäure-tert.butylester und Trifluoressigsäure in Methylenchlorid.
Ausbeute: 87 % der Theorie,
Schmelzpunkt: 295-297°C
$R_f$-Wert: 0,34 (Kieselgel; Methylenchlorid/Ethanol = 9:1)
$C_{33}H_{30}N_4O_2$ x $H_2O$ (532,65)

| | | | | | | |
|---|---|---|---|---|---|---|
| Ber. | C | 74,41 | H | 6,06 | N | 10,52 |
| Gef. | | 74,81 | | 6,05 | | 10,43 |

Beispiel 44

4'-[(2-n-Propyl-4-methyl-6-(2-pyridyl)-benzimidazol-1-yl)-methyl]-2-(1H-tetrazol-5-yl)-biphenyl

Hergestellt analog Beispiel 2 aus 4'-[(2-n-Propyl-4-methyl-6-(2-pyridyl)-benzimidazol-1-yl)-methyl]-2-cyano-biphenyl und Natriumazid in Dimethylformamid.
Ausbeute: 56 % der Theorie,
Schmelzpunkt: ab 136°C (Zers.)
$C_{30}H_{27}N_7$ x 0,5 $H_2O$ (494,60)

| | | | | | | |
|---|---|---|---|---|---|---|
| Ber. | C | 72,85 | H | 5,71 | N | 19,83 |
| Gef. | | 72,45 | | 6,01 | | 19,83 |

Beispiel 45

4'-[(2-n-Propyl-4-methyl-6-(8-methyl-imidazo[1,2-a]pyridin-2-yl)-benzimidazol-1-yl)-methyl]-2-(1H-tetrazol-5-yl)-biphenyl

Hergestellt analog Beispiel 2 aus 4'-[(2-n-Propyl-4-methyl-6-(8-methyl-imidazo[1,2-a]pyridin-2-yl)-benzimidazol-1-yl)-methyl]-2-cyano-biphenyl und Natriumazid in Dimethylformamid. Ausbeute: 19 % der Theorie,
Schmelzpunkt: amorph
$R_f$-Wert: 0,36 (Kieselgel; Methylenchlorid/Ethanol = 9:1)
$C_{33}H_{30}N_8$ (538,61)
Massenspektrum: m/e = 538

Beispiel 46

4'-[2-Ethyl-4-methyl-6-(5,6,7,8-tetrahydro-imidazo[1,2-a]pyridin-2-yl)-benzimidazol-1-yl)-methyl]-biphenyl-2-carbonsäure

Hergestellt analog Beispiel A aus 4'-[2-Ethyl-4-methyl-6-(5,6,7,8-tetrahydro-imidazo[1,2-a]pyridin-2-yl)-benzimidazol-1-yl)-methyl]-biphenyl-2-carbonsäure-tert.butylester und Trifluoressigsäure in Methylenchlorid.
Ausbeute: 50 % der Theorie,
Schmelzpunkt: > 300°C
$R_f$-Wert: 0,16 (Kieselgel; Methylenchlorid/Ethanol = 9:1)

Beispiel 47

4'-[(2-n-Propyl-4-methyl-6-(1-isopropyl-imidazol-4-yl)-benzimidazol-1-yl)-methyl]-biphenyl-2-carbonsäure

Hergestellt analog Beispiel A aus 4'-[(2-n-Propyl-4-methyl-6-(1-isopropyl-imidazol-4-yl)-benzimidazol-1-yl)-methyl]-biphenyl-2-carbonsäure-tert.butylester und Trifluoressigsäure in Methylenchlorid.
Ausbeute: 84 % der Theorie,
Schmelzpunkt: 285-286°C
$R_f$-Wert: 0,55 (Kieselgel; Methylenchlorid/Methanol = 9:1)

Beispiel 48

4'-[(2-n-Propyl-4-methyl-6-(1-isopropyl-imidazol-4-yl)-benzimidazol-1-yl)-methyl]-2-(1H-tetrazol-5-yl)-biphenyl

Hergestellt analog Beispiel 2 aus 4'-[(2-n-Propyl-4-methyl-6-(1-isopropyl-imidazol-4-yl)-benzimidazol-1-yl)-methyl]-2-cyano-biphenyl und Natriumazid in Dimethylformamid.
Ausbeute: 18 % der Theorie,

Schmelzpunkt: amorph
$R_f$-Wert: 0,29 (Kieselgel; Methylenchlorid/Methanol = 9:1)
$C_{31}H_{32}N_8$ (516,66)
Massenspektrum: m/e = 516

Beispiel 49

4'-[(2-n-Propyl-4-methyl-6-(1-benzyl-imidazol-4-yl)-benzimidazol-1-yl)-methyl]-biphenyl-2-carbonsäure

Hergestellt analog Beispiel A aus 4'-[(2-n-Propyl-4-methyl-6-(1-benzyl-imidazol-4-yl)-benzimidazol-1-yl)-methyl]-biphenyl-tert.butylester und Trifluoressigsäure in Methylenchlorid.

Beispiel 50

4'-[(2-n-Propyl-4-methyl-6-(1-benzyl-imidazol-4-yl)-benzimidazol-1-yl)-methyl]-2-(1H-tetrazol-5-yl)-biphenyl

Hergestellt analog Beispiel 2 aus 4'-[(2-n-Propyl-4-methyl-6-(1-benzyl-imidazol-4-yl)-benzimidazol-1-yl)-methyl]-2-cyanobiphenyl und Natriumazid in Dimethylformamid.

Beispiel 51

| Ampullen, enthaltend 50 mg Wirkstoff pro 5 ml | |
|---|---|
| Wirkstoff | 50 mg |
| $KH_2PO_4$ | 2 mg |
| $Na_2HPO_4$ x $2H_2O$ | 50 mg |
| NaCl | 12 mg |
| Wasser für Injektionszwecke ad | 5 ml |

Herstellung:

In einem Teil des Wassers werden die Puffersubstanzen und das Isotonans gelöst. Der Wirkstoff wird zugegeben und nach vollständiger Lösung mit Wasser auf das Nennvolumen aufgefüllt.

Beispiel 52

| Ampullen, enthaltend 100 mg Wirkstoff pro 5 ml | |
|---|---|
| Wirkstoff | 100 mg |
| Methylglucamin | 35 mg |
| Glykofurol | 1000 mg |
| Polyethylenglykol-Polypropylenglykol-Blockpolymer | 250 mg |
| Wasser für Injektionszwecke ad | 5 ml |

Herstellung:

In einem Teil des Wassers wird Methylglucamin gelöst und der Wirkstoff unter Rühren und Erwärmen in Lösung gebracht. Nach Zugabe der Lösungsmittel wird mit Wasser auf das Nennvolumen aufgefüllt.

Beispiel 53

| Tabletten, enthaltend 50 mg Wirkstoff | |
|---|---|
| Wirkstoff | 50,0 mg |

(fortgesetzt)

| Tabletten, enthaltend 50 mg Wirkstoff | |
|---|---|
| Calciumphosphat | 70,0 mg |
| Milchzucker | 40,0 mg |
| Maisstärke | 35,0 mg |
| Polyvinylpyrrolidon | 3,5 ml |
| Magnesiumstearat | 1,5 mg |
| | 200,0 mg |

Herstellung:

Der Wirkstoff, $CaHPO_4$, Milchzucker und Maisstärke werden mit einer wässrigen PVP-Lösung gleichmäßig befeuchtet. Die Masse wird durch ein 2-mm-Sieb gegeben, im Umlufttrockenschrank bei 50°C getrocknet und erneut gesiebt.

Nach Zumischen des Schmiermittels wird das Granulat auf einer Tablettiermaschine verpresst.

Beispiel 54

| Dragees, enthaltend 50 mg Wirkstoff | |
|---|---|
| Wirkstoff | 50,0 mg |
| Lysin | 25,0 mg |
| Milchzucker | 60,0 mg |
| Maisstärke | 34,0 mg |
| Gelatine | 10,0 mg |
| Magnesiumstearat | 1,0 mg |
| | 180,0 mg |

Herstellung:

Der Wirkstoff wird mit den Hilfsstoffen gemischt und mit einer wässrigen Gelatine-Lösung befeuchtet. Nach Siebung und Trocknung wird das Granulat mit Magnesiumstearat vermischt und zu Kernen verpresst.

Die so hergestellten Kerne werden nach bekannten Verfahren mit einer Hülle überzogen. Der Dragiersuspension oder -lösung kann Farbstoff zugegeben werden.

Beispiel 55

| Dragees, enthaltend 100 mg Wirkstoff | |
|---|---|
| Wirkstoff | 100,0 mg |
| Lysin | 50,0 mg |
| Milchzucker | 86,0 mg |
| Maisstärke | 50,0 mg |
| Polyvinylpyrrolidon | 2,8 mg |
| Mikrokristalline Cellulose | 60,0 mg |
| Magnesiumstearat | 1,2 mg |
| | 350,0 mg |

Herstellung:

Der Wirkstoff wird mit den Hilfsstoffen gemischt und mit einer wässrigen PVP-Lösung befeuchtet. Die feuchte Masse wird durch ein 1,5-mm-Sieb gegeben und bei 45°C getrocknet. Nach dem Trocknen wird erneut gesiebt und das Magnesiumstearat zugemischt. Diese Mischung wird zu Kernen verpreßt.

Die so hergestellten Kerne werden nach bekannten Verfahren mit einer Hülle überzogen. Der Dragiersuspension oder -lösung können Farbstoffe zugegeben werden.

Beispiel 56

| Kapseln, enthaltend 250 mg Wirkstoff | |
|---|---|
| Wirkstoff | 250,0 mg |
| Maisstärke | 68,5 mg |
| Magnesiumstearat | 1.5 mg |
| | 320,0 mg |

Herstellung:

Wirkstoff und Maisstärke werden gemischt und mit Wasser befeuchtet. Die feuchte Masse wird gesiebt und getrocknet. Das trockene Granulat wird gesiebt und mit Magnesiumstearat gemischt. Die Endmischung wird in Hartgelatinekapseln Größe 1 abgefüllt.

Beispiel 57

| Orale Suspension, enthaltend 50 mg Wirkstoff pro 5 ml | |
|---|---|
| Wirkstoff | 50,0 mg |
| Hydroxyethylcellulose | 50,0 mg |
| Sorbinsäure | 5,0 mg |
| Sorbit 70%ig | 600,0 mg |
| Glycerin | 200,0 mg |
| Aroma | 15,0 mg |
| Wasser ad | 5,0 ml |

Herstellung:

Destilliertes Wasser wird auf 70°C erhitzt. Hierin wird unter Rühren Hydroxyethylcellulose gelöst. Durch Zugabe von Sorbitlösung und Glycerin wird auf Raumtemperatur abgekühlt. Bei Raumtemperatur werden Sorbinsäure, Aroma und Wirkstoff zugegeben. Zur Entlüftung der Suspension wird unter Rühren evakuiert. Eine Dosis = 50 mg ist enthalten in 5,0 ml.

Beispiel 58

| Suppositorien, enthaltend 100 mg Wirkstoff | |
|---|---|
| Wirkstoff | 100,0 mg |
| Adeps solidus | 1600,0 mg |
| | 1700,0 mg |

Herstellung:

Das Hartfett wird geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

**Patentansprüche**

**Patentansprüche für folgende Vertragsstaaten : AT, BE, CH, DE, DK, FR, GB, IT, LI, LU, NL, SE**

1. Benzimidazole der allgemeinen Formel

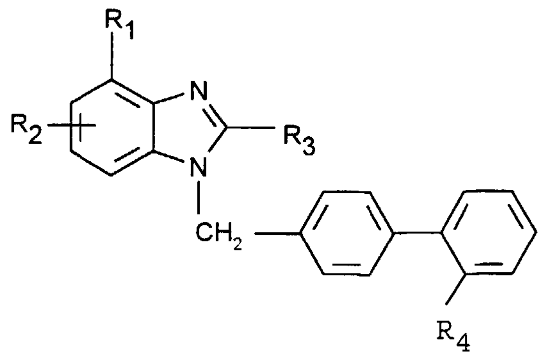

(I),

in der
$R_1$ ein Fluor-, Chlor- oder Bromatom, eine Alkyl-, Cycloalkyl-, Fluormethyl-, Difluormethyl- oder Trifluormethylgruppe,
$R_2$ eine gegebenenfalls durch eine oder zwei Alkylgruppen oder durch eine Tetramethylen- oder Pentamethylengruppe substituierte 5-, 6- oder 7-gliedrige Alkylenimino- oder Alkenyleniminogruppe, in welcher eine Methylengruppe durch eine Carbonyl- oder Sulfonylgruppe ersetzt ist,
eine gegebenenfalls durch eine Alkyl- oder Phenylgruppe monooder disubstituierte Maleinsäureimidogruppe, wobei die Substituenten gleich oder verschieden sein können,
eine gegebenenfalls in 1-Stellung durch eine Alkylgruppe mit 1 bis 6 Kohlenstoffatomen oder eine Cycloalkylgruppe substituierte Benzimidazol-2-yl- oder 4,5,6,7-Tetrahydro-benzimidazol-2-yl-gruppe, wobei der Phenylkern einer der vorstehend erwähnten Benzimidazolgruppen zusätzlich durch ein Fluoratom, eine Methyl- oder Trifluormethylgruppe substituiert sein kann, eine Imidazo[2,1-b]thiazol-6-yl-, Imidazo[1,2-a]pyridin-2-yl-, 5,6,7, 8-Tetrahydro-imidazo[1,2-a]pyridin-2-yl-, Imidazo-[1,2-a]pyrimidin-2-yl-, Imidazo[4,5-b]pyridin-2-yl-, Imidazo-[4,5-c]pyridin-2-yl-, Imidazo[1,2-c]pyrimidin-2-yl-, Imidazo-[1,2-a]pyrazin-2-yl-, Imidazo[1,2-b]pyridazin-2-yl-, Purin-8-yl-, Imidazo [4,5-b]pyrazin-2-yl-, Imidazo[4,5-c]pyridazin-2-yl- oder Imidazo[4,5-d]pyridazin-2-yl-gruppe,
eine Pyridylgruppe oder
eine gegebenenfalls in 1-Stellung durch eine Alkyl- oder Benzylgruppe über ein Kohlenstoffatom gebundene Imidazolylgruppe, welche zusätzlich im Kohlenstoffgerüst durch eine Alkylgruppe substituiert sein kann,
$R_3$ eine Alkylgruppe mit 1 bis 5 Kohlenstoffatomen oder eine Cycloalkylgruppe mit 3 bis 5 Kohlenstoffatomen und
$R_4$ eine Carboxy- oder IH-Tetrazolylgruppe bedeuten,
und deren Salze mit anorganischen oder organischen Säuren oder Basen,
wobei, soweit nichts anderes erwähnt wurde, ein vorstehend erwähnter Alkylteil jeweils 1 bis 3 Kohlenstoffatome sowie ein vorstehend erwähnter Cycloalkylteil jeweils 3 bis 7 Kohlenstoffatome enthalten kann.

2. Benzimidazole der allgemeinen Formel I gemäß Anspruch 1, in der
$R_1$ ein Chloratom, eine Alkylgruppe mit 1 bis 3 Kohlenstoffatomen oder eine Trifluormethylgruppe,
$R_2$ eine 5-, 6- oder 7-gliedrige Alkyleniminogruppe, in welcher eine Methylengruppe durch eine Carbonyl- oder Sulfonylgruppe ersetzt ist,
eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können,
eine gegebenenfalls in 1-Stellung durch eine Alkylgruppe mit 1 bis 6 Kohlenstoffatomen oder eine Cycloalkylgruppe substituierte Benzimidazol-2-yl- oder 4,5,6,7-Tetrahydro-benzimidazol-2-yl-gruppe, wobei der Phenylkern einer der vorstehend erwähnten Benzimidazolgruppen zusätzlich durch ein Fluoratom, eine Methyl- oder Trifluormethylgruppe substituiert sein kann, eine Imidazo[2,1-b]thiazol-6-yl-, Imidazo[1,2-a]pyridin-2-yl-, 5,6,7,8-Tetrahydroimidazo[1,2-a]pyridin-2-yl-,Imidazo[1,2-a]-pyrimidin-2-yl-, Imidazo[4,5-b]pyridin-2-yl-, Imidazo[4,5-c]-pyridin-2-yl-, Imidazo[1,2-c]pyrimidin-2-yl-, Imidazo[1,2-a]-pyrazin-2-yl-, Imidazo[1,2-b]pyridazin-2-yl-, Purin-8-yl-, Imidazo [4,5-b]pyrazin-2-yl-, Imidazo[4,5-c]pyridazin-2-yl-oder Imidazo[4,5-d]pyridazin-2-yl-gruppe,
eine Pyridylgruppe oder

eine in 1-Stellung durch eine Alkylgruppe mit 1 bis 3 Kohlenstoffatomen oder durch eine Benzylgruppe substituierte Imidazol-4-yl-gruppe, welche zusätzlich im Kohlenstoffgerüst durch eine Alkylgruppe mit 1 bis 3 Kohlenstoffatomen substituiert sein kann,
$R_3$ eine Alkylgruppe mit 1 bis 5 Kohlenstoffatomen oder eine Cycloalkylgruppe mit 3 bis 5 Kohlenstoffatomen und
$R_4$ eine Carboxy- oder IH-Tetrazolylgruppe bedeuten,
und deren Salze mit anorganischen oder organischen Säuren oder Basen.

**3.** Benzimidazole der allgemeinen Formel I gemäß Anspruch 1, in der
$R_1$ eine Methylgruppe oder ein Chloratom,
$R_2$ eine 5-, 6- oder 7-gliedrige Alkyleniminogruppe, in welcher eine Methylengruppe durch eine Carbonyl- oder Sulfonylgruppe ersetzt ist,
eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können,
eine gegebenenfalls in 1-Stellung durch eine Alkylgruppe mit 1 bis 3 Kohlenstoffatomen substituierte Benzimidazol-2-yl- oder 4,5,6,7-Tetrahydro-benzimidazol-2-yl-gruppe, wobei der Phenylkern einer der vorstehend erwähnten Benzimidazolgruppen zusätzlich durch ein Fluoratom substituiert sein kann, eine Imidazo[1,2-a]pyridin-2-yl-, 5,6,7,8-Tetrahydro-imidazo[1,2-a]- pyridin-2-yl-, Imidazo[1,2-a]pyrimidin-2-yl- oder Imidazo-[2,1-b]thiazol-6-yl-gruppe oder
eine in 1-Stellung durch eine Alkylgruppe mit 1 bis 3 Kohlenstoffatomen substituierte Imidazol-4-yl-gruppe,
$R_3$ eine Alkylgruppe mit 1 bis 5 Kohlenstoffatomen oder eine Cycloalkylgruppe mit 3 bis 5 Kohlenstoffatomen und
$R_4$ eine Carboxy- oder IH-Tetrazolylgruppe bedeuten,
und deren Salze mit anorganischen oder organischen Säuren oder Basen.

**4.** Folgende Benzimidazole der allgemeinen Formel I gemäß Anspruch 1 :
(a) 4'-[[2-n-Propyl-4-methyl-6-(1-methylbenzimidazol-2-yl)-benzimidazol-1-yl]-methyl]-biphenyl-2-carbonsäure,
(b) 4'-[[2-n-Propyl-4-methyl-6-(1-methylbenzimidazol-2-yl)-benzimidazol-1-yl]-methyl]-2-(1H-tetrazol-5-yl)-biphenyl,
(c) 4'-[[2-n-Propyl-4-methyl-6-(butansultam-1-yl)-benzimidazol-1-yl]-methyl]-2-(1H-tetrazol-5-yl)-biphenyl,
(d) 4'-[[2-n-Butyl-6-(2,3-dimethylmaleinsäureimino)-4-methylbenzimidazol-1-yl]-methyl]-biphenyl-2-carbonsäure,
(e) 4'-[(2-Cyclopropyl-4-methyl-6-(1-methylbenzimidazol-2-yl)-benzimidazol-1-yl)-methyl]-biphenyl-2-carbonsäure,
(f) 4'-[(2-n-Propyl-4-methyl-6-(1-methyl-5-fluor-benzimidazol-2-yl)-benzimidazol-1-yl)-methyl]-biphenyl-2-carbonsäure,
(g) 4'-[(2-n-Propyl-4-methyl-6-(imidazo[1,2-a]pyrimidin-2-yl)-benzimidazol-1-yl)-methyl]-2-(1H-tetrazol-5-yl)-biphenyl,
(h) 4'-[(2-n-Propyl-4-methyl-6-(5,6,7,8-tetrahydro-imidazo-[1,2-a]pyridin-2-yl)-benzimidazol-1-yl)-methyl]-biphenyl-2-carbonsäure,
(i) 4'-[(2-n-Propyl-4-methyl-6-(5,6,7,8-tetrahydro-imidazo-[1,2-a]pyridin-2-yl)-benzimidazol-1-yl)-methyl]-2-(1H-tetrazol-5-yl)-biphenyl,
(j) 4'-[(2-n-Propyl-4-chlor-6-(1-methylbenzimidazol-2-yl)-benzimidazol-1-yl)-methyl]-2-(1H-tetrahol-5-yl)-biphenyl,
(k) 4'-[[2-n-Propyl-4-methyl-6-(imidazo[2,1-b]thiazol-6-yl)benzimidazol-1-yl]-methyl]-biphenyl-2-carbonsäure,
(l) 4'-[[2-Ethyl-4-methyl-6-(butansultam-1-yl)-benzimidazol-1-yl]-methyl]-2-(1H-tetrazol-5-yl)-biphenyl,
(m) 4'-[[2-n-Butyl-4-methyl-6-(1-methylbenzimidazol-2-yl)-benzimidazol-1-yl]-methyl]-2-(1H-tetrazol-5-yl)-biphenyl,
(n) 4'-[[2-n-Propyl-4-methyl-6-(imidazo[1,2-a]pyridin-2-yl)-benzimidazol-1-yl]-methyl]-biphenyl-2-carbonsäure,
(o) 4'-[[2-n-Propyl-4-methyl-6-(imidazo[l,2-a]pyridin-2-yl)- benzimidazol-1-yl]-methyl]-2-(1H-tetrazol-5-yl)-biphenyl,
(p) 4'-[[2-n-Propyl-4-methyl-6-(imidazo[2,1-b]thiazol-6-yl)-benzimidazol-1-yl]-methyl]-2-(1H-tetrazol-5-yl)-biphenyl,
(q) 4'-[(2-n-Propyl-4-methyl-6-(1-methyl-6-fluor-benzimidazol-2-yl)-benzimidazol-1-yl)-methyl]-biphenyl-2-carbonsäure und
(r) 4'-[(2-Ethyl-4-methyl-6-(5,6,7,8-tetrahydro-imidazo-[1,2-a]pyridin-2-yl)-benzimidazol-1-yl)-methyl]-biphenyl-2-carbonsäure,
und deren Salze mit anorganischen oder organischen Säuren oder Basen.

**5.** 4'-[[2-n-Propyl-4-methyl-6-(l-methylbenzimidazol-2-yl)-benzimidazol-1-yl]-methyl]-biphenyl-2-carbonsäure und dessen Salze mit anorganischen oder organischen Säuren oder Basen.

**6.** 4'-[(2-Ethyl-4-methyl-6-(5,6,7,8-tetrahydro-imidazo[1,2-a]-pyridin-2-yl)-benzimidazol-1-yl)-methyl]-biphenyl-2-carbonsäure und dessen Salze mit anorganischen oder organischen Säuren oder Basen.

**7.** Physiologisch verträgliche Salze der Verbindungen nach mindestens einem der Ansprüche 1 bis 6 mit anorganischen oder organischen Säuren oder Basen.

**8.** Arzneimittel, enthaltend eine Verbindung nach mindestens einem der Ansprüche 1 bis 6 oder ein physiologisch verträgliches Salz gemäß Anspruch 7 neben gegebenenfalls einem oder mehreren inerten Trägerstoffen und/oder Verdünnungsmitteln.

**9.** Verwendung einer Verbindung nach mindestens einem der Ansprüche 1 bis 7 zur Herstellung eines Arzneimittels mit Angiotensin-antagonistischer Wirkung.

**10.** Verfahren zur Herstellung eines Arzneimittels gemäß Anspruch 8, dadurch gekennzeichnet, daß auf nichtchemischem Wege eine Verbindung nach mindestens einem der Ansprüche 1 bis 7 in einen oder mehrere inerte Trägerstoffe und/oder Verdünnungsmittel eingearbeitet wird.

**11.** Verfahren zur Herstellung der Benzimidazole gemäß den Ansprüchen 1 bis 7, dadurch gekennzeichnet, daß

a) eine Verbindung der allgemeinen Formel

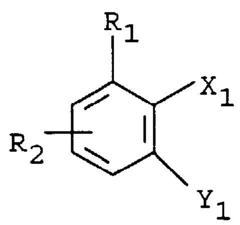

(II),

in der
$R_1$ und $R_2$ wie in den Ansprüchen 1 bis 6 definiert sind, einer der Reste $X_1$ oder $Y_1$ eine Gruppe der allgemeinen Formel

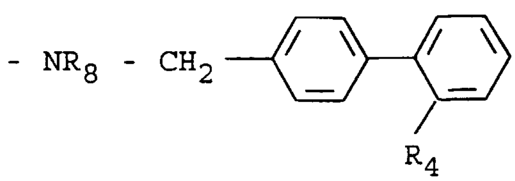

und der andere der Reste $X_1$ oder $Y_1$ eine Gruppe der allgemeinen Formel

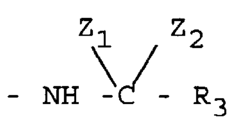

darstellen, wobei
$R_3$ und $R_4$ wie in den Ansprüchen 1 bis 6 definiert sind,
$R_8$ ein Wasserstoffatom oder eine $R_3CO$-Gruppe, wobei $R_3$ wie vorstehend erwähnt definiert ist,
$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder
$Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxyoder Alkylendithiogruppe mit jeweils 2 oder 3 Kohlenstoffatomen bedeuten, wobei jedoch einer der Reste $X_1$ oder $Y_1$ eine Gruppe der allgemeinen

Formel

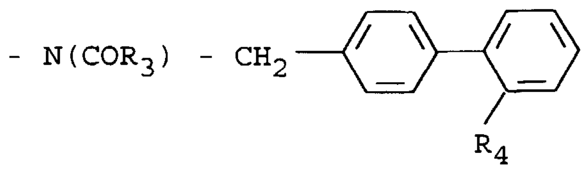

oder

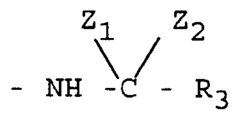

darstellen muß, cyclisiert und ein gegebenenfalls so erhaltenes entsprechendes N-Oxid reduziert wird oder

b) ein Benzimidazol der allgemeinen Formel

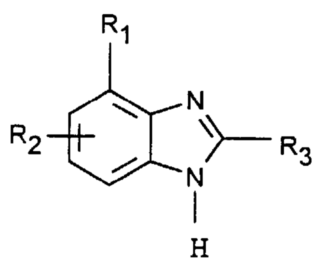

(III),

in der

$R_1$ bis $R_3$ wie in den Ansprüchen 1 bis 6 definiert sind, mit einer Biphenylverbindung der allgemeinen Formel

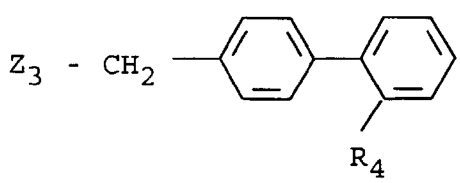

(IV),

in der

$R_4$ wie in den Ansprüchen 1 bis 6 definiert ist und

$Z_3$ eine nukleophile Austrittsgruppe darstellt, umgesetzt wird oder

c) zur Herstellung einer Verbindung der allgemeinen Formel I, in der $R_4$ eine Carboxygruppe darstellt, eine Verbindung der allgemeinen Formel

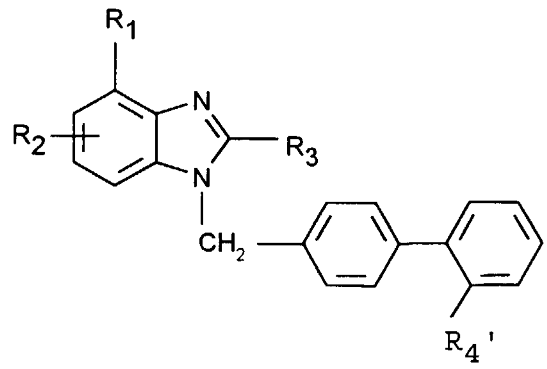

(V),

in der

$R_1$ bis $R_3$ wie in den Ansprüchen 1 bis 6 definiert sind und

$R_4$' eine mittels Hydrolyse, Thermolyse oder Hydrogenolyse in eine Carboxygruppe überführbare Gruppe darstellen, in eine entsprechende Carboxyverbindung übergeführt wird oder

d) zur Herstellung einer Verbindung der allgemeinen Formel I, in der $R_4$ eine IH-Tetrazolylgruppe darstellt, ein Schutzrest von einer Verbindung der allgemeinen Formel

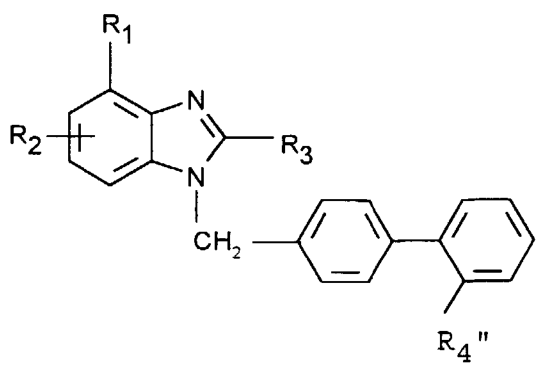

(VI),

in der

$R_1$, $R_2$ und $R_3$ wie in den Ansprüchen 1 bis 6 definiert sind und $R_4$" eine in 1- oder 3-Stellung durch einen Schutzrest geschützte 1H-Tetrazolylgruppe darstellt, abgespalten wird oder

e) zur Herstellung einer Verbindung der allgemeinen Formel I, in der $R_4$ eine 1H-Tetrazolylgruppe darstellt, eine Verbindung der allgemeinen Formel

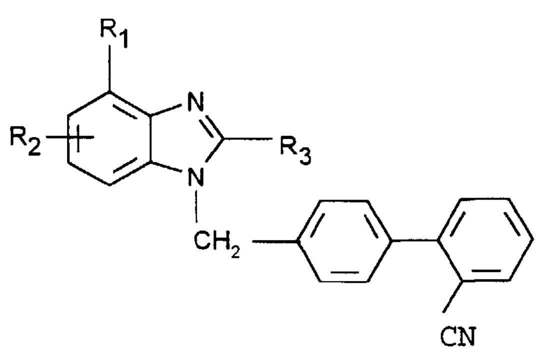

(VII),

in der

$R_1$ bis $R_3$ wie in den Ansprüchen 1 bis 6 definiert sind, mit Stickstoffwasserstoffsäure oder deren Salzen umgesetzt wird oder

f) zur Herstellung von Verbindungen der allgemeinen Formel I, in der $R_2$ eine der in den Ansprüchen 1 bis 6

erwähnten Imidazo[l,2-a]pyridin-2-yl-, Imidazo[1,2-a]pyrimidin-2-yl-, Imidazo[1,2-c]pyrimidin-2-yl-, Imidazo [1,2-a]pyrazin-2-yl-, Imidazo[1,2-b]pyridazin-2-yl- oder Imidazo[2,1-b]thiazol-6-ylgruppen darstellt, eine Verbindung der allgemeinen Formel

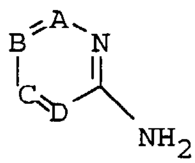

(VIII),

in der
einer der Reste A, B, C oder D eine gegebenenfalls durch eine Methylgruppe substituierte Methingruppe oder ein Stickstoffatom und
die übrigen der Reste A, B, C oder D Methingruppen oder A und B jeweils eine Methingruppe und die -C=D-Gruppe ein Schwefelatom bedeuten, mit einer Verbindung der allgemeinen Formel

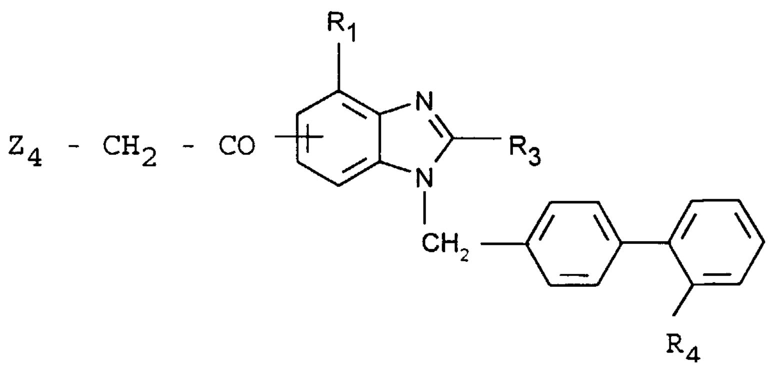

(IX),

in der
$R_1$, $R_3$ und $R_4$ wie in den Ansprüchen 1 bis 6 definiert sind und $Z_4$ eine nukleophile Austrittsgruppe wie ein Halogenatom, z.B. ein Chlor- oder Bromatom, darstellt, umgesetzt wird oder
g) zur Herstellung von Verbindungen der allgemeinen Formel I, in der $R_2$ eine der in den Ansprüchen 1 bis 6 erwähnten Benzimidazol-2-yl-, Imidazo[4,5-b]pyridin-2-yl-, Imidazo[4,5-c]-pyridin-2-yl-, Imidazo[4,5-b]pyrazin-2-yl-, Imidazo[4,5-c]-pyridazin-2-yl-, Imidazo[4,5-d]pyridazin-2-yl- oder Purin-8-yl-gruppen darstellt, eine Verbindung der allgemeinen Formel

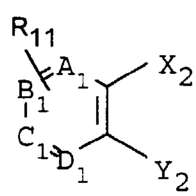

(X),

in der
null, einer oder zwei der Reste $A_1$, $B_1$, $C_1$ oder $D_1$ ein Stickstoffatom und
die verbleibenden Reste der Reste $A_1$, $B_1$, $C_1$ oder $D_1$ Methingruppen sowie
$R_{11}$ ein Wasserstoff- oder Fluoratom, eine Methyl- oder Trifluormethylgruppe,
einer der Reste $X_2$ oder $Y_2$ eine $R_{13}$-NH-Gruppe und der andere der Reste $X_2$ oder $Y_2$ eine Gruppe der allgemeinen Formel

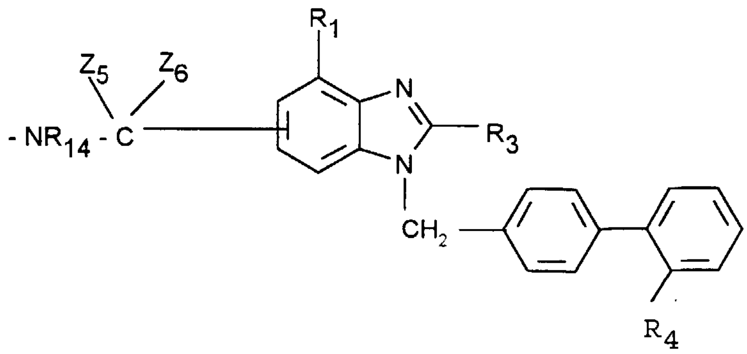

darstellen, wobei $R_1$, $R_3$ und $R_4$ wie in den Ansprüchen 1 bis 6 definiert sind, einer der Reste $R_{13}$ oder $R_{14}$ ein Wasserstoffatom und der andere der Reste $R_{13}$ oder $R_{14}$ ein Wasserstoffatom, eine Alkylgruppe mit 1 bis 6 Kohlenstoffatomen oder eine Cycloalkylgruppe mit 3 bis 7 Kohlenstoffatomen, $Z_5$ und $Z_6$, die gleich oder verschieden sein können, gegebenenfalls substituierte Aminogruppen oder gegebenenfalls durch niedere Alkylgruppen substituierte Hydroxy- oder Mercaptogruppen oder $Z_5$ und $Z_6$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert und ein gegebenenfalls so erhaltenes entsprechendes N-Oxid reduziert und eine so erhaltene Verbindung gegebenenfalls anschließend hydrolysiert wird und erforderlichenfalls ein während der Umsetzungen a) bis g) zum Schutze von reaktiven Gruppen verwendeter Schutzrest abgespalten wird und/oder erforderlichenfalls ein so erhaltenes Isomerengemisch in seine Isomeren aufgetrennt wird und/oder eine so erhaltene Verbindung der allgemeinen Formel I in ihr Salz, insbesondere für die pharmazeutische Anwendung in ihr physiologisch verträgliches Salz mit einer anorganischen oder organischen Säure oder Base, übergeführt wird.

**Patentansprüche für folgende Vertragsstaaten : ES, GR, PT**

**1.** Verfahren zur Herstellung der Benzimidazole der allgemeinen Formel

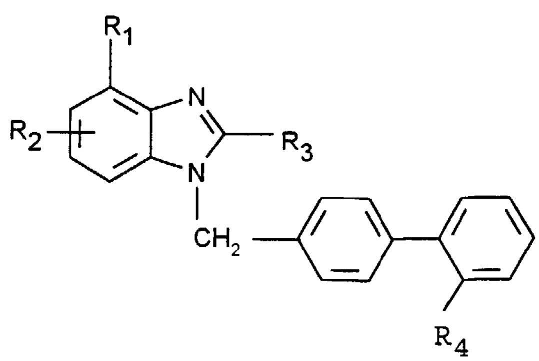

(I),

in der
$R_1$ ein Fluor-, Chlor- oder Bromatom, eine Alkyl-, Cycloalkyl-, Fluormethyl-, Difluormethyl- oder Trifluormethylgruppe,
$R_2$ eine gegebenenfalls durch eine oder zwei Alkylgruppen oder durch eine Tetramethylen- oder Pentamethylengruppe substituierte 5-, 6- oder 7-gliedrige Alkylenimino- oder Alkenyleniminogruppe, in welcher eine Methylengruppe durch eine Carbonyl- oder Sulfonylgruppe ersetzt ist,
eine gegebenenfalls durch eine Alkyl- oder Phenylgruppe mono-oder disubstituierte Maleinsäureimidogruppe, wobei die Substituenten gleich oder verschieden sein können,
eine gegebenenfalls in 1-Stellung durch eine Alkylgruppe mit 1 bis 6 Kohlenstoffatomen oder eine Cycloalkylgruppe substituierte Benzimidazol-2-yl- oder 4,5,6,7-Tetrahydro-benzimidazol-2-yl-gruppe, wobei der Phenylkern einer

der vorstehend erwähnten Benzimidazolgruppen zusätzlich durch ein Fluoratom, eine Methyl- oder Trifluormethylgruppe substituiert sein kann, eine Imidazo[2,1-b]thiazol-6-yl-, Imidazo[1,2-a]pyridin-2-yl-, 5,6,7,8-Tetrahydro-imidazo[1,2-a]pyridin-2-yl-, Imidazo-[1,2-a]pyrimidin-2-yl-, Imidazo[4,5-b]pyridin-2-yl-, Imidazo-[4,5-c]pyridin-2-yl-, Imidazo[1,2-c]pyrimidin-2-yl-, Imidazo-[1,2-a]pyrazin-2-yl-, Imidazo[1,2-b]pyridazin-2-yl-, Purin-8-yl-, Imidazo [4,5-b]pyrazin-2-yl-, Imidazo[4,5-c]pyridazin-2-yl- oder Imidazo[4,5-d]pyridazin-2-yl-gruppe,
eine Pyridylgruppe oder
eine gegebenenfalls in 1-Stellung durch eine Alkyl- oder Benzylgruppe über ein Kohlenstoffatom gebundene Imidazolylgruppe, welche zusätzlich im Kohlenstoffgerüst durch eine Alkylgruppe substituiert sein kann,
$R_3$ eine Alkylgruppe mit 1 bis 5 Kohlenstoffatomen oder eine Cycloalkylgruppe mit 3 bis 5 Kohlenstoffatomen und
$R_4$ eine Carboxy- oder IH-Tetrazolylgruppe bedeuten,
und von deren Salze mit anorganischen oder organischen Säuren oder Basen, insbesondere von deren physiologisch verträglichen Salzen,
wobei, soweit nichts anderes erwähnt wurde, ein vorstehend erwähnter Alkylteil jeweils 1 bis 3 Kohlenstoffatome sowie ein vorstehend erwähnter Cycloalkylteil jeweils 3 bis 7 Kohlenstoffatome enthalten kann.
dadurch gekennzeichnet, daß

a) eine Verbindung der allgemeinen Formel

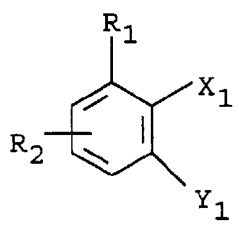

(II),

in der
$R_1$ und $R_2$ wie eingangs definiert sind,
einer der Reste $X_1$ oder $Y_1$ eine Gruppe der allgemeinen Formel

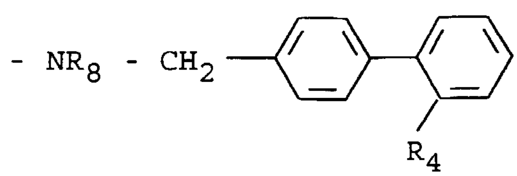

und der andere der Reste $X_1$ oder $Y_1$ eine Gruppe der allgemeinen Formel

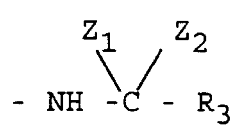

darstellen, wobei
$R_3$ und $R_4$ wie eingangs definiert sind,
$R_8$ ein Wasserstoffatom oder eine $R_3CO$-Gruppe, wobei $R_3$ wie vorstehend erwähnt definiert ist,
$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder
$Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxyoder Alkylendithiogruppe mit jeweils 2 oder 3 Kohlenstoffatomen bedeuten, wobei jedoch einer der Reste $X_1$ oder $Y_1$ eine Gruppe der allgemeinen Formel

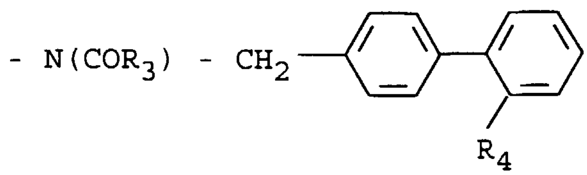

oder

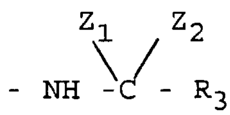

darstellen muß, cyclisiert und ein gegebenenfalls so erhaltenes entsprechendes N-Oxid reduziert wird oder

b) ein Benzimidazol der allgemeinen Formel

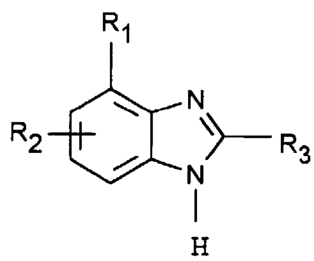

(III),

in der
$R_1$ bis $R_3$ wie eingangs definiert sind, mit einer Biphenylverbindung der allgemeinen Formel

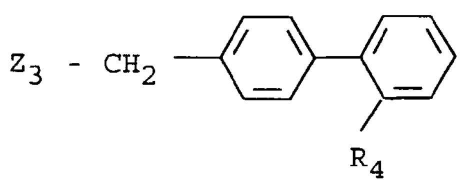

(IV),

in der
$R_4$ wie eingangs definiert ist und
$Z_3$ eine nukleophile Austrittsgruppe darstellt, umgesetzt wird oder

c) zur Herstellung einer Verbindung der allgemeinen Formel I, in der $R_4$ eine Carboxygruppe darstellt, eine Verbindung der allgemeinen Formel

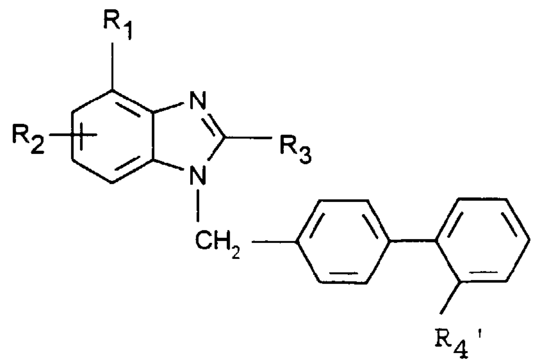

(V),

in der
$R_1$ bis $R_3$ wie eingangs definiert sind und
$R_4$' eine mittels Hydrolyse, Thermolyse oder Hydrogenolyse in eine Carboxygruppe überführbare Gruppe darstellen, in eine entsprechende Carboxyverbindung übergeführt wird oder

d) zur Herstellung einer Verbindung der allgemeinen Formel I, in der $R_4$ eine IH-Tetrazolylgruppe darstellt, ein Schutzrest von einer Verbindung der allgemeinen Formel

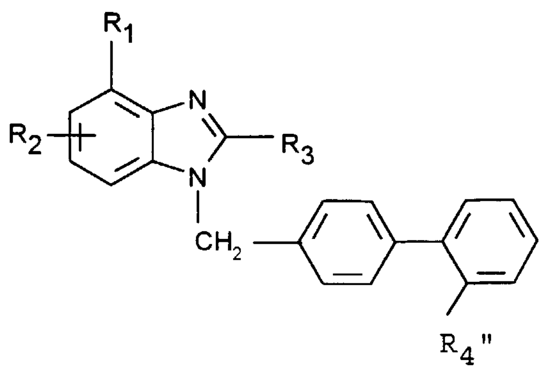

(VI),

in der
$R_1$, $R_2$ und $R_3$ wie eingangs definiert sind und
$R_4$" eine in 1- oder 3-Stellung durch einen Schutzrest geschützte IH-Tetrazolylgruppe darstellt, abgespalten wird oder

e) zur Herstellung einer Verbindung der allgemeinen Formel I, in der $R_4$ eine 1H-Tetrazolylgruppe darstellt, eine Verbindung der allgemeinen Formel

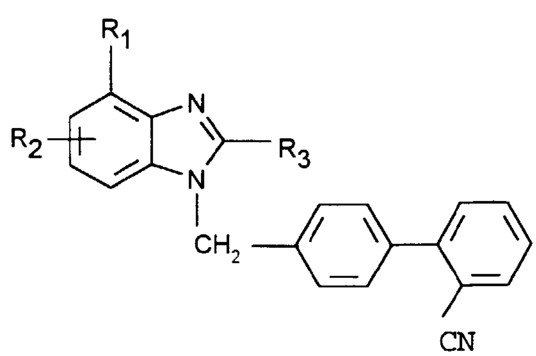

(VII),

in der
$R_1$ bis $R_3$ wie eingangs definiert sind, mit
Stickstoffwasserstoffsäure oder deren Salzen umgesetzt wird oder

f) zur Herstellung von Verbindungen der allgemeinen Formel I, in der $R_2$ eine der eingangs erwähnten Imidazo [1,2-a]pyridin-2-yl-, Imidazo[1,2-a]pyrimidin-2-yl-, Imidazo[1,2-c]- pyrimidin-2-yl-, Imidazo[1,2-a]pyrazin-2-yl-, Imidazo-[1,2-b]pyridazin-2-yl- oder Imidazo[2,1-b]thiazol-6-yl-gruppen darstellt, eine Verbindung der allgemeinen Formel

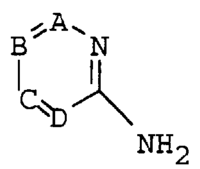
(VIII),

in der
einer der Reste A, B, C oder D eine gegebenenfalls durch eine Methylgruppe substituierte Methingruppe oder ein Stickstoffatom und
die übrigen der Reste A, B, C oder D Methingruppen oder A und B jeweils eine Methingruppe und die -C=D-Gruppe ein Schwefelatom bedeuten, mit einer Verbindung der allgemeinen Formel

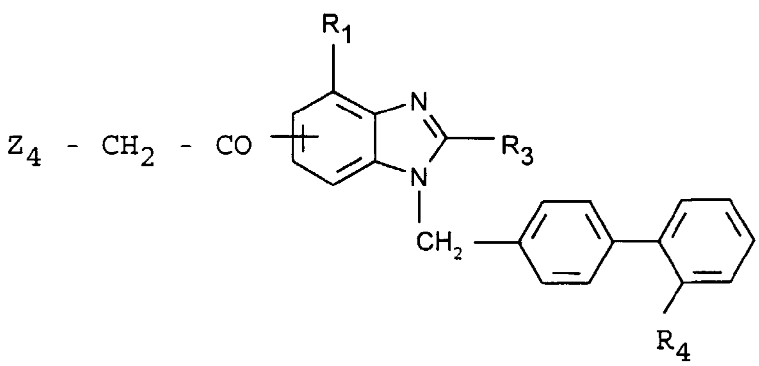
(IX),

in der
$R_1$, $R_3$ und $R_4$ wie eingangs definiert sind und
$Z_4$ eine nukleophile Austrittsgruppe wie ein Halogenatom, z.B. ein Chlor- oder Bromatom, darstellt, umgesetzt wird oder

g) zur Herstellung von Verbindungen der allgemeinen Formel I, in der $R_2$ eine der eingangs erwähnten Benzimidazol-2-yl-, Imidazo[4,5-b]pyridin-2-yl-, Imidazo[4,5-c]pyridin-2-yl-, Imidazo[4,5-b]pyrazin-2-yl-, Imidazo [4,5-c]pyridazin-2-yl-, Imidazo[4,5-d]pyridazin-2-yl- oder Purin-8-yl-gruppen darstellt, eine Verbindung der allgemeinen Formel

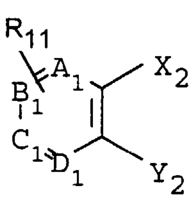
(X),

in der
null, einer oder zwei der Reste $A_1$, $B_1$, $C_1$ oder $D_1$ ein Stickstoffatom und
die verbleibenden Reste der Reste $A_1$, $B_1$, $C_1$ oder $D_1$ Methingruppen sowie

$R_{11}$ ein Wasserstoff- oder Fluoratom, eine Methyl- oder Trifluormethylgruppe,
einer der Reste $X_2$ oder $Y_2$ eine $R_{13}$-NH-Gruppe und der andere der Reste $X_2$ oder $Y_2$ eine Gruppe der allgemeinen Formel

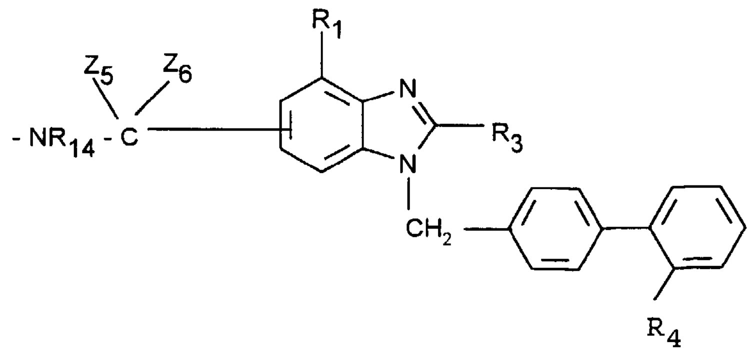

darstellen, wobei $R_1$, $R_3$ und $R_4$ wie eingangs definiert sind, einer der Reste $R_{13}$ oder $R_{14}$ ein Wasserstoffatom und der andere der Reste $R_{13}$ oder $R_{14}$ ein Wasserstoffatom, eine Alkylgruppe mit 1 bis 6 Kohlenstoffatomen oder eine Cycloalkylgruppe mit 3 bis 7 Kohlenstoffatomen,
$Z_5$ und $Z_6$, die gleich oder verschieden sein können, gegebenenfalls substituierte Aminogruppen oder gegebenenfalls durch niedere Alkylgruppen substituierte Hydroxy- oder Mercaptogruppen oder
$Z_5$ und $Z_6$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert und ein gegebenenfalls so erhaltenes entsprechendes N-Oxid reduziert und eine so erhaltene Verbindung gegebenenfalls anschließend hydrolysiert wird und
erforderlichenfalls ein während der Umsetzungen a) bis g) zum Schutze von reaktiven Gruppen verwendeter Schutzrest abgespalten wird und/oder
erforderlichenfalls ein so erhaltenes Isomerengemisch in seine Isomeren aufgetrennt wird und/oder
eine so erhaltene Verbindung der allgemeinen Formel I in ihr Salz, insbesondere für die pharmazeutische Anwendung in ihr physiologisch verträgliches Salz mit einer anorganischen oder organischen Säure oder Base, übergeführt wird.

**2.** Benzimidazole der allgemeinen Formel I gemäß Anspruch 1, in der
$R_1$ ein Chloratom, eine Alkylgruppe mit 1 bis 3 Kohlenstoffatomen oder eine Trifluormethylgruppe,
$R_2$ eine 5-, 6- oder 7-gliedrige Alkyleniminogruppe, in welcher eine Methylengruppe durch eine Carbonyl- oder Sulfonylgruppe ersetzt ist,
eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können,
eine gegebenenfalls in 1-Stellung durch eine Alkylgruppe mit 1 bis 6 Kohlenstoffatomen oder eine Cycloalkylgruppe substituierte Benzimidazol-2-yl- oder 4,5,6,7-Tetrahydro-benzimidazol-2-yl-gruppe, wobei der Phenylkern einer der vorstehend erwähnten Benzimidazolgruppen zusätzlich durch ein Fluoratom, eine Methyl- oder Trifluormethylgruppe substituiert sein kann, eine Imidazo[2,1-b]thiazol-6-yl-, Imidazo[1,2-a]pyridin-2-yl-, 5,6,7,8-Tetrahydroimidazo[1,2-a]pyridin-2-yl-,Imidazo[1,2-a]-pyrimidin-2-yl-, Imidazo[4,5-b]pyridin-2-yl-, Imidazo[4,5-c]-pyridin-2-yl-, Imidazo[1,2-c]pyrimidin-2-yl-, Imidazo[1,2-a]-pyrazin-2-yl-, Imidazo[1,2-b]pyridazin-2-yl-, Purin-8-yl-, Imidazo [4,5-b]pyrazin-2-yl-, Imidazo[4,5-c]pyridazin-2-yl-oder Imidazo [4,5-d]pyridazin-2-yl-gruppe,
eine Pyridylgruppe oder
eine in 1-Stellung durch eine Alkylgruppe mit 1 bis 3 Kohlenstoffatomen oder durch eine Benzylgruppe substituierte Imidazol-4-yl-gruppe, welche zusätzlich im Kohlenstoffgerüst durch eine Alkylgruppe mit 1 bis 3 Kohlenstoffatomen substituiert sein kann,
$R_3$ eine Alkylgruppe mit 1 bis 5 Kohlenstoffatomen oder eine Cycloalkylgruppe mit 3 bis 5 Kohlenstoffatomen und
$R_4$ eine Carboxy- oder lH-Tetrazolylgruppe bedeuten,
und deren Salze mit anorganischen oder organischen Säuren oder Basen, insbesondere deren physiologisch verträglichen Salzen, hergestellt gemäß Anspruch 1.

**3.** Benzimidazole der allgemeinen Formel I gemäß Anspruch 1, in der
$R_1$ eine Methylgruppe oder ein Chloratom,

$R_2$ eine 5-, 6- oder 7-gliedrige Alkyleniminogruppe, in welcher eine Methylengruppe durch eine Carbonyl- oder Sulfonylgruppe ersetzt ist,
eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können,
eine gegebenenfalls in 1-Stellung durch eine Alkylgruppe mit 1 bis 3 Kohlenstoffatomen substituierte Benzimidazol-2-yl- oder 4,5,6,7-Tetrahydro-benzimidazol-2-yl-gruppe, wobei der Phenylkern einer der vorstehend erwähnten Benzimidazolgruppen zusätzlich durch ein Fluoratom substituiert sein kann, eine Imidazo[1,2-a]pyridin-2-yl-, 5,6,7,8-Tetrahydro-imidazo[1,2-a]- pyridin-2-yl-, Imidazo[1,2-a]pyrimidin-2-yl- oder Imidazo-[2,1-b]thiazol-6-yl-gruppe oder
eine in 1-Stellung durch eine Alkylgruppe mit 1 bis 3 Kohlenstoffatomen substituierte Imidazol-4-yl-gruppe,
$R_3$ eine Alkylgruppe mit 1 bis 5 Kohlenstoffatomen oder eine Cycloalkylgruppe mit 3 bis 5 Kohlenstoffatomen und
$R_4$ eine Carboxy- oder 1H-Tetrazolylgruppe bedeuten,
und deren Salze mit anorganischen oder organischen Säuren oder Basen, insbesondere deren physiologisch verträglichen Salzen, hergestellt gemäß Anspruch 1.

**4.** Folgende Benzimidazole der allgemeinen Formel I hergestellt gemäß Anspruch 1:
(a) 4'-[[2-n-Propyl-4-methyl-6-(1-methylbenzimidazol-2-yl)benzimidazol-1-yl]-methyl]-biphenyl-2-carbonsäure,
(b) 4'-[[2-n-Propyl-4-methyl-6-(1-methylbenzimidazol-2-yl)benzimidazol-1-yl]-methyl]-2-(1H-tetrazol-5-yl)-biphenyl,
(c) 4'-[[2-n-Propyl-4-methyl-6-(butansultam-1-yl)-benzimidazol-1-yl]-methyl]-2-(1H-tetrazol-5-yl)-biphenyl,
(d) 4'-[[2-n-Butyl-6-(2,3-dimethylmaleinsäureimino) -4-methylbenzimidazol-1-yl]-methyl]-biphenyl-2-carbonsäure,
(e) 4'-[(2-Cyclopropyl-4-methyl-6-(1-methylbenzimidazol-2-yl)-benzimidazol-1-yl)-methyl]-biphenyl-2-carbonsäure,
(f) 4'-[(2-n-Propyl-4-methyl-6-(l-methyl-5-fluor-benzimidazol-2-yl)-benzimidazol-1-yl)-methyl]-biphenyl-2-carbonsäure,
(g) 4'-[(2-n-Propyl-4-methyl-6-(imidazo[1,2-a]pyrimidin-2-yl)-benzimidazol-1-yl)-methyl]-2-(1H-tetrazol-5-yl)-biphenyl,
(h) 4'-[(2-n-Propyl-4-methyl-6-(5,6,7,8-tetrahydro-imidazo-[1,2-a]pyridin-2-yl)-benzimidazol-1-yl)-methyl]-biphenyl-2-carbonsäure,
(i) 4'-[(2-n-Propyl-4-methyl-6-(5,6,7,8-tetrahydro-imidazo-[1,2-a]pyridin-2-yl)-benzimidazol-1-yl)-methyl]-2-(1H-tetrazol-5-yl)-biphenyl,
(j) 4'-[(2-n-Propyl-4-chlor-6-(1-methylbenzimidazol-2-yl)-benzimidazol-1-yl)-methyl]-2-(1H-tetrahol-5-yl)-biphenyl,
(k) 4'-[[2-n-Propyl-4-methyl-6-(imidazo[2,1-b]thiazol-6-yl)-benzimidazol-1-yl]-methyl]-biphenyl-2-carbonsäure,
(l) 4'-[[2-Ethyl-4-methyl-6-(butansultam-1-yl)-benzimidazol-1-yl]-methyl]-2-(1H-tetrazol-5-yl)-biphenyl,
(m) 4'-[[2-n-Butyl-4-methyl-6-(1-methylbenzimidazol-2-yl)-benzimidazol-1-yl]-methyl]-2-(1H-tetrazol-5-yl)-biphenyl,
(n) 4'-[[2-n-Propyl-4-methyl-6-(imidazo[1,2-a]pyridin-2-yl)-benzimidazol-1-yl]-methyl]-biphenyl-2-carbonsäure,
(o) 4'-[[2-n-Propyl-4-methyl-6-(imidazo[1,2-a]pyridin-2-yl)-benzimidazol-1-yl]-methyl]-2-(1H-tetrazol-5-yl)-biphenyl,
(p) 4'-[[2-n-Propyl-4-methyl-6-(imidazo[2,1-b]thiazol-6-yl)-benzimidazol-1-yl]-methyl]-2-(1H-tetrazol-5-yl)-biphenyl,
(q) 4'-[(2-n-Propyl-4-methyl-6-(1-methyl-6-fluor-benzimidazol-2-yl)-benzimidazol-1-yl)-methyl]-biphenyl-2-carbonsäure und
(r) 4'-[(2-Ethyl-4-methyl-6-(5,6,7,8-tetrahydro-imidazo-[1,2-a]pyridin-2-yl)-benzimidazol-1-yl)-methyl]-biphenyl-2-carbonsäure,
und deren Salze mit anorganischen oder organischen Säuren oder Basen, insbesondere deren physiologisch verträglichen Salzen.

**5.** 4'-[[2-n-Propyl-4-methyl-6-(1-methylbenzimidazol-2-yl)-benzimidazol-1-yl]-methyl]-biphenyl-2-carbonsäure und dessen Salze mit anorganischen oder organischen Säuren oder Basen, insbesondere dessen physiologisch verträglichen Salze, hergestellt gemäß Anspruch 1.

**6.** 4'-[(2-Ethyl-4-methyl-6-(5,6,7,8-tetrahydro-imidazo[1,2-a]-pyridin-2-yl)-benzimidazol-1-yl)-methyl]-biphenyl-2-carbonsäure und dessen Salze mit anorganischen oder organischen Säuren oder Basen, insbesondere dessen physiologisch verträglichen Salze, hergestellt gemäß Anspruch 1.

**7.** Verfahren zur Herstellung eines Arzneimittels, dadurch gekennzeichnet, daß auf nichtchemischem Wege eine Verbindung hergestellt nach einem der Ansprüche 1 bis 6 in einen oder mehrere inerte Trägerstoffe und/oder

Verdünnungsmittel eingearbeitet wird.

## Claims

### Claims for the following Contracting States : AT, BE, CH, DE, DK, FR, GB, IT, LI, LU, NL, SE

**1.** Benzimidazoles of general formula

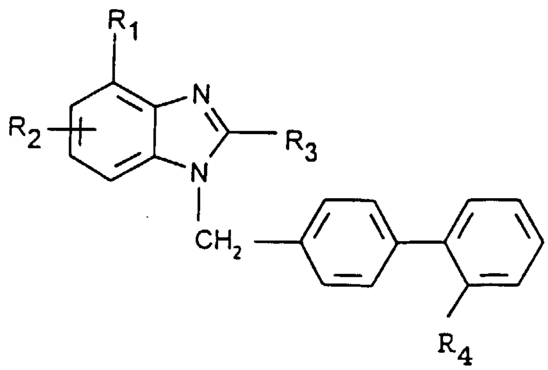

(I),

wherein
$R_1$ represents a fluorine, chlorine or bromine atom, an alkyl, cycloalkyl, fluoromethyl, difluoromethyl or trifluoromethyl group and
$R_2$ represents a 5-, 6- or 7-membered alkyleneimino or alkenyleneimino group, optionally substituted by one or two alkyl groups or by a tetramethylene or pentamethylene group, wherein a methylene group may be replaced by a carbonyl or sulphonyl group,
a maleic acid imido group optionally mono- or disubstituted by an alkyl or phenyl group, whilst the substituents may be identical or different,
a benzimidazol-2-yl or 4,5,6,7-tetrahydro-benzimidazol-2-yl group optionally substituted in the 1-position by $C_{1-6}$-alkyl or a cycloalkyl group, whilst the phenyl nucleus of one of the abovementioned benzimidazole groups may additionally be substituted by a fluorine atom or by a methyl or trifluoromethyl group, $R_2$ may represent an imidazo [2,1-b]thiazol-6-yl,
imidazo[1,2-a]pyridin-2-yl, 5,6,7,8-tetrahydroimidazo[1,2-a]pyridin-2-yl, imidazo[1,2-a]pyrimidin-2-yl, imidazo [4,5-b]pyridin-2-yl, imidazo [4,5-c] pyridin-2-yl, imidazo[1,2-c]pyrimidin-2-yl, imidazo[1,2-a]pyrazin-2-yl, imidazo [1,2-b]-pyridazin-2-yl, purin-8-yl,
imidazo[4,5-b]pyrazin-2-yl, imidazo[4,5-c]pyridazin-2-yl or imidazo [4,5-d]pyridazin-2-yl group,
a pyridyl group or
a carbon attached imidazolyl group optionally substituted in the 1-position by an alkyl or benzyl group, and which may also be substituted in the carbon skeleton by an alkyl group,
$R_3$ represents a $C_{1-5}$-alkyl group or a $C_{3-5}$-cycloalkyl group and
$R_4$ represents a carboxy or IH-tetrazolyl group,
and the salts thereof with inorganic or organic acids or bases,
whilst, unless otherwise specified, an alkyl moiety as mentioned hereinbefore may in each case contain 1 to 3 carbon atoms and a cycloalkyl moiety mentioned above may contain from 3 to 7 carbon atoms.

**2.** Benzimidazoles of general formula I according to claim 1, wherein
$R_1$ represents a chlorine atom, or a $C_{1-3}$-alkyl or a trifluoromethyl group,
a 5-, 6- or 7-membered alkyleneimino group wherein a methylene group is replaced by a carbonyl or sulphonyl group,
a maleic acid imido group optionally mono- or disubstituted by a $C_{1-3}$-alkyl or phenyl group, whilst the substituents may be identical or different,
a benzimidazol-2-yl or 4,5,6,7-tetrahydro-benzimidazol-2-yl group optionally substituted in the 1-position by a $C_{1-6}$-alkyl or by a cycloalkyl group, whilst the phenyl nucleus of one of the abovementioned benzimidazole groups may additionally be substituted by a fluorine atom or by a methyl or trifluoromethyl group, or $R_2$ may represent an

imidazo[2,1-b]thiazol-6-yl, imidazo[1,2-a]pyridin-2-yl, 5,6,7,8-tetrahydroimidazo[1,2-a]pyridin-2-yl, imidazo[1,2-a]pyrimidin-2-yl, imidazo [4,5-b]pyridin-2-yl, imidazo[4,5-c]pyridin-2-yl, imidazo[1,2-c]pyrimidin-2-yl, imidazo[1,2-a]pyrazin-2-yl, imidazo [1,2-b]-pyridazin-2-yl, purin-8-yl, imidazo [4,5-b]pyrazin-2-yl, imidazo [4,5-c]pyridazin-2-yl or imidazo [4,5-d]pyridazin-2-yl group, a pyridyl group or an imidazol-4-yl group substituted in the 1-position by a $C_{1-3}$ alkyl group or by a benzyl group which may also be substituted in the carbon skeleton by a $C_{1-3}$ alkyl group, $R_3$ represents a $C_{1-5}$-alkyl group or a $C_{3-5}$-cycloalkyl group and $R_4$ represents a carboxy or lH-tetrazolyl group, and the salts thereof with inorganic or organic acids or bases.

**3.** Benzimidazoles of general formula I according to claim 1, wherein $R_1$ represents a methyl group or a chlorine atom and $R_2$ represents a 5-, 6- or 7-membered alkyleneimino group, wherein a methylene group is replaced by a carbonyl or sulphonyl group, a maleic acid imido group optionally mono- or disubstituted by a $C_{1-3}$-alkyl or phenyl group, whilst the substituents may be identical or different, a benzimidazol-2-yl or 4,5,6,7-tetrahydro-benzimidazol-2-yl group optionally substituted in the 1-position by a $C_{1-3}$-alkyl group, whilst the phenyl nucleus of one of the abovementioned benzimidazole groups may additionally be substituted by a fluorine atom, or $R_2$ may represent an imidazo[1,2-a]-pyridin-2-yl group, 5,6,7,8-tetrahydroimidazo [1,2-a]-pyridin-2-yl, imidazo[1,2-a]pyrimidin-2-yl or imidazo [2,1-b]thiazol-6-yl group, an imidazol-4-yl group substituted in the 1-position by a $C_{1-3}$ alkyl group, $R_3$ represents a $C_{1-5}$-alkyl group or a $C_{3-5}$-cycloalkyl group and $R_4$ represents a carboxy or lH-tetrazolyl group, and the salts thereof with inorganic or organic acids or bases.

**4.** The following benzimidazoles of general formula I according to claim 1:

(a) 4'-[[2-n-propyl-4-methyl-6-(1-methylbenzimidazol-2-yl)-benzimidazol-1-yl]-methyl]-biphenyl-2-carboxylic acid,

(b) 4'-[[2-n-propyl-4-methyl-6-(1-methylbenzimidazol-2-yl)-benzimidazol-1-yl]-methyl]-2-(1H-tetrazol-5-yl)-biphenyl,

(c) 4'-[[2-n-propyl-4-methyl-6-(butanesultam-1-yl)-benzimidazol-1-yl]-methyl]-2-(1H-tetrazol-5-yl)-biphenyl,

(d) 4'-[[2-n-butyl-6-(2,3-dimethylmaleic acid imino)-4-methylbenzimidazol-1-yl]-methyl]-biphenyl-2-carboxylic acid,

(e) 4'-[(2-cyclopropyl-4-methyl-6-(1-methylbenzimidazol-2-yl)-benzimidazol-1-yl)-methyl]-biphenyl-2-carboxylic acid,

(f) 4'-[(2-n-propyl-4-methyl-6-(1-methyl-5-fluorobenzimidazol-2-yl)-benzimidazol-1-yl)-methyl]-biphenyl-2-carboxylic acid,

(g) 4'-[(2-n-propyl-4-methyl-6-(imidazo[1,2-a]pyrimidin-2-yl)-benzimidazol-1-yl)-methyl]-2-(1H-tetrazol-5-yl)-biphenyl,

(h) 4'-[(2-n-propyl-4-methyl-6-(5,6,7,8-tetrahydroimidazo[1,2-a]pyridin-2-yl)-benzimidazol-1-yl)-methyl]-biphenyl-2-carboxylic acid,

(i) 4'-[(2-n-propyl-4-methyl-6-(5,6,7,8-tetrahydroimidazo[1,2-a]pyridin-2-yl)-benzimidazol-1-yl)-methyl]-2-(1H-tetrazol-5-yl)-biphenyl,

(j) 4'-[(2-n-propyl-4-chloro-6-(1-methylbenzimidazol-2-yl)-benzimidazol-1-yl)-methyl]-2-(1H-tetrazol-5-yl)-biphenyl,

(k) 4'-[[2-n-propyl-4-methyl-6-(imidazo[2,1-b]thiazol-6-yl)-benzimidazol-1-yl]-methyl]-biphenyl-2-carboxylic acid,

(l) 4'-[[2-ethyl-4-methyl-6-(butanesultam-1-yl)-benzimidazol-1-yl]-methyl]-2-(1H-tetrazol-5-yl)-biphenyl,

(m) 4'-[[2-n-butyl-4-methyl-6-(1-methylbenzimidazol-2-yl)-benzimidazol-1-yl]-methyl]-2-(1H-tetrazol-5-yl)-biphenyl,

(n) 4'-[[2-n-propyl-4-methyl-6-(imidazo[1,2-a]pyridin-2-yl)-benzimidazol-1-yl]-methyl]-biphenyl-2-carboxylic acid,

(o) 4'-[[2-n-propyl-4-methyl-6-(imidazo[1,2-a]pyridin-2-yl)-benzimidazol-1-yl]-methyl]-2-(1H-tetrazol-5-yl)-biphenyl,

(p) 4'-[[2-n-propyl-4-methyl-6-(imidazo[2,1-b]thiazol-6-yl)-benzimidazol-1-yl]-methyl]-2-(1H-tetrazol-5-yl)-biphenyl,

(q) 4'-[(2-n-propyl-4-methyl-6-(1-methyl-6-fluorobenzimidazol-2-yl)-benzimidazol-1-yl)-methyl]-biphenyl-2-carboxylic acid, and

(r) 4'-[(2-ethyl-4-methyl-6-(5,6,7,8-tetrahydroimidazo[1,2-a]pyridin-2-yl)-benzimidazol-1-yl)-methyl]-biphenyl-2-carboxylic acid,
and the salts thereof with inorganic or organic acids or bases.

**5.** 4'-[[2-n-Propyl-4-methyl-6-(1-methylbenzimidazol-2-yl)-benzimidazol-1-yl]-methyl]-biphenyl-2-carboxylic acid and the salts thereof with inorganic or organic acids or bases.

**6.** 4'-[(2-Ethyl-4-methyl-6-(5,6,7,8-tetrahydroimidazo[1,2-a]pyridin-2-yl)-benzimidazol-1-yl)-methyl]-biphenyl-2-carboxylic acid and the salts thereof with inorganic or organic acids or bases.

**7.** Physiologically acceptable salts of the compounds according to at least one of claims 1 to 6 with inorganic or organic acids or bases.

**8.** Pharmaceutical compositions containing a compound according to at least one of claims 1 to 6 or a physiologically acceptable salt according to claim 7 optionally together with one or more inert carriers and/or diluents.

**9.** Use of a compound according to at least one of claims 1 to 7 for preparing a pharmaceutical composition with an angiotensin-antagonist activity.

**10.** Process for preparing a pharmaceutical composition according to claim 8, characterised in that a compound according to at least one of claims 1 to 7 is incorporated in one or more inert carriers and/or diluents by a non-chemical method.

**11.** Process for preparing the benzimidazoles according to claims 1 to 7, characterised in that

a) a compound of general formula

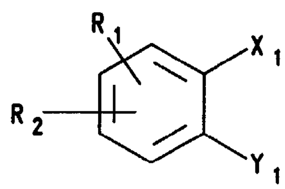

(II)

wherein
$R_1$ and $R_2$ are defined as in claims 1 to 6,
one of the groups $X_1$ or $Y_1$ represents a group of general formula

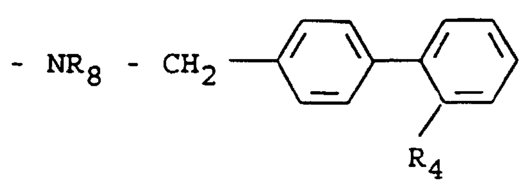

and the other group $X_1$ or $Y_1$ represents a group of the general formula

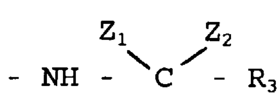

wherein
$R_2$ and $R_4$ are defined as in claims 1 to 6,
$R_8$ represents a hydrogen atom or an $R_3CO$- group, wherein $R_3$ is defined as hereinbefore,
$Z_1$ and $Z_2$, which may be identical or different, represent optionally substituted amino groups or hydroxy or mercapto groups optionally substituted by lower alkyl groups or
$Z_1$ and $Z_2$ together represent an oxygen or sulphur atom, an optionally $C_{1-3}$-alkyl substituted imino group, an

alkylenedioxy or alkylenedithio group, each having 2 or 3 carbon atoms, but one of the groups $X_1$ or $Y_1$ must represent a group of general formula

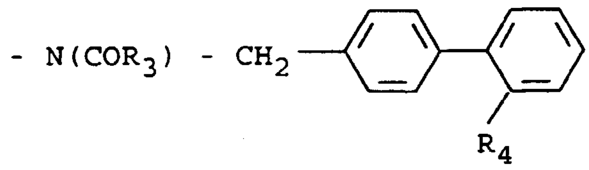

or

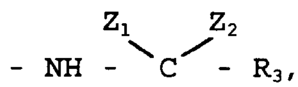

is cyclised and a corresponding N-oxide which might thus be obtained is reduced or

b) a benzimidazole of general formula

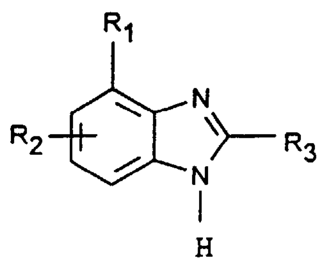

(III),

wherein
$R_1$ to $R_3$ are defined as in claims 1 to 6,
is reacted with a biphenyl compound of general formula

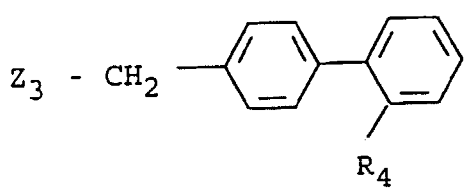

(IV),

wherein
$R_4$ is defined as in claims 1 to 6 and
$Z_3$ represents a nucleophilic leaving group, or

c) in order to prepare a compound of general formula I wherein $R_4$ represents a carboxy group, a compound of general formula

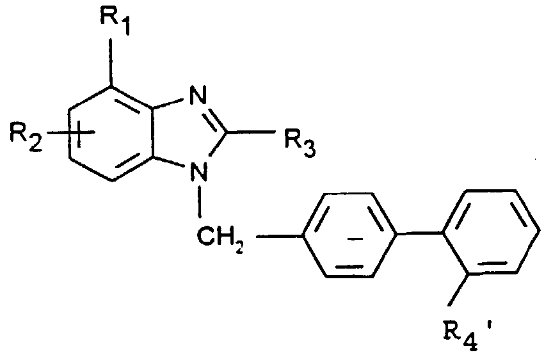

(V),

wherein
$R_1$ to $R_3$ are defined as in claims 1 to 6 and
$R_4$' represents a group which may be converted into a carboxy group by hydrolysis, thermolysis or hydrogenolysis, is converted into a corresponding carboxy compound or

d) in order to prepare a compound of general formula I
a maleic acid imido group optionally mono- or disubstituted by a $C_{1-3}$-alkyl or phenyl group, whilst the substituents may be identical or different,
a benzimidazol-2-yl or 4,5,6,7-tetrahydro-benzimidazol-2-yl group optionally substituted in the 1-position by a $C_{1-3}$-alkyl group, whilst the phenyl nucleus of one of the abovementioned benzimidazole groups may additionally be substituted by a fluorine atom, or $R_2$ may represent an imidazo[1,2-a]-pyridin-2-yl group, 5,6,7,8-tetrahydroimidazo[1,2-a]-pyridin-2-yl, imidazo[1,2-a]pyrimidin-2-yl or imidazo[2,1-b]thiazol-6-yl group,
an imidazol-4-yl group substituted in the 1-position by a $C_{1-3}$ alkyl group,
$R_3$ represents a $C_{1-5}$-alkyl group or a $C_{3-5}$-cycloalkyl group and
$R_4$ represents a carboxy or IH-tetrazolyl group,
and the salts thereof with inorganic or organic acids or bases, particularly the physiologically acceptable salts thereof, prepared according to claim 1.

4. The following benzimidazoles of general formula I prepared according to claim 1:
(a) 4'-[[2-n-propyl-4-methyl-6-(l-methylbenzimidazol-2-yl)-benzimidazol-1-yl]-methyl]-biphenyl-2-carboxylic acid,
(b) 4'-[[2-n-propyl-4-methyl-6-(1-methylbenzimidazol-2-yl)-benzimidazol-1-yl]-methyl]-2-(1H-tetrazol-5-yl)-biphenyl,
(c) 4'-[[2-n-propyl-4-methyl-6- (butanesultam-1-yl)-
a compound of general formula

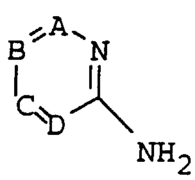

(VIII),

wherein
one of the groups A, B, C or D represents an optionally methyl-substituted methine group or a nitrogen atom and the remaining groups A, B, C or D represent methine groups or
A and B each represent methine and the -C=D- group represents a sulphur atom,
is reacted with a compound of general formula

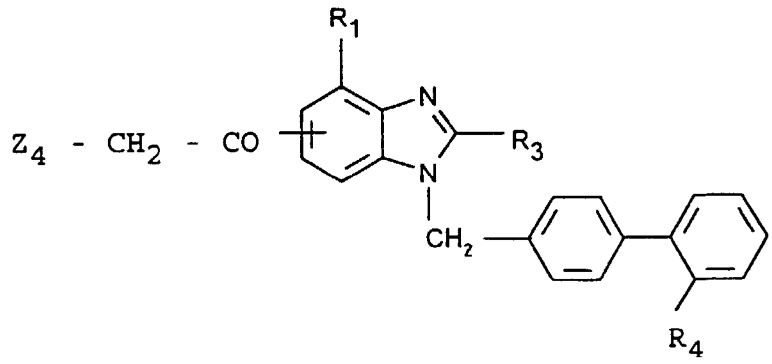

(IX),

wherein

$R_1$, $R_3$ and $R_4$ are defined as in claims 1 to 6 and $Z_4$ represents a nucleophilic leaving group such as a halogen atom, e.g. a chlorine or bromine atom, or

g) in order to prepare compounds of general formula I wherein $R_2$ represents one of the benzimidazol-2-yl, imidazo [4,5-b]pyridin-2-yl, imidazo[4,5-c]pyridin-2-yl, imidazo[4,5-b] pyrazin-2-yl, imidazo[4,5-c]pyridazin-2-yl, imidazo[4,5-d]pyridazin-2-yl or purin-8-yl groups mentioned in claims 1 to 6,
a compound of general formula

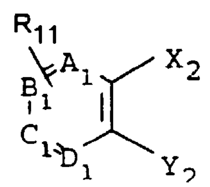

(X),

wherein

none, one or two of the groups $A_1$, $B_1$, $C_1$ or $D_1$ represents a nitrogen atom and
the remaining groups $A_1$, $B_1$, $C_1$ or $D_1$ represent methine groups,
$R_{11}$ represents a hydrogen or fluorine atom or a methyl or trifluoromethyl group,
one of the groups $X_2$ or $Y_2$ represents an $R_{13}$-NH- group and the other $X_2$ or $Y_2$ group represents a group of general formula

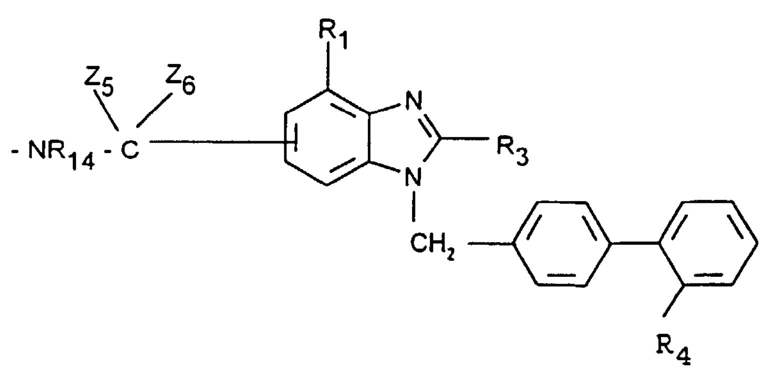

wherein

$R_1$, $R_3$ and $R_4$ are defined as in claims 1 to 6,
one of the groups $R_{13}$ or $R_{14}$ represents a hydrogen atom and the other $R_{13}$ or $R_{14}$ group represents a hydrogen atom, a $C_{1-6}$-alkyl group or a $C_{3-7}$-cycloalkyl group,
$Z_5$ and $Z_6$, which may be identical or different, represent optionally substituted amino groups or hydroxy or mercapto groups optionally substituted by lower alkyl groups or

$Z_5$ and $Z_6$ together represent an oxygen or sulphur atom, an optionally $C_{1-3}$-alkyl-substituted imino group, an alkylenedioxy or alkylenedithio group each having 2 or 3 carbon atoms,
is cyclised and a corresponding N-oxide which might be thus obtained is reduced and a compound thus obtained is optionally subsequently hydrolysed, and
if necessary a protecting group used during the reactions a) to g) in order to protect reactive groups is cleaved and/or
if desired an isomer mixture thus obtained is resolved into its isomers, and/or
a compound of general formula I thus obtained is converted into a salt thereof, more particularly for pharmaceutical use a physiologically acceptable salt thereof with an inorganic or organic acid or base.

**Claims for the following Contracting States : ES, GR, PT**

1. Process for preparing the benzimidazoles of general formula

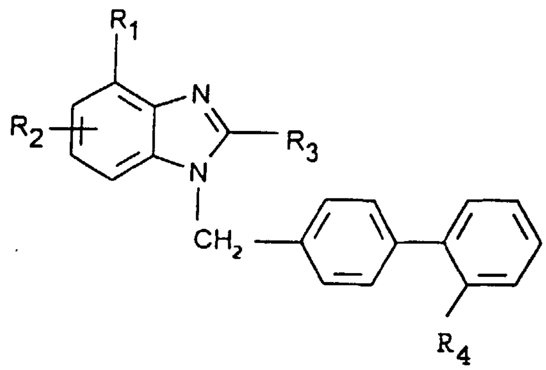

(I),

wherein
$R_1$ represents a fluorine, chlorine or bromine atom, an alkyl, cycloalkyl, fluoromethyl, difluoromethyl or trifluoromethyl group and
$R_2$ represents a 5-, 6- or 7-membered alkyleneimino or alkenyleneimino group, optionally substituted by one or two alkyl groups or by a tetramethylene or pentamethylene group, wherein a methylene group may be replaced by a carbonyl or sulphonyl group,
a maleic acid imido group optionally mono- or disubstituted by an alkyl or phenyl group, whilst the substituents may be identical or different,
a benzimidazol-2-yl or 4,5,6,7-tetrahydro-benzimidazol-2-yl group optionally substituted in the 1-position by $C_{1-6}$-alkyl or a cycloalkyl group, whilst the phenyl nucleus of one of the abovementioned benzimidazole groups may additionally be substituted by a fluorine atom or by a methyl or trifluoromethyl group, $R_2$ may represent an imidazo [2,1-b]thiazol-6-yl,
imidazo[1,2-a]pyridin-2-yl, 5,6,7,8-tetrahydroimidazo[1,2-a]pyridin-2-yl, imidazo[1,2-a]pyrimidin-2-yl, imidazo [4,5-b]pyridin-2-yl, imidazo[4,5-c]pyridin-2-yl, imidazo[1,2-c]pyrimidin-2-yl, imidazo[l,2-a]pyrazin-2-yl, imidazo [1,2-b]-pyridazin-2-yl, purin-8-yl,
imidazo [4,5-b]pyrazin-2-yl, imidazo[4,5-c]pyridazin-2-yl or imidazo [4,5-d]pyridazin-2-yl group,
a pyridyl group or
a carbon attached imidazolyl group optionally substituted in the 1-position by an alkyl or benzyl group, and which may also be substituted in the carbon skeleton by an alkyl group,
$R_3$ represents a $C_{1-5}$-alkyl group or a $C_{3-5}$-cycloalkyl group and
$R_4$ represents a carboxy or lH-tetrazolyl group,
and the salts thereof with inorganic or organic acids or bases, particularly the physiologically acceptable salts thereof,
whilst, unless otherwise specified, an alkyl moiety as mentioned hereinbefore may in each case contain 1 to 3 carbon atoms and a cycloalkyl moiety mentioned above may contain from 3 to 7 carbon atoms,
characterised in that

a) a compound of general formula

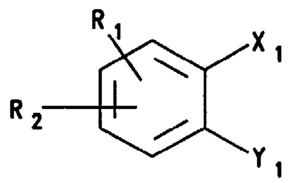

(II)

wherein
$R_1$ and $R_2$ are defined as hereinbefore,
one of the groups $X_1$ or $Y_1$ represents a group of general formula

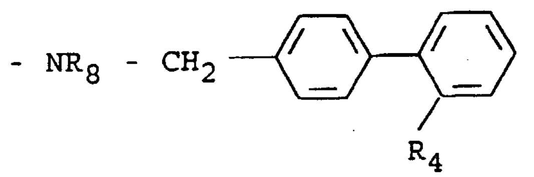

and the other group $X_1$ or $Y_1$ represents a group of the general formula

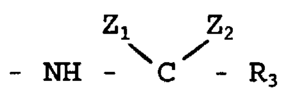

wherein
$R_2$ and $R_4$ are defined as hereinbefore,
$R_8$ represents a hydrogen atom or an $R_3CO$- group, wherein $R_3$ is defined as hereinbefore,
$Z_1$ and $Z_2$, which may be identical or different, represent optionally substituted amino groups or hydroxy or mercapto groups optionally substituted by lower alkyl groups or
$Z_1$ and $Z_2$ together represent an oxygen or sulphur atom, an optionally $C_{1-3}$-alkyl substituted imino group, an alkylenedioxy or alkylenedithio group, each having 2 or 3 carbon atoms, but one of the groups $X_1$ or $Y_1$ must represent a group of general formula

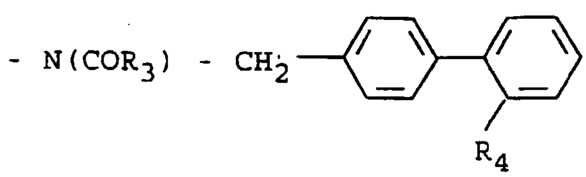

or

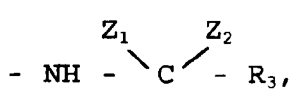

is cyclised and a corresponding N-oxide which might thus be obtained is reduced or

b) a benzimidazole of general formula

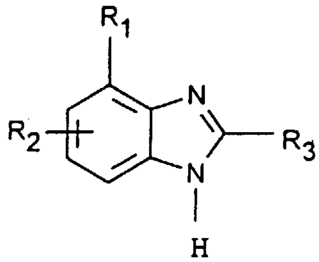

(III),

wherein
$R_1$ to $R_3$ are defined as hereinbefore,
is reacted with a biphenyl compound of general formula

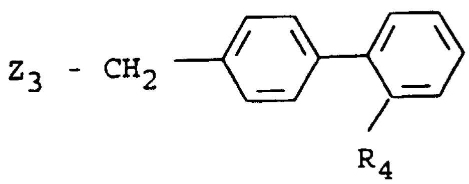

(IV),

wherein
$R_4$ is defined as hereinbefore and
$Z_3$ represents a nucleophilic leaving group, or

c) in order to prepare a compound of general formula I wherein $R_4$ represents a carboxy group, a compound of general formula

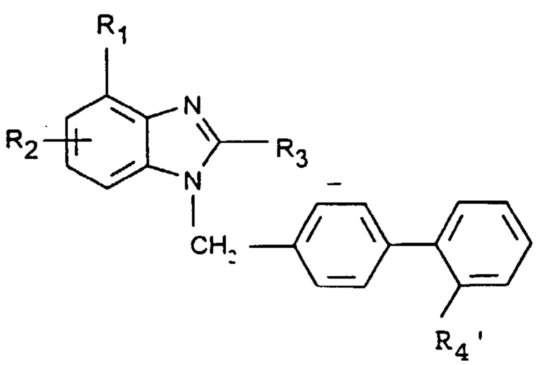

(V),

wherein
$R_1$ to $R_3$ are defined as hereinbefore and
$R_4$' represents a group which may be converted into a
carboxy group by hydrolysis, thermolysis or hydrogenolysis, is converted into a corresponding carboxy compound or

d) in order to prepare a compound of general formula I wherein $R_4$ represents a 1H-tetrazolyl group, a protecting group is split off from a compound of general formula

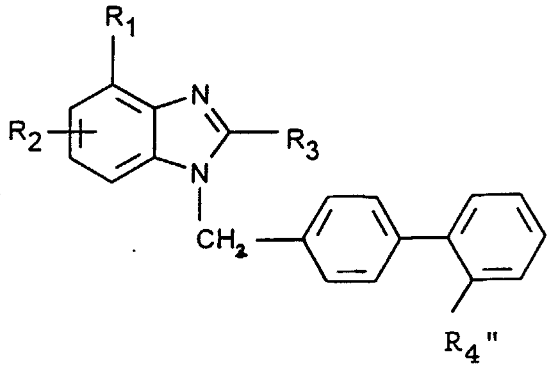

(VI),

wherein
$R_1$, $R_2$ and $R_3$ are defined as hereinbefore and
$R_4$" represents a 1H-tetrazolyl group protected in the 1-or 3-position by a protecting group, or

e) in order to prepare a compound of general formula I wherein $R_4$ represents a 1H-tetrazolyl group, a compound of general formula

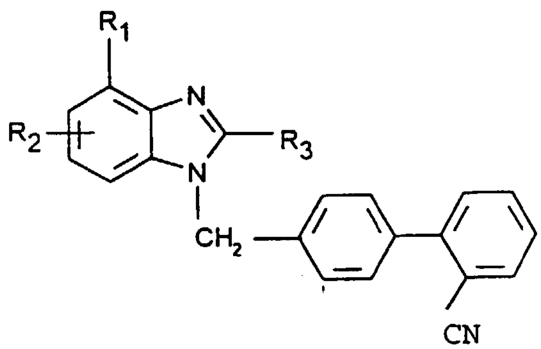

(VII),

wherein
$R_1$ to $R_3$ are defined as hereinbefore, is reacted with hydrazoic acid or the salts thereof or

f) in order to prepare compounds of general formula I wherein $R_2$ represents one of the imidazo[1,2-a]-pyridin-2-yl, imidazo[l,2-a]pyrimidin-2-yl, imidazo[1,2-c]-pyrimidin-2-yl, imidazo[1,2-a]pyrazin-2-yl, imidazo-[1,2-b]pyridazin-2-yl or imidazo[2,1-b]thiazol-6-yl groups mentioned hereinbefore,
a compound of general formula

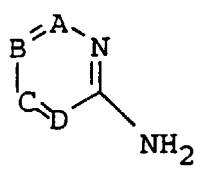

(VIII),

wherein
one of the groups A, B, C or D represents an optionally methyl-substituted methine group or a nitrogen atom and the remaining groups A, B, C or D represent methine groups or
A and B each represent methine and the -C=D- group represents a sulphur atom,
is reacted with a compound of general formula

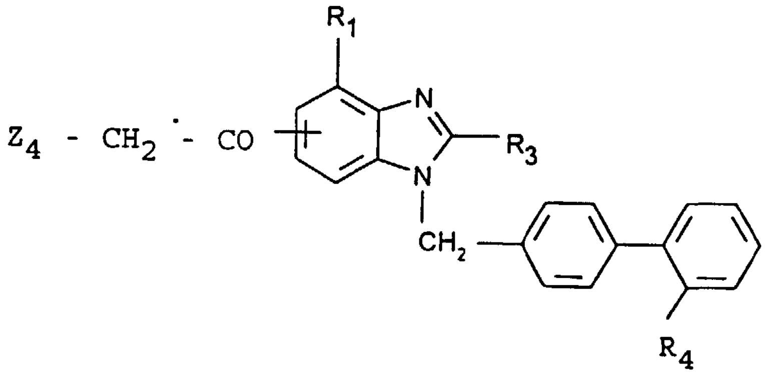

(IX),

wherein
$R_1$, $R_3$ and $R_4$ are defined as hereinbefore and
$Z_4$ represents a nucleophilic leaving group such as a halogen atom, e.g. a chlorine or bromine atom, or

g) in order to prepare compounds of general formula I wherein $R_2$ represents one of the benzimidazol-2-yl, imidazo [4,5-b]pyridin-2-yl, imidazo [4,5-c]pyridin-2-yl, imidazo[4,5-b]pyrazin-2-yl, imidazo[4,5-c]pyridazin-2-yl, imidazo[4,5-d]pyridazin-2-yl or purin-8-yl groups mentioned in claims 1 to 6,
a compound of general formula

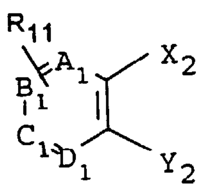

(X),

wherein
none, one or two of the groups $A_1$, $B_1$, $C_1$ or $D_1$ represents a nitrogen atom and
the remaining groups $A_1$, $B_1$, $C_1$ or $D_1$ represent methine groups,
$R_{11}$ represents a hydrogen or fluorine atom or a methyl or trifluoromethyl group,
one of the groups $X_2$ or $Y_2$ represents an $R_{13}$-NH- group and the other $X_2$ or $Y_2$ group represents a group of general formula

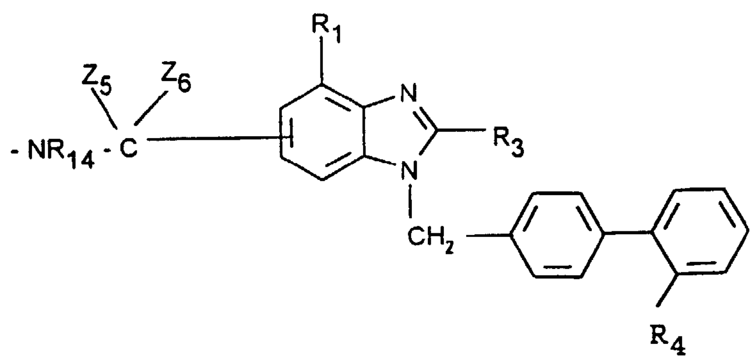

wherein
$R_1$, $R_3$ and $R_4$ are defined as hereinbefore,
one of the groups $R_{13}$ or $R_{14}$ represents a hydrogen atom and the other $R_{13}$ or $R_{14}$ group represents a hydrogen atom, a $C_{1-6}$-alkyl group or a $C_{3-7}$-cycloalkyl group,

$Z_5$ and $Z_6$, which may be identical or different, represent optionally substituted amino groups or hydroxy or mercapto groups optionally substituted by lower alkyl
groups or
$Z_5$ and $Z_6$ together represent an oxygen or sulphur atom, an optionally $C_{1-3}$-alkyl-substituted imino group, an alkylenedioxy or alkylenedithio group each having 2 or 3 carbon atoms,
is cyclised and a corresponding N-oxide which might be thus obtained is reduced and a compound thus obtained is optionally subsequently hydrolysed, and
if necessary a protecting group used during the reactions a) to g) in order to protect reactive groups is cleaved and/or
if desired an isomer mixture thus obtained is resolved into its isomers, and/or
a compound of general formula I thus obtained is converted into a salt thereof, more particularly for pharmaceutical use a physiologically acceptable salt thereof with an inorganic or organic acid or base.

**2.** Benzimidazoles of general formula I according to claim 1, wherein
$R_1$ represents a chlorine atom, or a $C_{1-3}$-alkyl or a trifluoromethyl group,
a 5-, 6- or 7-membered alkyleneimino group wherein a methylene group is replaced by a carbonyl or sulphonyl group,
a maleic acid imido group optionally mono- or disubstituted by a $C_{1-3}$-alkyl or phenyl group, whilst the substituents may be identical or different,
a benzimidazol-2-yl or 4,5,6,7-tetrahydro-benzimidazol-2-yl group optionally substituted in the 1-position by a $C_{1-6}$-alkyl or by a cycloalkyl group, whilst the phenyl nucleus of one of the abovementioned benzimidazole groups may additionally be substituted by a fluorine atom or by a methyl or trifluoromethyl group, or $R_2$ may represent an imidazo[2,1-b]thiazol-6-yl,
imidazo[1,2-a]pyridin-2-yl, 5,6,7,8-tetrahydroimidazo[1,2-a]pyridin-2-yl, imidazo[1,2-a]pyrimidin-2-yl, imidazo [4,5-b]pyridin-2-yl, imidazo[4,5-c]pyridin-2-yl, imidazo[1,2-c]pyrimidin-2-yl, imidazo[1,2-a]pyrazin-2-yl, imidazo [1,2-b]-pyridazin-2-yl, purin-8-yl,
imidazo[4,5-b]pyrazin-2-yl, imidazo [4,5-c]pyridazin-2-yl or imidazo [4,5-d]pyridazin-2-yl group,
a pyridyl group or
an imidazol-4-yl group substituted in the 1-position by a $C_{1-3}$ alkyl group or by a benzyl group which may also be substituted in the carbon skeleton by a $C_{1-3}$ alkyl group,
$R_3$ represents a $C_{1-5}$-alkyl group or a $C_{3-5}$-cycloalkyl group and
$R_4$ represents a carboxy or IH-tetrazolyl group,
and the salts thereof with inorganic or organic acids or bases, particularly the physiologically acceptable salts thereof, prepared according to claim 1.

**3.** Benzimidazoles of general formula I according to claim 1, wherein
$R_1$ represents a methyl group or a chlorine atom and
$R_2$ represents a 5-, 6- or 7-membered alkyleneimino group, wherein a methylene group is replaced by a carbonyl or sulphonyl group,
a maleic acid imido group optionally mono- or disubstituted by a $C_{1-3}$-alkyl or phenyl group, whilst the substituents may be identical or different,
a benzimidazol-2-yl or 4,5,6,7-tetrahydro-benzimidazol-2-yl group optionally substituted in the 1-position by a $C_{1-3}$-alkyl group, whilst the phenyl nucleus of one of the abovementioned benzimidazole groups may additionally be substituted by a fluorine atom, or $R_2$ may represent an imidazo[1,2-a]-pyridin-2-yl group, 5,6,7,8-tetrahydroimidazo [1,2-a]-pyridin-2-yl, imidazo[1,2-a]pyrimidin-2-yl or imidazo [2,1-b]thiazol-6-yl group,
an imidazol-4-yl group substituted in the 1-position by a $C_{1-3}$ alkyl group,
$R_3$ represents a $C_{1-5}$-alkyl group or a $C_{3-5}$-cycloalkyl group and
$R_4$ represents a carboxy or IH-tetrazolyl group,
and the salts thereof with inorganic or organic acids or bases, particularly the physiologically acceptable salts thereof, prepared according to claim 1.

**4.** The following benzimidazoles of general formula I prepared according to claim 1:
(a) 4'-[[2-n-propyl-4-methyl-6-(1-methylbenzimidazol-2-yl)-benzimidazol-1-yl]-methyl]-biphenyl-2-carboxylic acid,
(b) 4'-[[2-n-propyl-4-methyl-6-(1-methylbenzimidazol-2-yl)-benzimidazol-1-yl]-methyl]-2-(1H-tetrazol-5-yl)-biphenyl,
(c) 4'-[[2-n-propyl-4-methyl-6-(butanesultam-1-yl)-benzimidazol-1-yl]-methyl]-2-(1H-tetrazol-5-yl)-biphenyl,
(d) 4'-[[2-n-butyl-6-(2,3-dimethylmaleic acid imino)-4-methylbenzimidazol-1-yl]-methyl]-biphenyl-2-carboxylic acid,

(e) 4'-[(2-cyclopropyl-4-methyl-6-(1-methylbenzimidazol-2-yl)-benzimidazol-1-yl)-methyl]-biphenyl-2-carboxylic acid,
(f) 4'-[(2-n-propyl-4-methyl-6-(1-methyl-5-fluorobenzimidazol-2-yl)-benzimidazol-1-yl)-methyl]-biphenyl-2-carboxylic acid,
(g) 4'-[(2-n-propyl-4-methyl-6-(imidazo[1,2-a]pyrimidin-2-yl)-benzimidazol-1-yl)-methyl]-2-(1H-tetrazol-5-yl)-biphenyl,
(h) 4'-[(2-n-propyl-4-methyl-6-(5,6,7,8-tetrahydroimidazo[1,2-a]pyridin-2-yl)-benzimidazol-1-yl)-methyl]-biphenyl-2-carboxylic acid,
(i) 4'-[(2-n-propyl-4-methyl-6-(5,6,7,8-tetrahydroimidazo[1,2-a]pyridin-2-yl)-benzimidazol-1-yl)-methyl]-2-(1H-tetrazol-5-yl)-biphenyl,
(j) 4'-[(2-n-propyl-4-chloro-6-(1-methylbenzimidazol-2-yl)-benzimidazol-1-yl)-methyl]-2-(1H-tetrazol-5-yl)-biphenyl,
(k) 4'-[[2-n-propyl-4-methyl-6-(imidazo[2,1-b]thiazol-6-yl)-benzimidazol-1-yl]-methyl]-biphenyl-2-carboxylic acid,
(l) 4'-[[2-ethyl-4-methyl-6-(butanesultam-1-yl)- benzimidazol-1-yl]-methyl]-2-(1H-tetrazol-5-yl)-biphenyl,
(m) 4'-[[2-n-butyl-4-methyl-6-(1-methylbenzimidazol-2-yl)-benzimidazol-1-yl]-methyl]-2-(1H-tetrazol-5-yl)-biphenyl,
(n) 4'-[[2-n-propyl-4-methyl-6-(imidazo[1,2-a]pyridin-2-yl)-benzimidazol-1-yl]-methyl]-biphenyl-2-carboxylic acid,
(o) 4'-[[2-n-propyl-4-methyl-6-(imidazo[1,2-a]pyridin-2-yl)-benzimidazol-1-yl]-methyl]-2-(1H-tetrazol-5-yl)-biphenyl,
(p) 4'-[[2-n-propyl-4-methyl-6-(imidazo [2,1-b]thiazol-6-yl)-benzimidazol-1-yl]-methyl]-2-(1H-tetrazol-5-yl)-biphenyl,
(q) 4'-[(2-n-propyl-4-methyl-6-(1-methyl-6-fluorobenzimidazol-2-yl)-benzimidazol-1-yl)-methyl]-biphenyl-2-carboxylic acid, and
(r) 4'-[(2-ethyl-4-methyl-6-(5,6,7,8-tetrahydroimidazo[1,2-a]pyridin-2-yl)-benzimidazol-1-yl)-methyl]-biphenyl-2-carboxylic acid,
and the salts thereof with inorganic or organic acids or bases, particularly the physiologically acceptable salts thereof.

**5.** 4'-[[2-n-Propyl-4-methyl-6-(1-methylbenzimidazol-2-yl)-benzimidazol-1-yl]-methyl]-biphenyl-2-carboxylic acid and the salts thereof with inorganic or organic acids or bases, particularly the physiologically acceptable salts thereof, prepared according to claim 1.

**6.** 4'-[(2-Ethyl-4-methyl-6-(5,6,7,8-tetrahydroimidazo[1,2-a]pyridin-2-yl)-benzimidazol-1-yl)-methyl]-biphenyl-2-carboxylic acid and the salts thereof with inorganic or organic acids or bases, particularly the physiologically acceptable salts thereof, prepared according to claim 1.

**7.** Process for preparing a pharmaceutical composition, characterised in that a compound according to at least one of claims 1 to 6 is incorporated in one or more inert carriers and/or diluents by a non-chemical method.

## Revendications

### Revendications pour les Etats contractants suivants : AT, BE, CH, DE, DK, FR, GB, IT, LI, LU, NL, SE

**1.** Benzimidazoles de formule générale

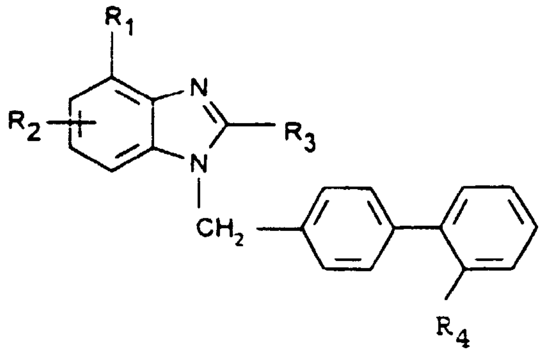

(I)

où

$R_1$ représente un atome de fluor, de chlore ou de brome, un groupe alkyle, cycloalkyle, fluorométhyle, difluorométhyle ou trifluorométhyle,

$R_2$ représente un groupe alkyléne-imino ou alcénylène-imino à 5, 6 ou 7 chaînons éventuellement substitué par un ou deux groupes alkyle ou par un groupe tétraméthylène ou pentaméthylene, dans lequel un groupe méthylène est remplacé par un groupe carbonyle ou sulfonyle,

un groupe maléimido éventuellement mono- ou disubstitué par un groupe alkyle ou phényle, les substituants pouvant être identiques ou différents,

un groupe benzimïdazol-2-yle ou 4,5,6,7-tétrahydrobenzimïdazol-2-yle éventuellement substitué en position 1 par un groupe alkyle de 1 à 6 atomes de carbone ou par un groupe cycloalkyle, le noyau phényle de l'un des groupes benzimidazole cités précédemment pouvant être substitué en outre par un atome de fluor ou un groupe méthyle ou trifluorométhyle, un groupe imidazo[2,1-b]thiazol-6-yle, imidazo[1,2-a]pyridin-2-yle, 5,6,7, 8-tétrahydro-imidazo-[1,2-a]pyridin-2-yle, imidazo[1,2-a]pyrimidin-2-yle, imidazo[4,5-b]pyridin-2-yle, imidazo[4,5-c]pyridin-2-yle, imidazo[1,2-c]pyrimidin-2-yle, imidazo[1,2-a]pyrazin-2-yle, imidazo[1,2-b]pyridazin-2-yle, purin-8-yle, imidazo[4,5-b]-pyrazin-2-yle, imidazo[4,5-c]pyridazin-2-yle ou imidazo-[4,5-d]pyridazin-2-yle,

un groupe pyridyle ou

un groupe imidazolyle, éventuellement fixé en position 1 par un groupe alkyle ou benzyle par l'intermédiaire d'un atome de carbone, qui peut en outre être substitué dans le squelette carboné par un groupe alkyle,

$R_3$ représente un groupe alkyle de 1 à 5 atomes de carbone ou un groupe cycloalkyle de 3 à 5 atomes de carbone et

$R_4$ représente un groupe carboxyle ou 1H-tétrazolyle, et leurs sels avec des acides ou des bases inorganiques ou organiques,

où, sauf indication contraire, une partie alkyle citée précédemment peut contenir dans chaque cas 1 à 3 atomes de carbone et une partie cycloalkyle citée précédemment peut contenir dans chaque cas 3 à 7 atomes de carbone.

**2.** Benzimidazoles de formule générale I selon la revendication 1

où

$R_1$ représente un atome de chlore, un groupe alkyle de 1 à 3 atomes de carbone ou un groupe trifluorométhyle,

$R_2$ représente un groupe alkyléne-imino à 5, 6 ou 7 chaînons dans lequel un groupe méthylène est remplacé par un groupe carbonyle ou sulfonyle,

un groupe maléimido éventuellement mono- ou disubstitué par un groupe alkyle de 1 à 3 atomes de carbone ou par un groupe phényle, les substituants pouvant être identiques ou différents,

un groupe benzimidazol-2-yle ou 4,5,6,7-tétrahydrobenzimïdazol-2-yle éventuellement substitué en position 1 par un groupe alkyle de 1 à 6 atomes de carbone ou par un groupe cycloalkyle, le noyau phényle de l'un des groupes benzimidazole cités précédemment pouvant être substitué en outre par un atome de fluor ou un groupe méthyle ou trifluorométhyle, un groupe imidazo[2,1-b]thiazol-o-yle, imidazo[1,2-a]pyridin-2-yle, 5,6,7, 8-tétrahydro-imidazo [1,2-a]pyridin-2-yle, imidazo[1,2-a]pyrimidin-2-yle, imidazo[4,5-b]pyridin-2-yle, imidazo[4,5-c]pyridin-2-yle, imidazo[1,2-c]pyrimidin-2-yle, imidazo[1,2-a]pyrazin-2-yle, imidazo[1,2-b]pyridazin-2-yle, purin-8-yle, imidazo[4,5-b]-pyrazin-2-yle, imidazo[4,5-c]pyridazin-2-yle ou imidazo-[4,5-d]pyridazin-2-yle,

un groupe pyridyle ou

un groupe imidazol-4-ylesubstitué en position 1 par un groupe alkyle de 1 à 3 atomes de carbone ou par un groupe benzyle, qui peut en outre être substitué dans le squelette carboné par un groupe alkyle de 1 à 3 atomes de carbone,

$R_3$ représente un groupe alkyle de 1 à 5 atomes de carbone ou un groupe cycloalkyle de 3 à 5 atomes de carbone et

$R_4$ représente un groupe carboxyle ou IH-tétrazolyle,

et leurs sels avec des acides ou des bases inorganiques ou organiques.

**3.** Benzimidazoles de formule générale I selon la revendication 1 où
$R_1$ représente un groupe méthyle ou un atome de chlore,
$R_2$ représente un groupe alkyléne-imino à 5, 6 ou 7 chaînons dans lequel un groupe méthylène est remplacé par un groupe carbonyle ou sulfonyle,
un groupe maléimido éventuellement mono- ou disubstitué par un groupe alkyle de 1 à 3 atomes de carbone ou par un groupe phényle, les substituants pouvant être identiques ou différents,
un groupe benzimidazol-2-yle ou 4,5,6,7-tétrahydrobenzimidazol-2-yle éventuellement substitué en position 1 par un groupe alkyle de 1 à 3 atomes de carbone, le noyau phényle de l'un des groupes benzimidazole cités précédemment pouvant être substitué en outre par un atome de fluor, un groupe imidazo[1,2-a]pyridin-2-yle, 5,6,7,8-tétrahydroimidazo[1,2-a]pyridin-2-yle, imidazo[1,2-a]pyrimidin-2-yle ou imidazo[2,1-b]thiazol-6-yle ou un groupe imidazol-4-ylesubstitué en position 1 par un groupe alkyle de 1 à 3 atomes de carbone,
R3 représente un groupe alkyle de 1 à 5 atomes de carbone ou un groupe cycloalkyle de 3 à 5 atomes de carbone et R4 représente un groupe carboxyle ou 1H-tétrazolyle, et leurs sels avec des acides ou des bases inorganiques ou organiques.

**4.** Benzimidazoles de formule générale I selon la revendication 1 suivants:
(a) acide 4'-[[2-n-propyl-4-methyl-6-(1-méthylbenzimidazol-2-yl)-benzimidazol-1-yl]-méthyl]-biphényl-2-carboxylique,
(b) 4'-[[2-n-propyl-4-méthyl-6-(1-methylbenzimidazol-2-yl)-benzimidazol-1-yl]-méthyl]-2-(1H-tétrazol-5-yl)-biphényle,
(c) 4'-[[2-n-propyl-4-methyl-6-(butanesultam-1-yl)-benzimidazol-1-yl]-méthyl]-2-(1H-tétrazol-5-yl)-biphényle,
(d) acide 4'-[[2-n-butyl-6-(2,3-diméthylmaléimino)-4-méthyl-benzimidazol-1-yl]-méthyl]-biphényl-2-carboxylique,
(e) acide 4'-[(2-cyclopropyl-4-methyl-6-(1-methylbenzimidazol-2-yl)-benzimidazol-1-yl)-methyl]-biphenyl-2-carboxylique,
(f) acide 4'-[(2-n-propyl-4-méthyl-6-(1-méthyl-5-fluorobenzimidazol-2-yl)-benzimidazol-1-yl)-méthyl]-biphényl-2-carboxylique,
(g) 4'-[(2-n-propyl-4-methyl-6-(imidazo[1,2-a]pyrimidin-2-yl)-benzimidazol-1-yl)-méthyl]-2-(1H-tétrazol-5-yl)-biphényle,
(h) acide 4'-[(2-n-propyl-4-methyl-6-(5,6,7,8-tétrahydroimidazo[1,2-a]pyridin-2-yl)-benzimidazol-1-yl)-méthyl]-biphényl-2-carboxylique,
(i) 4'-[(2-n-propyl-4-méthyl-6-(5,6,7,8-tétrahydroimidazo[1,2-a]pyridin-2-yl)-benzimidazol-1-yl)-méthyl]-2-(1H-tétrazol-5-yl)-biphényle,
(j) 4'-[(2-n-propyl-4-chloro-6-(1-méthylbenzimidazol-2-yl)-benzimidazol-1-yl)-méthyl]-2-(1H-tétrazol-5-yl)-biphényle,
(k) acide 4'-[[2-n-propyl-4-méthyl-6-(imidazo[2,1-b]thiazol-6-yl)-benzimidazol-1-yl]-méthyl]-biphényl-2-carboxylique,
(l) 4'-[[2-éthyl-4-methyl-6-(butanesultam-1-yl)-benzimidazol-1-yl]-méthyl]-2-(1H-tétrazol-5-yl)-biphényle,
(m) 4'-[[2-n-butyl-4-méthyl-6-(1-méthylbenzimidazol-2-yl)-benzimidazol-1-yl]-méthyl]-2-(1H-tétrazol-5-yl)-biphényle,
(n) acide 4'-[[2-n-propyl-4-méthyl-6-(imidazo[1,2-a]pyridin-2-yl)-benzimidazol-1-yl]-méthyl]-biphényl-2-carboxylique,
(o) 4'-[[2-n-propyl-4-méthyl-6-(imidazo[1,2-a]pyridin-2-yl)-benzimidazol-1-yl]-méthyl]-2-(1H-tétrazol-5-yl)-biphényle,
(p) 4'-[[2-n-propyl-4-méthyl-6-(imidazo]2,1-b]thiazol-6-yl)-benzimidazol-1-yl]-méthyl]-2-(1H-tétrazol-5-yl)-biphényle,
(q) acide 4'-[(2-n-propyl-4-méthyl-6-(1-méthyl-6-fluorobenzimidazol-2-yl)-benzimidazol-1-yl)-méthyl]-biphényl-2-carboxylique et
(r) acide 4'-[(2-éthyl-4-methyl-6-(5,6,7,8-tétrahydroimidazo[1,2-a]pyridin-2-yl)-benzimidazol-1-yl)-méthyl]-biphényl-2-carboxylique,
et leurs sels avec des acides ou des bases inorganiques ou organiques.

**5.** Acide 4'-[[2-n-propyl-4-méthyl-6-(1-methylbenzimidazol-2-yl)-benzimidazol-1-yl]-méthyl]-biphényl-2-carboxylique et ses sels avec des acides ou des bases inorganiques ou organiques.

**6.** Acide 4'-[(2-éthyl-4-méthyl-6-(5,6,7,8-tétrahydroimidazo[1,2-a]pyridin-2-yl)-benzimidazol-1-yl)-méthyl]-biphényl-2-carboxylique et ses sels avec des acides ou des bases inorganiques ou organiques.

**7.** Sels physiologiquement acceptables des composés selon au moins l'une des revendications 1 à 6 avec des acides

ou des bases inorganiques ou organiques.

**8.** Médicament contenant un composé selon au moins l'une des revendications 1 à 6 ou un sel physiologiquement acceptable selon la revendication 7 outre éventuellement un ou plusieurs supports et/ou diluants inertes.

**9.** Utilisation d'un composé selon au moins l'une des revendications 1 à 7 pour la préparation d'un médicament à effet antagoniste de l'angiotensine.

**10.** Procédé de préparation d'un médicament selon la revendication 8 caractérisé en ce que, par voie non chimique, un composé selon au moins l'une des revendications 1 à 7 est incorporé dans un ou plusieurs supports et/ou diluants inertes.

**11.** Procédé de préparation des benzimidazoles selon les revendications 1 à 7 caractérisé en ce que

a) un composé de formule générale

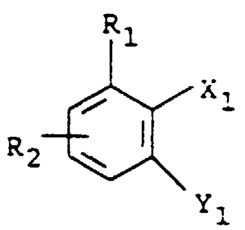

(II)

où
$R_1$ et $R_2$ sont définis comme dans les revendications 1 à 6, l'un des restes $X_1$ et $Y_1$ représente un groupe de formule générale

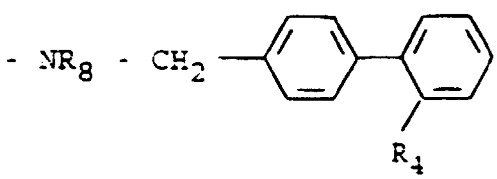

et l'autre des restes $X_1$ et $Y_1$ représente un groupe de formule générale

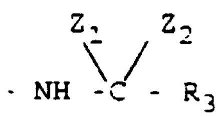

où
$R_3$ et $R_4$ sont définis comme dans les revendications 1 à 6, $R_8$ représente un atome d'hydrogène ou un groupe $R_3CO$ où $R_3$ est défini comme indiqué précédement,
$Z_1$ et $Z_2$, qui peuvent être identiques ou différents, représentent des groupes amino éventuellement substitués ou
des groupes hydroxyle ou mercapto éventuellement substitués par des groupes alkyle inférieurs ou
$Z_1$ et $Z_2$ représentent, ensemble un atome d'oxygène ou de soufre, un groupe imino éventuellement substitué par un groupe alkyle de 1 à 3 atomes de carbone, un groupe alkylènedioxy ou alkylènedithio de 2 ou 3 atomes de carbone dans chaque cas, l'un des restes $X_1$ et $Y_1$ devant toutefois représenter un groupe de formule générale

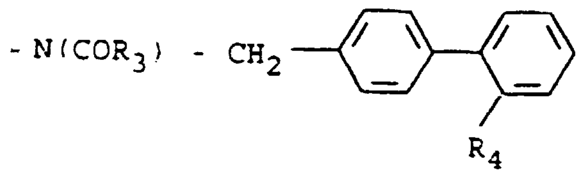

ou

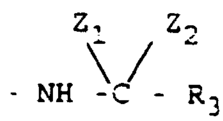

est cyclisé et un N-oxyde correspondant éventuellement ainsi obtenu est réduit ou

b) un benzimidazole de formule générale

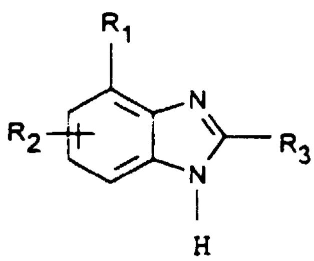
(III)

où
$R_1$ à $R_3$ sont définis comme dans les revendications 1 à 6 est mis à réagir avec un composé du biphényle de formule générale

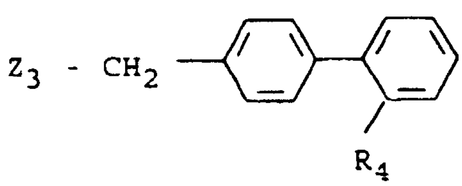
(IV)

où
$R_4$ est défini comme dans les revendications 1 à 6 et
$Z_3$ représente un groupe partant nucléophile
ou

c) pour la préparation d'un composé de formule générale I où $R_4$ représente un groupe carboxyle, un composé de formule générale

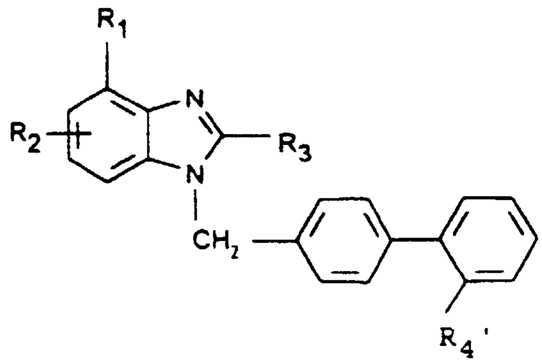

(V)

où
$R_1$ à $R_3$ sont définis comme dans les revendications 1 à 6 et
$R_{4'}$ représente un groupe convertible en un groupe carboxyle par hydrolyse, thermolyse ou hydrogénolyse est converti en un composé carboxylé correspondant ou

d) pour la préparation d'un composé de formule générale I où $R_4$ représente un groupe 1H-tétrazolyle, un reste protecteur d'un composé de formule générale

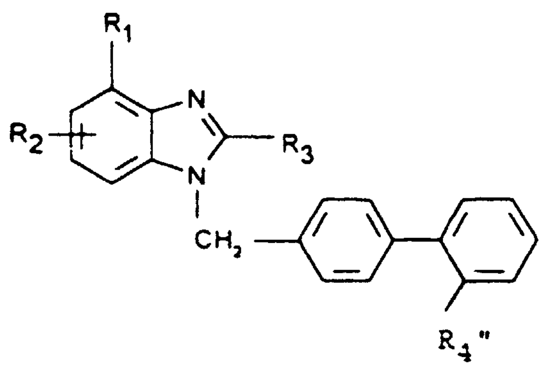

(VI)

où
$R_1$, $R_2$ et $R_3$ sont définis comme dans les revendications 1 à 6 et $R_4$" représente un groupe 1H-tétrazolyle protégé par un reste protecteur en position 1 ou 3 est clivé ou

e) pour la préparation d'un composé de formule générale I où $R_4$ représente un groupe 1H-tétrazolyle, un composé de formule générale

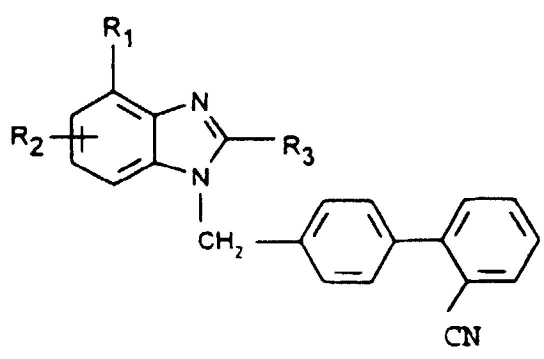

(VII)

où
$R_1$ à $R_3$ sont définis comme dans les revendications 1 à 6 est mis à réagir avec l'acide azothydrique ou ses sels

ou

f) pour la préparation de composés de formule générale I où $R_2$ représente l'un des groupes imidazo[1,2-a]pyridin-2-yle, imidazo[1,2-a]pyrimidin-2-yle, imidazo[1,2-c]pyrimidin-2-yle, imidazo[1,2-a]pyrazin-2-yle, imidazo[1,2-b]pyridazin-2-yle ou imidazo[2,1-b]thiazol-6-yle cités dans les revendications 1 à 6, un composé de formule générale

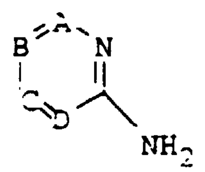

(VIII)

où
l'un des restes A, B, C et D représente un groupe méthine éventuellement substitué par un groupe méthyle ou un atome d'azote et
les autres restes parmi les restes A, B, C et D représentent des groupes méthines ou A et B représentent chacun un groupe méthine et le groupe -C=D représente un atome de soufre, est mis à réagir avec un composé de formule générale

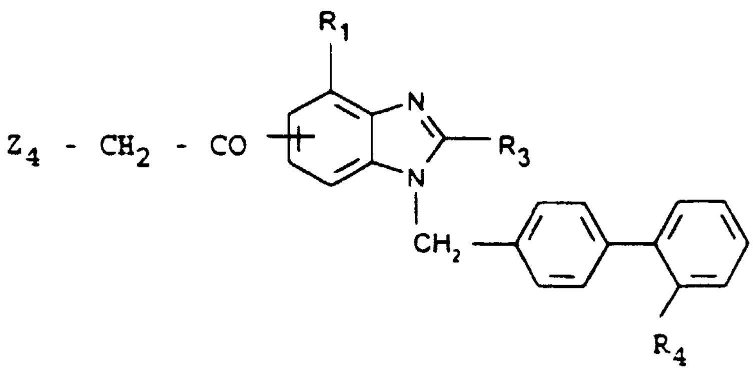

(IX)

où
$R_1$, $R_3$ et $R_4$ sont définis comme dans les revendications 1 à 6 et $Z_4$ représente un groupe partant nucléophile comme un atome d'halogène, par exemple un atome de chlore ou de brome, ou

g) pour la préparation de composés de formule générale I où $R_2$ représente l'un des groupes benzimidazol-2-yle, imidazo[4,5-b] pyridin-2-yle, imidazo[4,5-c]pyridin-2-yle, imidazo[4,5-b] pyrazin-2-yle, imidazo[4,5-c]pyridazin-2-yle, imidazo[4,5-d]pyridazin-2-yle ou purin-8-yle cités dans les revendications 1 à 6, un composé de formule générale

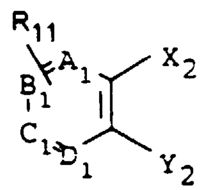

(X)

où
zéro, un ou deux des restes $A_1$, $B_1$, $C_1$ et $D_1$ représentent un atome d'azote et
les autres restes parmi les restes $A_1$, $B_1$, $C_1$ et $D_1$ représentent des groupes méthine et
$R_{11}$ représente un atome d'hydrogène ou de fluor, un groupe méthyle ou trifluorométhyle,
l'un des restes $X_2$ et $Y_2$ représente un groupe $R_{13}$-NH et l'autre des restes $X_2$ et $Y_2$ représente un groupe de formule générale

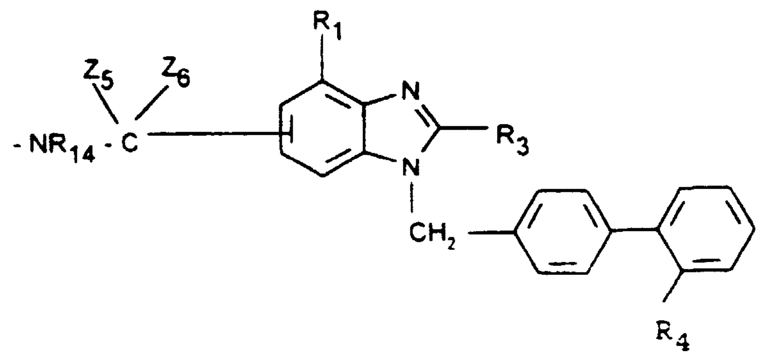

où $R_1$, $R_3$ et $R_4$ sont définis comme dans les revendications 1 à 6, l'un des restes $R_{13}$ et $R_{14}$ représente un atome d'hydrogène et l'autre des restes $R_{13}$ et $R_{14}$ représente un atome d'hydrogène, un groupe alkyle de 1 à 6 atomes de carbone ou un groupe cycloalkyle de 3 à 7 atomes de carbone, $Z_5$ et $Z_6$, qui peuvent être identiques ou différents, représentent des groupes amino éventuellement substitués ou des groupes hydroxyle ou mercapto éventuellement substitués par des groupes alkyle inférieurs ou $Z_5$ et $Z_6$ représentent, ensemble un atome d'oxygène ou de soufre, un groupe imino éventuellement substitué par un groupe alkyle de 1 à 3 atomes de carbone, un groupe alkylènedioxy ou alkylènedithio de 2 ou 3 atomes de carbone dans chaque cas, est cyclisé et un N-oxyde correspondant éventuellement ainsi obtenu est réduit et un composé ainsi obtenu est éventuellement hydrolysé ensuite et
si nécessaire, un reste protecteur utilisé pendant les réactions a) à g) pour la protection de groupes réactifs est clivé et/ou
si nécessaire, un mélange d'isomères ainsi obtenu est résolu en ses isomères et/ou un composé de formule générale I ainsi obtenu est converti en son sel, en particulier pour l'utilisation pharmaceutique en son sel physiologiquement acceptable avec un acide ou une base inorganique ou organique.

**Revendications pour les Etats contractants suivants : ES, GR, PT**

**1.** Benzimidazoles de formule générale

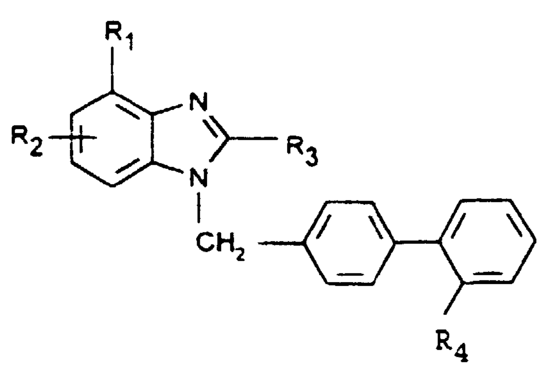

(I)

où
$R_1$ représente un atome de fluor, de chlore ou de brome, un groupe alkyle, cycloalkyle, fluorométhyle, difluorométhyle ou trifluorométhyle,
$R_2$ représente un groupe alkyléne-imino ou alcénylène-imino à 5, 6 ou 7 chaînons éventuellement substitué par un ou deux groupes alkyle ou par un groupe tétraméthylène ou pentaméthylène, dans lequel un groupe méthylène est remplacé par un groupe carbonyle ou sulfonyle,
un groupe maléimido éventuellement mono- ou disubstitué par un groupe alkyle ou phényle, les substituants pouvant être identiques ou différents,
un groupe benzimidazol-2-yle ou 4,5,6,7-tétrahydrobenzimidazol-2-yle éventuellement substitué en position 1 par un groupe alkyle de 1 à 6 atomes de carbone ou par un groupe cycloalkyle, le noyau phényle de l'un des groupes

benzimidazole cités précédemment pouvant être substitué en outre par un atome de fluor ou un groupe méthyle ou trifluorométhyle, un groupe imidazo[2,1-b]thiazol-6-yle, imidazo[1,2-a]pyridin-2-yle, 5,6,7, 8-tétrahydro-imidazo-[1,2-a]pyridin-2-yle, imidazo[1,2-a]pyrimidin-2-yle, imidazo[4,5-b]pyridin-2-yle, imidazo[4,5-c]pyridin-2-yle, imidazo[1,2-c]pyrimidin-2-yle, imidazo[1,2-a]pyrazin-2-yle, imidazo[1,2-b]pyridazin-2-yle, purin-8-yle, imidazo [4,5-b]-pyrazin-2-yle, imidazo[4,5-c]pyridazin-2-yle ou imidazo-[4,5-d]pyridazin-2-yle,
un groupe pyridyle ou
un groupe imidazolyle, éventuellement fixé en position 1 par un groupe alkyle ou benzyle par l'intermédiaire d'un atome de carbone, qui peut en outre être substitué dans le squelette carboné par un groupe alkyle,
$R_3$ représente un groupe alkyle de 1 à 5 atomes de carbone ou un groupe cycloalkyle de 3 à 5 atomes de carbone et
$R_4$ représente un groupe carboxyle ou IH-tétrazolyle, et leurs sels avec des acides ou des bases inorganiques ou organiques,
où, sauf indication contraire, une partie alkyle citée précédemment peut contenir dans chaque cas 1 à 3 atomes de carbone et une partie cycloalkyle citée précédemment peut contenir dans chaque cas 3 à 7 atomes de carbone, caractérisé en ce que

a) un composé de formule générale

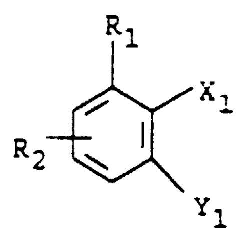

(II)

où
$R_1$ et $R_2$ sont définis comme au début,
l'un des restes $X_1$ et $Y_1$ représente un groupe de formule générale

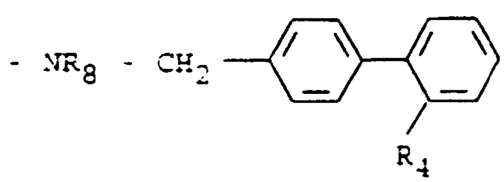

et l'autre des restes $X_1$ et $Y_1$ représente un groupe de formule générale

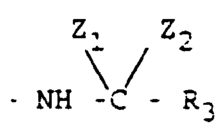

où
$R_3$ et $R_4$ sont définis comme au début,
$R_8$ représente un atome d'hydrogène ou un groupe $R_3CO$ où $R_3$ est défini comme indiqué précédemment,
$Z_1$ et $Z_2$, qui peuvent être identiques ou différents, représentent des groupes amino éventuellement substitués ou des groupes hydroxyle ou mercapto éventuellement substitués par des groupes alkyle inférieurs ou
$Z_1$ et $Z_2$ représentent, ensemble un atome d'oxygène ou de soufre, un groupe imino éventuellement substitué par un groupe alkyle de 1 à 3 atomes de carbone, un groupe alkylènedioxy ou alkylènedithio de 2 ou 3 atomes de carbone dans chaque cas, l'un des restes $X_1$ et $Y_1$ devant toutefois représenter un groupe de formule générale

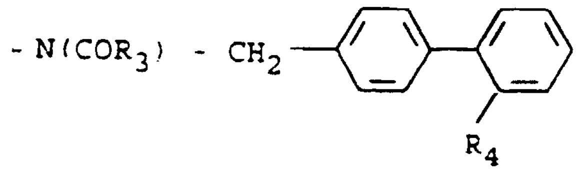

ou

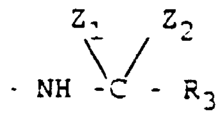

est cyclisé et un N-oxyde correspondant éventuellement ainsi obtenu est réduit ou

b) un benzimidazole de formule générale

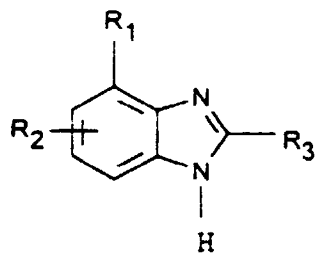

(III)

où
$R_1$ à $R_3$ sont définis comme au début, est mis à réagir avec un composé du biphényle de formule générale

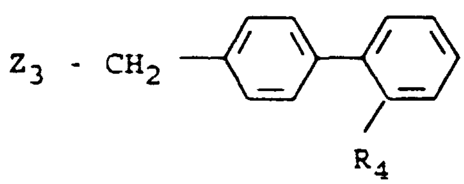

(IV)

où
$R_4$ est défini comme au début et
$Z_3$ représente un groupe partant nucléophile
ou

c) pour la préparation d'un composé de formule générale I où $R_4$ représente un groupe carboxyle, un composé de formule générale

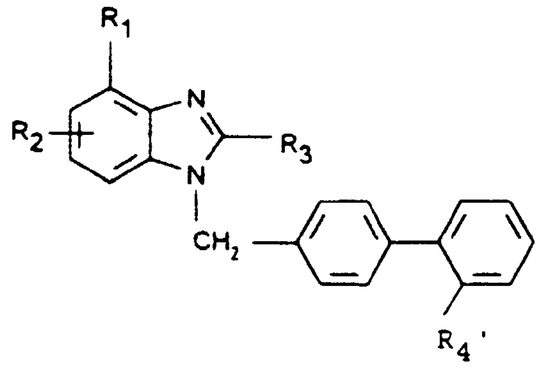

(V)

où
$R_1$ à $R_3$ sont définis comme au début et
$R_{4'}$ représente un groupe convertible en un groupe carboxyle par hydrolyse, thermolyse ou hydrogénolyse est converti en un composé carboxylé correspondant ou

d) pour la préparation d'un composé de formule générale I où $R_4$ représente un groupe 1H-tétrazolyle, un reste protecteur d'un composé de formule générale

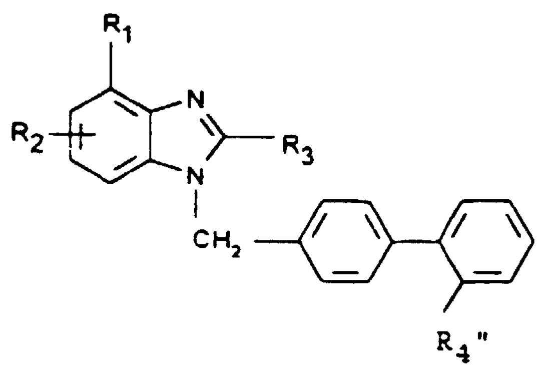

(VI)

où
$R_1$, $R_2$ et $R_3$ sont définis comme au début et $R_{4''}$ représente un groupe 1H-tétrazolyle protégé par un reste protecteur en position 1 ou 3 est clivé ou

e) pour la préparation d'un composé de formule générale I où $R_4$ représente un groupe 1H-tétrazolyle, un composé de formule générale

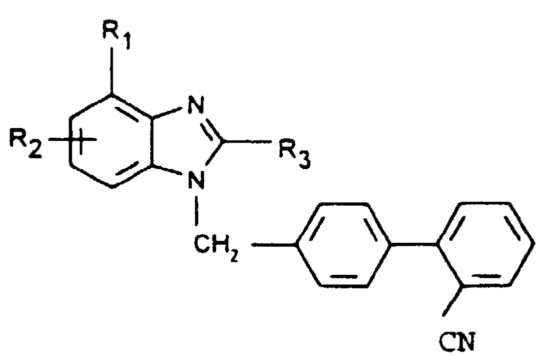

(VII)

où
$R_1$ à $R_3$ sont définis comme au début est mis à réagir avec l'acide azothydrique ou ses sels
ou

f) pour la préparation de composés de formule générale I où $R_2$ représente l'un des groupes imidazo[1,2-a]pyridin-2-yle, imidazo[1,2-a]pyrimidin-2-yle, imidazo[1,2-c]pyrimidin-2-yle, imidazo[1,2-a]pyrazin-2-yle, imidazo[1,2-b]pyridazin-2-yle ou imidazo[2,1-b]thiazol-6-yle cités au début, un composé de formule générale

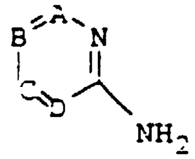

(VIII)

où
l'un des restes A, B, C et D représente un groupe méthine éventuellement substitué par un groupe méthyle ou un atome d'azote et
les autres restes parmi les restes A, B, C et D représentent des groupes méthines ou A et B représentent chacun un groupe méthine et le groupe -C=D représente un atome de soufre, est mis à réagir avec un composé de formule générale

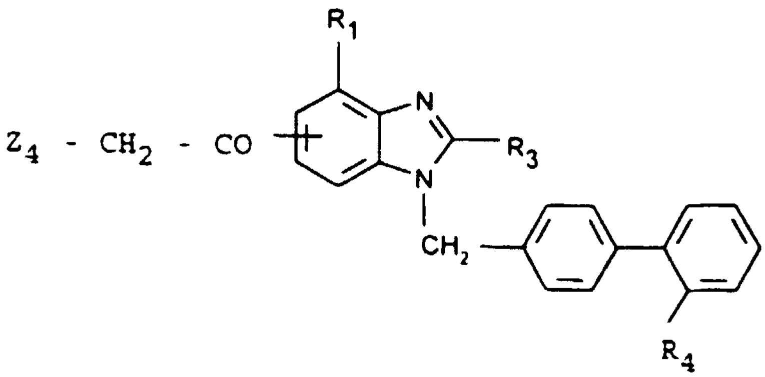

(IX)

où
$R_1$, $R_3$ et $R_4$ sont définis comme au début et $Z_4$ représente un groupe partant nucléophile comme un atome d'halogène, par exemple un atome de chlore ou de brome, ou

g) pour la préparation de composés de formule générale I où $R_2$ représente l'un des groupes benzimidazol-2-yle, imidazo[4,5-b]pyridin-2-yle, imidazo[4,5-c]pyridin-2-yle, imidazo[4,5-b]pyrazin-2-yle, imidazo[4,5-c]pyridazin-2-yle, imidazo[4,5-d]pyridazin-2-yle ou purin-8-yle cités au début, un composé de formule générale

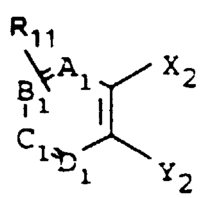

(X)

où
zéro, un ou deux des restes $A_1$, $B_1$, $C_1$ et $D_1$ représentent un atome d'azote et
les autres restes parmi les restes $A_1$, $B_1$, $C_1$ et $D_1$ représentent des groupes méthine et
$R_{11}$ représente un atome d'hydrogène ou de fluor, un groupe méthyle ou trifluorométhyle,
l'un des restes $X_2$ et $Y_2$ représente un groupe $R_{13}$-NH et
l'autre des restes $X_2$ et $Y_2$ représente un groupe de formule générale

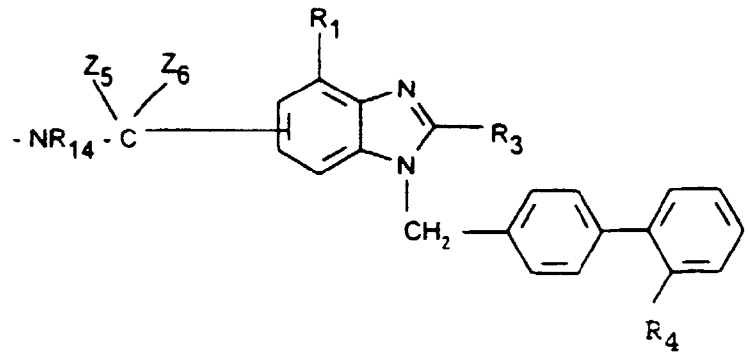

où $R_1$, $R_3$ et $R_4$ sont définis comme au début,
l'un des restes $R_{13}$ et $R_{14}$ représente un atome d'hydrogène et
l'autre des restes $R_{13}$ et $R_{14}$ représente un atome d'hydrogène, un groupe alkyle de 1 à 6 atomes de carbone ou un groupe cycloalkyle de 3 à 7 atomes de carbone,
$Z_5$ et $Z_6$, qui peuvent être identiques ou différents, représentent des groupes amino éventuellement substitués ou des groupes hydroxyle ou mercapto éventuellement substitués par des groupes alkyle inférieurs ou
$Z_5$ et $Z_6$ représentent, ensemble un atome d'oxygène ou de soufre, un groupe imino éventuellement substitué par un groupe alkyle de 1 à 3 atomes de carbone, un groupe alkylènedioxy ou alkylènedithio de 2 ou 3 atomes de carbone dans chaque cas, est cyclisé et un N-oxyde correspondant éventuellement ainsi obtenu est réduit et un composé ainsi obtenu est éventuellement hydrolysé ensuite et
si nécessaire, un reste protecteur utilisé pendant les réactions a) à g) pour la protection de groupes réactifs est clivé et/ou
si nécessaire, un mélange d'isomères ainsi obtenu est résolu en ses isomères et/ou un composé de formule générale I ainsi obtenu est converti en son sel, en particulier pour l'utilisation pharmaceutique en son sel physiologiquement acceptable avec un acide ou une base inorganique ou organique.

**2.** Benzimidazoles de formule générale I selon la revendication 1 où
$R_1$ représente un atome de chlore, un groupe alkyle de 1 à 3 atomes de carbone ou un groupe trifluorométhyle,
$R_2$ représente un groupe alkyléne-imino à 5, 6 ou 7 chaînons dans lequel un groupe méthylène est remplacé par un groupe carbonyle ou sulfonyle,
un groupe maléimido éventuellement mono- ou disubstitué par un groupe alkyle de 1 à 3 atomes de carbone ou par un groupe phényle, les substituants pouvant être identiques ou différents,
un groupe benzimidazol-2-yle ou 4,5,6,7-tétrahydrobenzimidazol-2-yle éventuellement substitué en position 1 par un groupe alkyle de 1 à 6 atomes de carbone ou par un groupe cycloalkyle, le noyau phényle de l'un des groupes benzimidazole cités précédemment pouvant être substitué en outre par un atome de fluor ou un groupe méthyle ou trifluorométhyle, un groupe imidazo[2,1-b]thiazol-6-yle, imidazo[1,2-a]pyridin-2-yle,5,6,7,8-tétrahydro-imidazo [1,2-a]pyridin-2-yle, imidazo[1,2-a]pyrimidin-2-yle, imidazo[4,5-b]pyridin-2-yle, imidazo[4,5-c]pyridin-2-yle, imidazo[1,2-c]pyrimidin-2-yle, imidazo[1,2-a]pyrazin-2-yle, imidazo[1,2-b]pyridazin-2-yle, purin-8-yle,imidazo[4,5-b]-pyrazin-2-yle, imidazo[4,5-c]pyridazin-2-yle ou imidazo-[4,5-d]pyridazin-2-yle,
un groupe pyridyle ou
un groupe imidazol-4-yle substitué en position 1 par un groupe alkyle de 1 à 3 atomes de carbone ou par un groupe benzyle, qui peut en outre être substitué dans le squelette carboné par un groupe alkyle de 1 à 3 atomes de carbone, $R_3$ représente un groupe alkyle de 1 à 5 atomes de carbone ou un groupe cycloalkyle de 3 à 5 atomes de carbone et $R_4$ représente un groupe carboxyle ou 1H-tétrazolyle, et leurs sels avec des acides ou des bases inorganiques ou organiques, en particulier leurs sels physiologiquement acceptables, préparés selon la revendication 1.

**3.** Benzimidazoles de formule générale I selon la revendication 1 où
$R_1$ représente un groupe méthyle ou un atome de chlore,
$R_2$ représente un groupe alkyléne-imino à 5, 6 ou 7 chaînons dans lequel un groupe méthylène est remplacé par un groupe carbonyle ou sulfonyle,
un groupe maléimido éventuellement mono- ou disubstitué par un groupe alkyle de 1 à 3 atomes de carbone ou par un groupe phényle, les substituants pouvant être identiques ou différents,
un groupe benzimidazol-2-yle ou 4,5,6,7-tétrahydrobenzimidazol-2-yle éventuellement substitué en position 1 par

un groupe alkyle de 1 à 3 atomes de carbone, le noyau phényle de l'un des groupes benzimidazole cités précédemment pouvant être substitué en outre par un atome de fluor, un groupe imidazo[1,2-a]pyridin-2-yle, 5,6,7,8-tétrahydroimidazo[1,2-a]pyridin-2-yle, imidazo[1,2-a]pyrimidin-2-yle ou imidazo[2,1-b]thiazol-6-yle ou un groupe imidazol-4-yle substitué en position 1 par un groupe alkyle de 1 à 3 atomes de carbone,
$R_3$ représente un groupe alkyle de 1 à 5 atomes de carbone ou un groupe cycloalkyle de 3 à 5 atomes de carbone et $R_4$ représente un groupe carboxyle ou 1H-tétrazolyle, et leurs sels avec des acides ou des bases inorganiques ou organiques, en particulier leurs sels physiologiquement acceptables, préparés selon la revendication 1.

**4.** Benzimidazoles de formule générale I préparés selon la revendication 1 suivants:
(a) acide 4'-[[2-n-propyl-4-méthyl-6-(1-méthylbenzimidazol-2-yl)-benzimidazol-1-yl]-méthyl]-biphényl-2-carboxylique,
(b) 4'-[[2-n-propyl-4-méthyl-6-(1-méthylbenzimidazol-2-yl)-benzimidazol-1-yl]-méthyl]-2-(1H-tétrazol-5-yl)-biphényle,
(c) 4'-[[2-n-propyl-4-methyl-6-(butanesultam-1-yl)-benzimidazol-1-yl]-méthyl]-2-(1H-tétrazol-5-yl)-biphényle,
(d) acide 4'-[[2-n-butyl-6-(2,3-diméthylmaléimino)-4-méthyl-benzimidazol-1-yl]-méthyl]-biphényl-2-carboxylique,
(e) acide 4'-[(2-cyclopropyl-4-méthyl-6-(1-méthylbenzimidazol-2-yl)-benzimidazol-1-yl)-méthyl]-biphényl-2-carboxylique,
(f) acide 4'-[(2-n-propyl-4-méthyl-6-(1-méthyl-5-fluorobenzimidazol-2-yl)-benzimidazol-1-yl)-méthyl]-biphényl-2-carboxylique,
(g) 4'-[(2-n-propyl-4-méthyl-6-(imidazo[1,2-a]pyrimidin-2-yl)-benzimidazol-1-yl)-méthyl]-2-(1H-tétrazol-5-yl)-biphényle,
(h) acide 4'-[(2-n-propyl-4-méthyl-6-(5,6,7,8-tétrahydroimidazo[1,2-a]pyridin-2-yl)-benzimidazol-1-yl)-méthyl]-biphényl-2-carboxylique,
(i) 4-[(2-n-propyl-4-méthyl-6-(5,6,7,8-tétrahydro-imidazo-[1,2-a]pyridin-2-yl)-benzimidazol-1-yl)-méthyl]-2-(1H-tétrazol-5-yl)-biphényle,
(j) 4'-[(2-n-propyl-4-chloro-6-(1-méthylbenzimidazol-2-yl)-benzimidazol-1-yl)-méthyl]-2-(1H-tétrazol-5-yl)-biphényle,
(k) acide 4'-[[2-n-propyl-4-méthyl-6-(imidazo[2,1-b]thiazol-6-yl)-benzimidazol-1-yl]-méthyl]-biphényl-2-carboxylique,
(l) 4'-[[2-éthyl-4-méthyl-6-(butanesultam-1-yl)-benzimidazol-1-yl]-méthyl]-2-(1H-tétrazol-5-yl)-biphényle,
(m) 4'-[[2-n-butyl-4-méthyl-6-(1-méthylbenzimidazol-2-yl)- benzimidazol-1-yl]-méthyl]-2-(1H-tétrazol-5-yl)-biphényle,
(n) acide 4'-[[2-n-propyl-4-méthyl-6-(imidazo[1,2-a]pyridin-2-yl)-benzimidazol-1-yl]-méthyl]-biphényl-2-carboxylique,
(o) 4'-[[2-n-propyl-4-méthyl-6-(imidazo[1,2-a]pyridin-2-yl)-benzimidazol-1-yl]-méthyl]-2-(1H-tétrazol-5-yl)-biphényle,
(p) 4'-[[2-n-propyl-4-méthyl-6-(imidazo[2,1-b]thiazol-6-yl)-benzimidazol-1-yl]-méthyl]-2-(1H-tétrazol-5-yl)-biphényle,
(q) acide 4'-[(2-n-propyl-4-méthyl-6-(1-méthyl-6-fluorobenzimidazol-2-yl)-benzimidazol-1-yl)-méthyl]-biphényl-2-carboxylique et
(r) acide 4'-[(2-éthyl-4-méthyl-6-(5,6,7,8-tétrahydroimidazo[1,2-a]pyridin-2-yl)-benzimidazol-1-yl)-méthyl]-biphényl-2-carboxylique,
et leurs sels avec des acides ou des bases inorganiques ou organiques, en particulier leurs sels physiologiquement acceptables.

**5.** Acide 4'-[[2-n-propyl-4-méthyl-6 -(1-méthylbenzimidazol-2-yl)-benzimidazol-1-yl]-méthyl]-biphényl-2-carboxylique et ses sels avec des acides ou des bases inorganiques ou organiques, en particulier ses sels physiologiquement acceptables, préparés selon la revendication 1.

**6.** Acide 4'-[(2-éthyl-4-méthyl-6 -(5,6,7,8-tétrahydroimidazo[1,2-a]pyridin-2-yl)-benzimidazol-1-yl)-méthyl]-biphényl-2-carboxylique et ses sels avec des acides ou des bases inorganiques ou organiques, en particulier ses sels physiologiquement acceptables, préparés selon la revendication 1.

**7.** Procédé de préparation d'un médicament caractérisé en ce que, par voie non chimique, un composé selon au moins l'une des revendications 1 à 6 est incorporé dans un ou plusieurs supports et/ou diluants inertes.